(19) Europäisches Patentamt
European Patent Office
Office européen des brevets

(11) Numéro de publication: **0 153 229 B1**

(12) # FASCICULE DE BREVET EUROPEEN

(45) Date de publication de fascicule du brevet: **07.04.93**

(21) Numéro de dépôt: **85400218.5**

(22) Date de dépôt: **11.02.85**

(51) Int. Cl.5: **C07D 513/04**, C07D 303/48, C07D 401/04, C07C 69/716, C07C 69/738, A61K 31/54, //(C07D513/04,279:00,205:00)

Le dossier contient des informations techniques présentées postérieurement au dépôt de la demande et ne figurant pas dans le présent fascicule.

(54) **Nouveaux dérivés de l'acide 1-déthia 2-thia céphalosporanique, leur procédé et leurs intermédiaires de préparation, leur application comme médicaments et les compositions les renfermant.**

(30) Priorité: **13.02.84 FR 8402138**

(43) Date de publication de la demande: **28.08.85 Bulletin 85/35**

(45) Mention de la délivrance du brevet: **07.04.93 Bulletin 93/14**

(84) Etats contractants désignés: **AT BE CH DE FR GB IT LI LU NL SE**

(56) Documents cités:
**EP-A- 0 031 982**
**FR-A- 2 324 639**
**FR-A- 2 505 840**

**CHEMICAL ABSTRACTS, vol. 72, no. 25, 22 juin 1970, page 333, no. 132401c, Columbus, Ohio, US; J. VILLIERAS et al.: "Alpha-bromoglycidate and beta-bromopyruvate esters" & C.R. ACAD. SCI., SER C 1970, 270(14), 1250-1252**

(73) Titulaire: **ROUSSEL-UCLAF**
**35, Boulevard des Invalides**
**F-75007 Paris(FR)**

(72) Inventeur: **Teutsch, Jean-Georges**
**Résidence Lavoisier, Bât. 3 3, rue Lavoisier**
**F-93500-Pantin(FR)**
Inventeur: **Bonnet, Alain**
**19, allée Lucien Michard**
**F-93190-Livry-Gargan(FR)**
Inventeur: **Aszodi, Jozsef**
**53, avenue d'Alfortville**
**F-94600-Choisy-Le-Roi(FR)**
Inventeur: **Costerousse, Germain**
**10, rue des Réservoirs**
**F-94410-Saint-Maurice(FR)**

(74) Mandataire: **Bourgouin, André et al**
**Département des Brevets ROUSSEL UCLAF**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.5x/3.0.1)

CANADIAN JOURNAL OF CHEMISTRY, vol. 60, no. 21, 1982, pages 2707-2710; A. OURARI et al.: "Méthode générale d'obtention des alpha-céto esters beta-florés"

CHEMICAL ABSTRACTS, vol. 98, no. 9, 28 février 1983, page 602, no. 71800v, Columbus, Ohio, US; & JP - A - 57 116 091 (SUMITOMO CHEMICAL CO., LTD.) 19-07-1982

RECENT ADVANCES IN THE CHEMISTRY OF BETA-LACTAM ANTIBIOTICS, publ. no. 38, 1980, pages 80-87, The Royal Society of Chemistry, London, GB; A.K. BOSE et al.: "Convenient stereoselective synthesis of isocephalosporins from Threonine"

CHEMICAL ABSTRACTS, vol. 80, no. 7, 18 février 1974, page 260, no. 36678t, Columbus, Ohio, US; B. MORAUD et al.: "Preparation and properties of alpha-alkoxy-alpha-beta-epoxy esters" & C.R. ACAD. SCI., SER C 1973, 277(13), 523-525

CHEMICAL ABSTRACTS, vol. 84, no. 1, 5 janvier 1976, page 369, no. 4315p, Columbus, Ohio, US; M. JUY et al.: "Stereochemistry of the organomagnesium addition to beta-halo-alpha-ketoesters" & C.R. HEBD. SEANCES ACAD. SCI., SER. C 1974, 279(11), 469-472

THE JOURNAL OF ORGANIC CHEMISTRY, vol. 44, no. 25, 7 décembre 1979, pages 4741-4742, The American Chemical Society; L.G. MUELLER et al.: "Ring expansion synthesis of fused trans-alpha-methylene gamma-lactones"

## Description

La présente demande concerne de nouveaux dérivés de l'acide 1-déthia 2-thia céphalosporanique, leur procédé et leurs intermédiaires de préparation, leur application comme médicaments et les compositions les renfermant.

Dans le brevet français BF 2 505 840 sont décrits des dérivés 2-thia céphalosporaniques comportant en position 3 des radicaux différents de ceux des produits de la présente demande. Par ailleurs, les produits de l'art antérieur sont moins actifs que ceux de la demande.

Le manuel Medicinal Chemistry, third edition Part I, pages 75 et 77, décrit le bioisostérisme entre certains groupements organiques.

La présente demande a pour objet les produits de formule générale (I') :

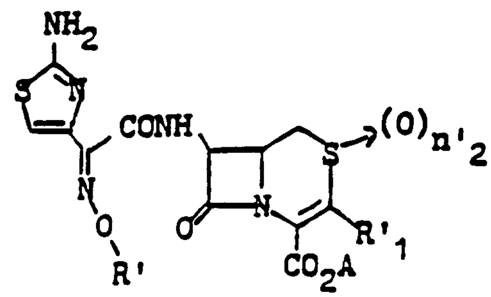

(I')

isomère syn

dans laquelle R' représente un atome d'hydrogène ou un radical alkyle ou alkényle ayant au plus 4 atomes de carbone,

- un radical carboxyméthyle ou 1-carboxyéthyle, ces radicaux étant éventuellement estérifiés ou salifiés,
- un radical phényle, $R'_1$ représente :

  a) un radical $Z'-R'_2$ dans lequel $R'_2$ représente un radical alkyle ou alkényle ayant au plus 4 atomes de carbone, éventuellement interrompu par un hétéroatome et éventuellement substitué par un radical halogène, amino, cyano, carboxy libre estérifié ou salifié, par un radical carbamoyle, Z' représente un atome de soufre ou d'oxygène ;

  soit $Z'-R'_2$ est choisi parmi les radicaux

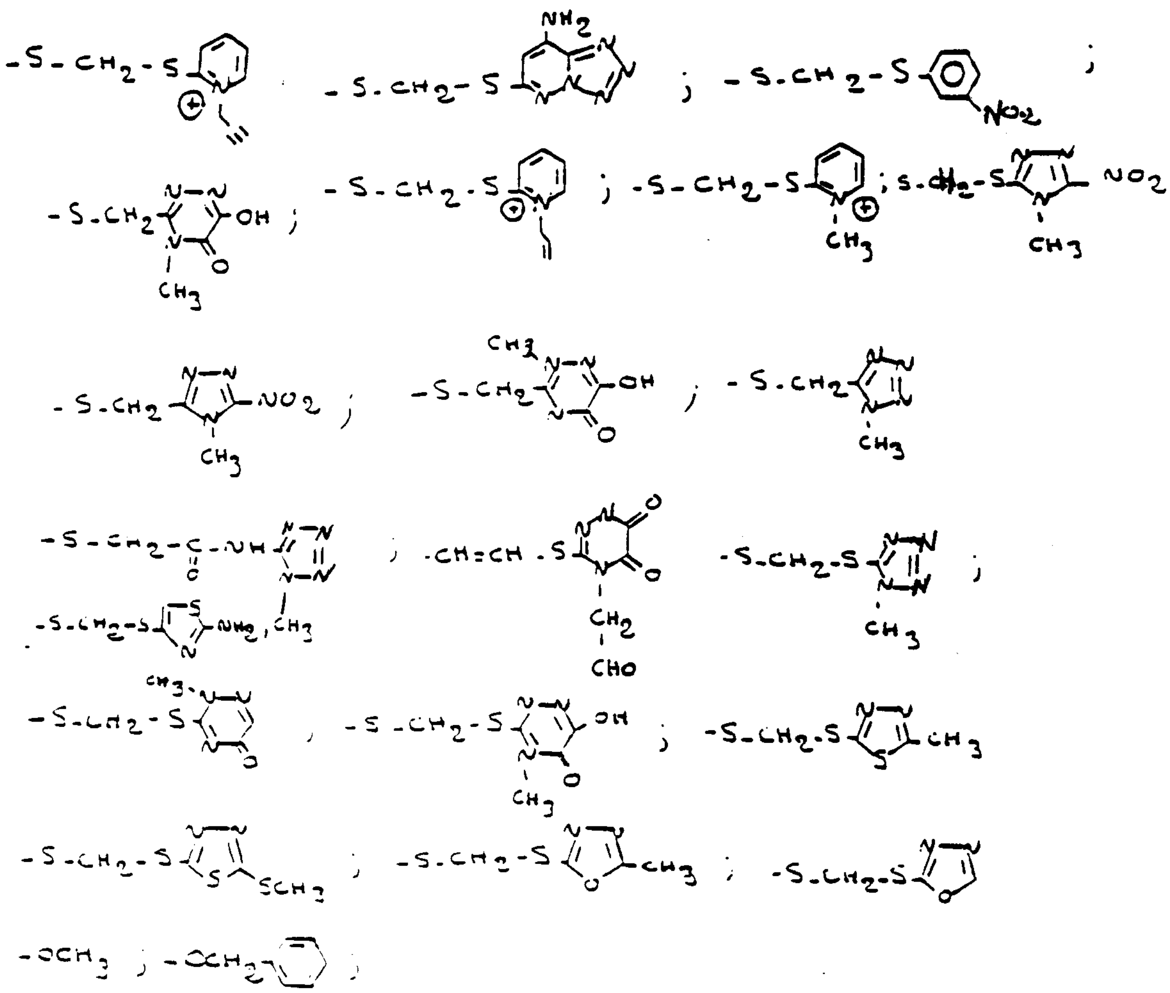

b) soit un radical Z'$_a$-R'$_3$ dans lequel R'$_3$ représente un radical phényle et Z'$_a$ représente un radical méthylène, $-CH_2-S-$ ou un atome de soufre, d'oxygène, de sélénium ou une simple liaison ; soit Z'$_a$-R'$_a$ est choisi parmi les radicaux

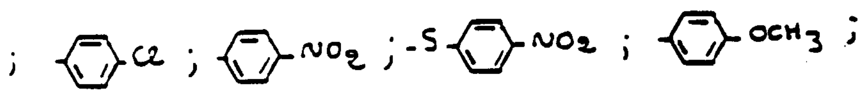

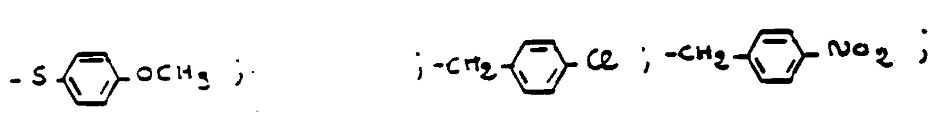

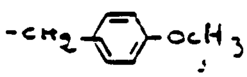 ; 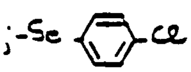 ; 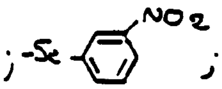 ; 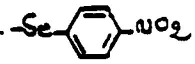 ;

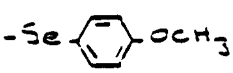 ; 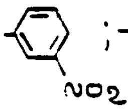 ; 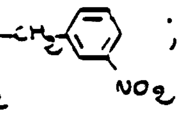 ; 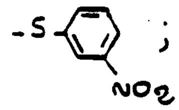 ; 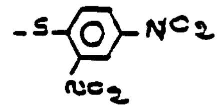 ;

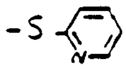 ; 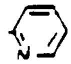 ; 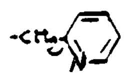 ; 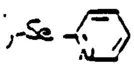 ; 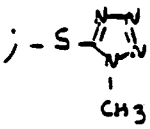 ;

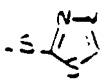 ; 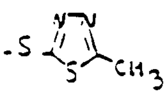 ; 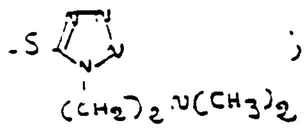 ; 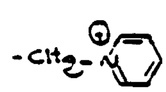 ; 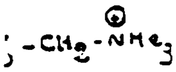 ;

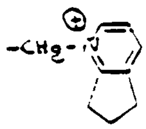 ; 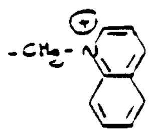 ; 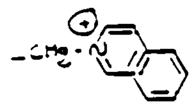 ;

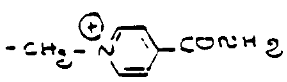 ; 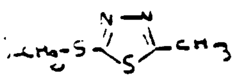 ; 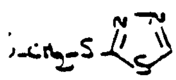 ; 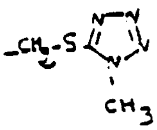

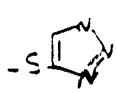 ; 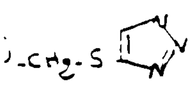 ; 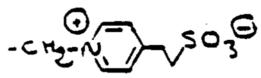 ; 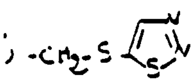 ;

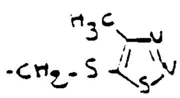 ; 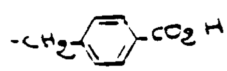 ; 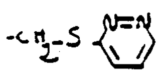 ; 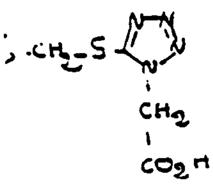 ;

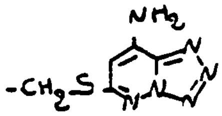 ; 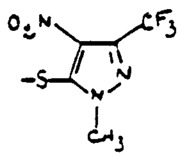 ; 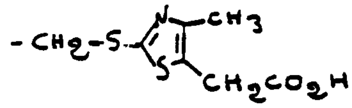 ;

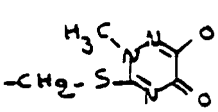 ; 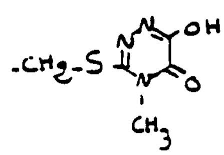 ; 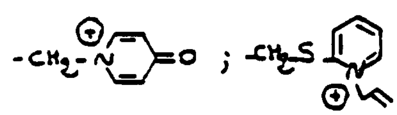 ;

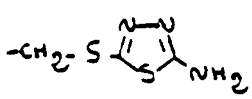 ; 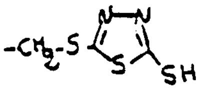 ; 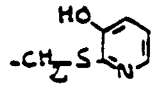 ; 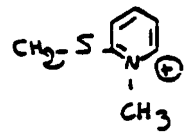 ;

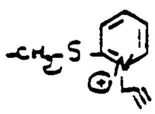 ; 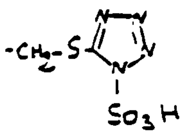 ; 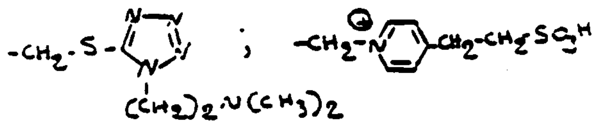 ;

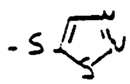 ; 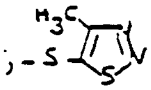 ; 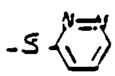 ; 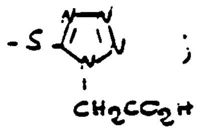 ; 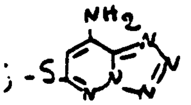 ;

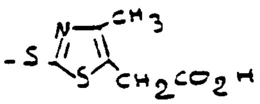 ; 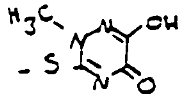 ; 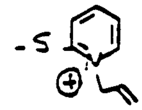 ; 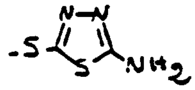 ;

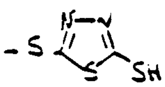 ; 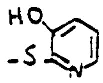 ; 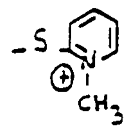 ; 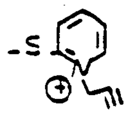 ; 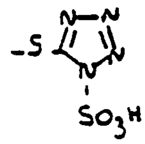 ;

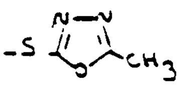 ; 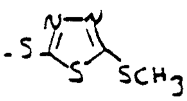 ; 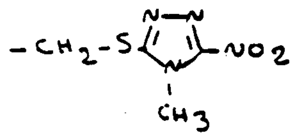 ; 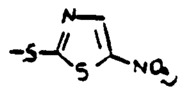

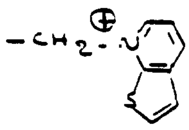 ; 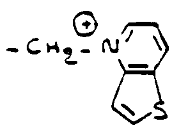 ; 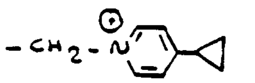 ; 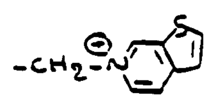

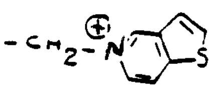 ; 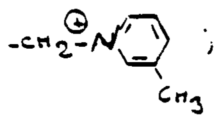 ; 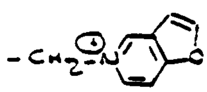 ; 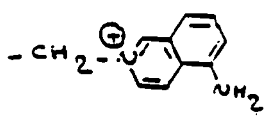

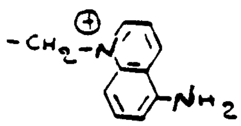 ;

n'$_2$ représente les nombres 0 ou 1 et A représente un atome d'hydrogène, un équivalent de métal alcalin, alcalino-terreux, de magnésium, d'ammonium ou d'une base organique aminée ou A représente un groupement ester ou $CO_2A$ représente un groupement $CO_2^-$ , ainsi que les sels, des produits de formule (I') avec les acides minéraux ou organiques.

Les produits de formule (I') peuvent se présenter sous forme racémique ou optiquement active.

Parmi les valeurs de A, on peut citer un équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris(hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements ester facilement clivables que peut représenter A, les groupements méthoxyméthyle, éthoxyméthyle, isopropyloxyméthyle, $\alpha$-méthoxyéthyle, $\alpha$-éthoxyéthyle, mé-

thylthiométhyle, éthylthiométhyle, isopropylthiométhyle, pivaloylloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadécanoyloxyéthyle, 1-propionyloxypropyle, 1-méthoxycarbonyloxyéthyle, méthoxycarbonyloxyméthyle, 1-acétyloxvbutyle, 1-acétyloxyhexyle, 1-acétyloxyheptyle, phtalidyle, 5,6-diméthoxyphtalidyle, tert-butylcarbonylméthyle, allyle, 2-chloroallyle, méthoxycarbonylméthyle, benzyle ou tert-butyle.

On peut encore citer entre autres restes de groupements esters que peut représenter A, les groupements méthoxyéthoxyméthyle, diméthylaminoéthyle, cyanométhyle, tert-butyloxycarbonyméthyle, 2,2-éthylènedioxyéthyle, cyanoéthyle, 2,2-diméthoxyéthyle, 2-chloroéthoxyméthyle, 2-hydroxyéthoxyéthyle, 2,3-époxypropyle, 3-diméthylamino, 2-hydroxypropyle, 2-hydroxyéthyle, 2-méthylaminoéthoxyméthyle, 2-aminoéthoxyméthyle, 3-méthoxy 2,4-thiadiazol-5-yle, 2-tétrahydropyranyle, 2-méthoxyprop-2-yle, 1-hydroxyprop-2-yle, isopropyle, carbamoylméthyle, chlorométhyle, 2-chloroéthyle, acétylméthyle, 2-méthylthioéthyle ou thiocyanatométhyle.

On peut encore citer entres autres restes de groupements esters que peut représenter A, les groupements 2-chloro 1-acétyloxyéthyle, 2-bromo 1-acétyloxyéthyle, 2-fluoro 1-acétyloxyéthyle, 2-méthoxy 1-acétyloxyéthyle, 2-méthyl 1-acétyloxypropyle, 2-acétyloxyprop-2-yle, 1-méthoxyacétyloxyéthyle, 1-acétylcarbonyloxyéthyle, 1-hydroxyacétyloxyéthyle, 1-formylcarbonyloxyéthyle, 1-(2-thiényl) carbonyloxyéthyle, 1-(2-furyl) carbonyloxyéthyle, 1-(5-nitro 2-furyl) carbonyloxyéthyle, 1-(2-pyrrolyl) carbonyloxyéthyle, 1-(propionyloxycarbonyloxy)éthyle,1-(propyloxycarbonyloxy)éthyle, 1-(isopropyloxycarbonyloxy)éthyle, 1-(méthoxyéthoxycarbonyloxy) éthyle, 1-(allyloxycarbonyloxy)éthyle, 1-(2,3-époxy)propyloxycarbonyloxy éthyle, 1-(2-furyl)méthyloxycarbonyloxy éthyle, 1-(2-fluoro)éthyloxycarbonyloxy éthyle, 1-(méthoxycarbonyloxy)-propyle, (2-méthoxycarbonyloxy)prop-2-yle, (méthoxycarbonyloxy)chlorométhyle, 1-(méthoxycarbonyloxy)2-chloroéthyle, 1-(méthoxycarbonyloxy)2-méthoxyéthyle, 1-(méthoxycarbonyloxy)1-allyle. A peut également représenter le groupement :

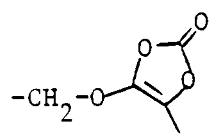

Les produits de formule (I') peuvent également se présenter sous forme de sels d'acides organiques ou de minéraux.

Parmi les acides avec lesquels on peut salifier le ou les groupements amino des produits (I), on peut citer entre autres, les acides acétique, trifluoroacétique, maléïque, tartrique, méthanesulfonique, benzène sulfonique, p-toluène sulfonique, trifluorométhanesulfonique, formique, phosphorique, sulfurique, chlorhydrique, bromhydrique, iodhydrique.

Parmi les valeurs de A, on peut notamment citer les groupements esters de formule :

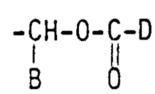

dans lesquels B représente un atome d'hydrogène ou un radical alkyle linéaire ou ramifié éventuellement substitué, renfermant de 1 à 5 atomes de carbone et D représente un radical alcoyle ou alcoxy linéaire ou ramifié, éventuellement substitué, renfermant de 1 à 15 atomesde carbone et notamment de 1 à 5 atomes de carbone, et plus particulièrement les groupements esters dans lesquels a représente un atome d'hydrogène ou un radical méthyle ou éthyle et D représente un radical méthyle, éthyle, méthoxy ou éthoxy.

Parmi les valeurs de A, on peut encore citer le radical

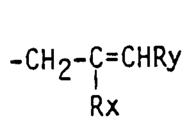

dans lequel Rx représente les valeurs hydrogène, alkyle, notamment méthyle, éthyle, halogène, notamment chlore, et Ry représente les valeurs hydrogène, halogène, aryle, notamment phényle, éventuellement substitué par méthyle, méthoxy ou halogène, ou Ry représente la valeur alkyle éventuellement substitué par acyloxy, par alcoxycarbonyl, par halogène.

Parmi les valeurs de A, on peut encore citer le radical

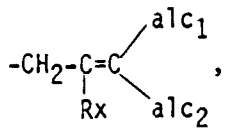

dans lequel Rx est défini comme ci-dessus et $alc_1$ et $alc_2$, identiques ou différents, représentent un radical alkyle renfermant de 1 à 4 atomes de carbone.

Parmi les valeurs de R' , on préfère la valeur méthyle, carboxyméthyle et 1-méthyl 1-carboxyéthyle, ces deux radicaux étant éventuellement estérifiés ou salifiés.

Parmi les valeurs de $R'_1$, on préfère les valeurs suivantes :

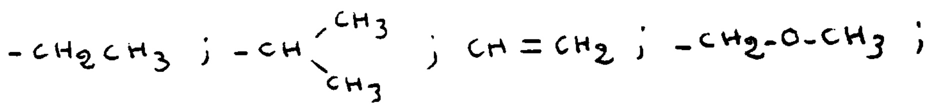

$-S-CH_2-CO_2H$ ; $S-CH_2-CO_2Et$ ; $-S-CH_2-CO_2tBu$ ;

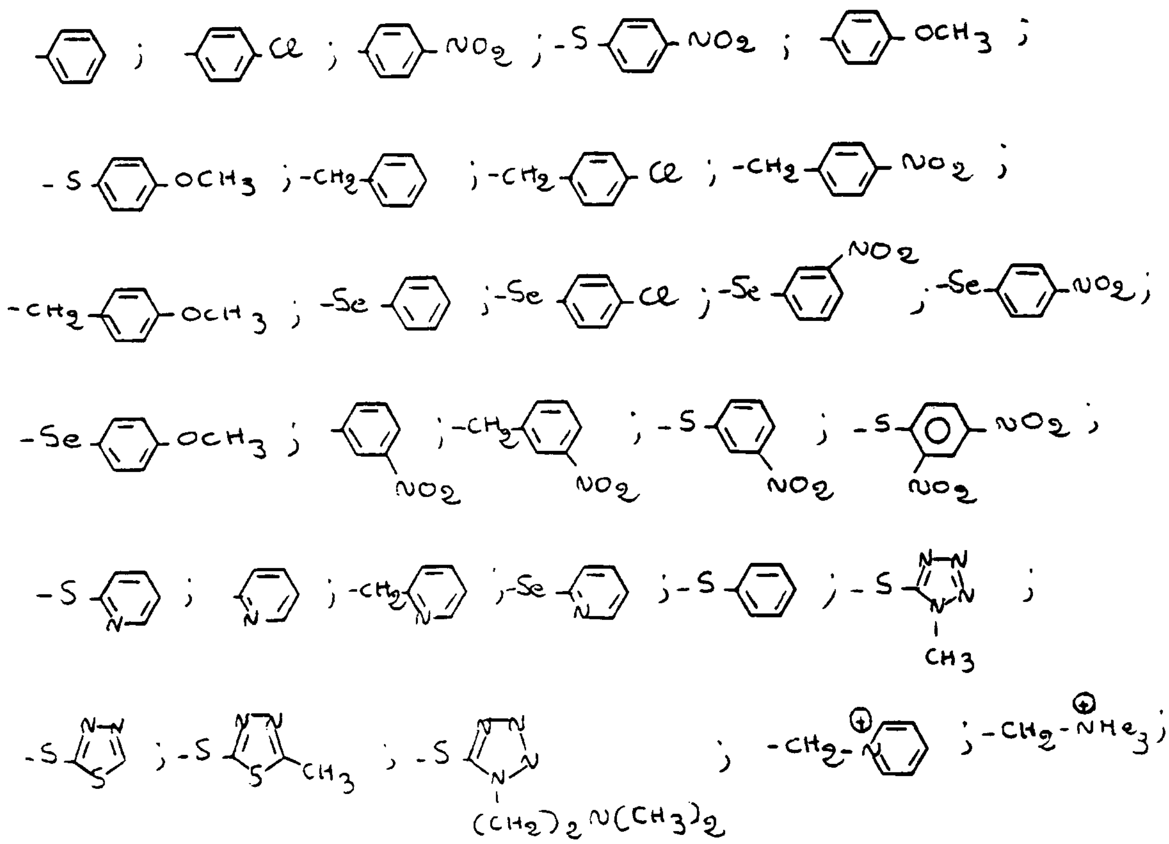

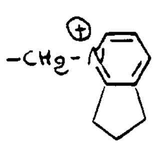 ; 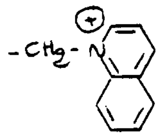 ; 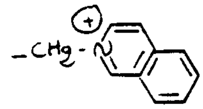 ; $-OCH_3$ ; 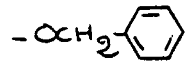 ;

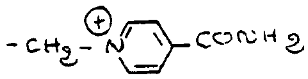 ; 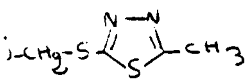 ; 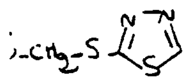 ; 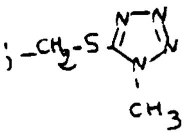 ;

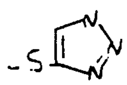 ; 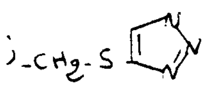 ; 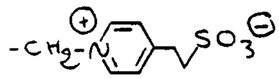 ; 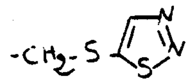 ;

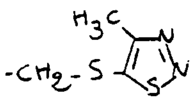 ; 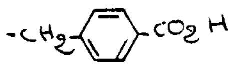 ; 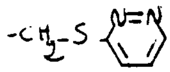 ; 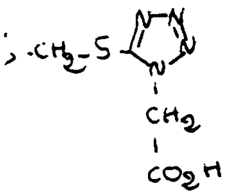 ;

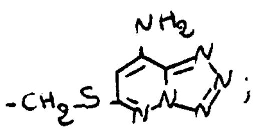 ; 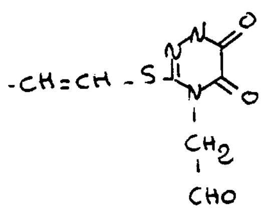 ; 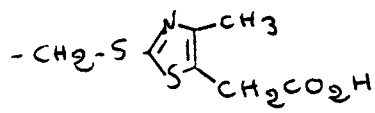 ;

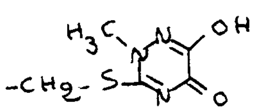 ; 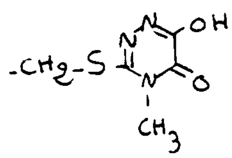 ; 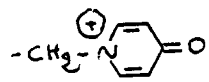 ; 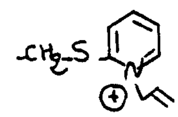 ;

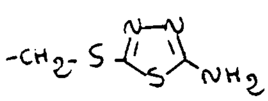 ; 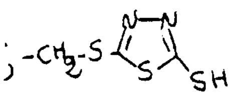 ; 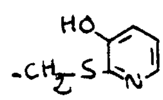 ; 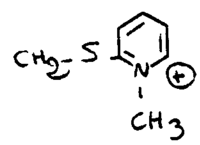 ;

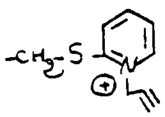 ; 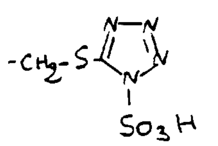 ; 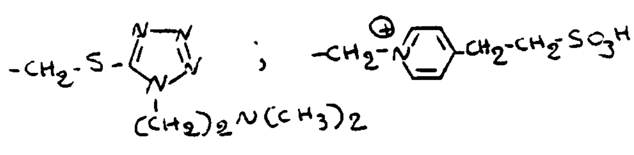

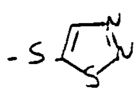 ; 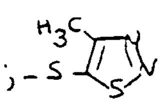 ; 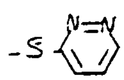 ; 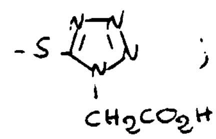 ; 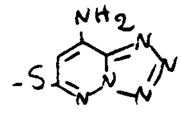 ;

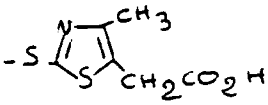 ; 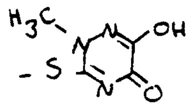 ; 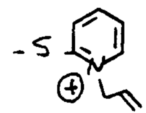 ; 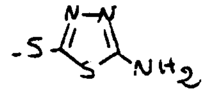 ;

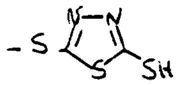 ; 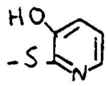 ; 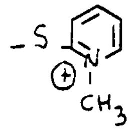 ; 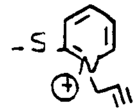 ; 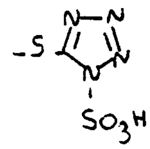 ;

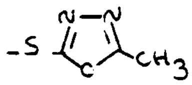 ; 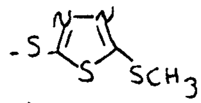 ; 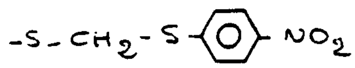 ;

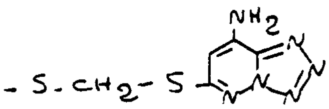 ; 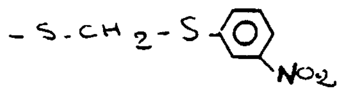 ; 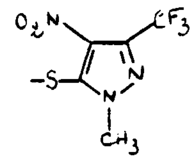

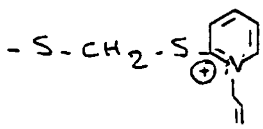 ; 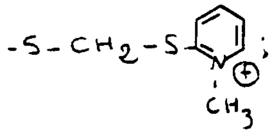 ; 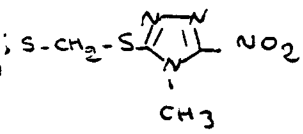 ;

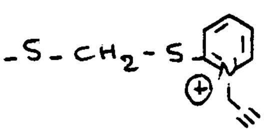 ; 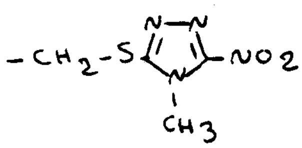 ; 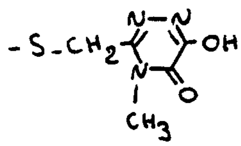 ;

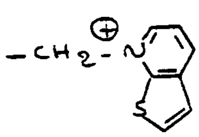 ; 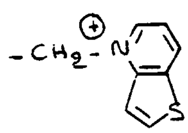 ; 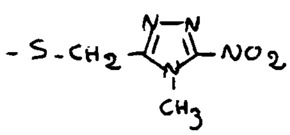 ; 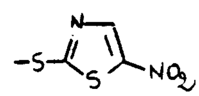

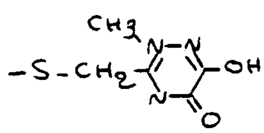 ; 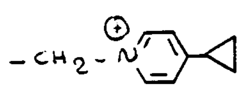 ; 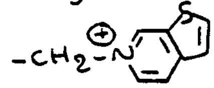 ;

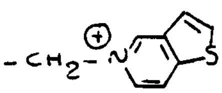 ; 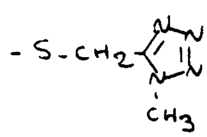 ; $-CH_2N_3$ ; 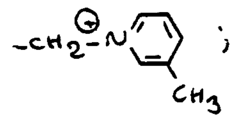 ;

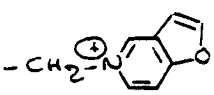 ; $-CH_2-NH_2$ ; 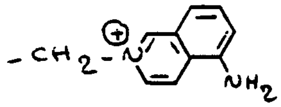 ; 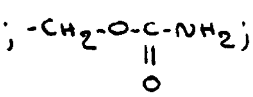 ;

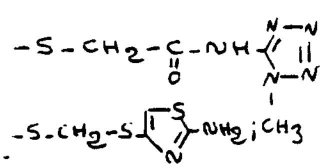 ; 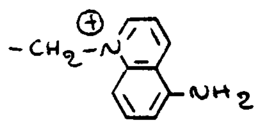 ; 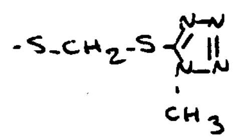 ;

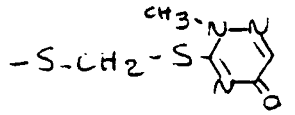 ; 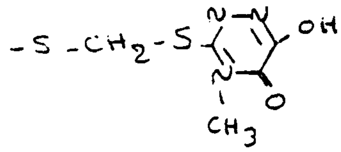 ; 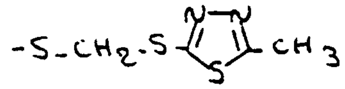 ;

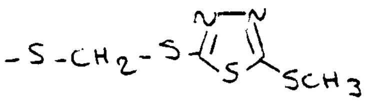 ; 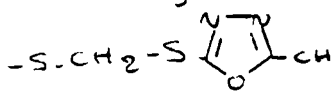 ; 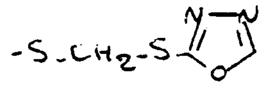 .

Parmi les produits de formule (I') telle que définie ci-dessus, on préfère les produits dans lesquels R' représente un atome d'hydrogène ou un radical méthyle ou allyle et $R'_1$ représente un radical choisi dans le groupe formé par les radicaux méthoxyméthyle, pyridinylthio, pyridinyle, phényl phénylthio ou thiénopyridinium éventuellement substitués par les radicaux méthyle, cyclopropyle, nitro, chloro ou méthoxy; phénylsélényle, méthylthio ou éthylthio éventuellement substitué par un radical carboxy, éthoxycarbonyle ou amino ; éthyle, isopropyle, méthyltétrazolylthio, méthyl ou thiométhyl thiadiazolylthio, méthyloxadiazolylthio, triméthylammonium méthyl, pyridinium éventuellement substitué ou dihydropyridinium, ainsi que ceux dans lesquels $R'_1$ représente un radical Z'a-$R'_3$ dans lequel Z'a représente un atome de soufre et $R'_3$ représente un radical aryle hétérocyclique à 5 ou 6 chaînons éventuellement substitué, ou Z'a représente un radical méthylène et $R'_3$ représente un radical ammonium quaternaire éventuellement substitué.

On peut également citer les valeurs suivantes de $R'_1$ $-S-CH_2CO_2H$ ; $-S-CH_2CO_2Et$ ; $-S-CH_2-CO_2tBu$ ;

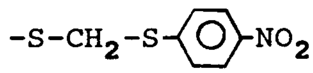 ;

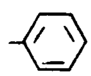 ; 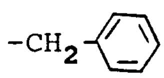 ; 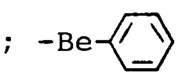 ; 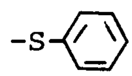 .

L'invention a plus particulièrement pour objet les produits définis ci-après dans les exemples, sous forme racémique ou optiquement active et spécialement :

- l'acide 7-/2-(2-aminothiazol-4-yl) 2-méthoxy imino acétamido/ 3-(3-nitrophénylthio) 8-oxo 4-thia-1-azabicyclo /4,2,0/ oct-2-ène2-carboxylique isomère syn ;
- l'acide 7-/2-(2-aminothiazol-4-yl) 2-méthoxyimino acétamido/ 3-(4-nitrophényl) 8-oxo 4-thia 1-azabicyclo /4,2,0/ oct-2-ène 2-carboxylique isomère syn ;
- l'acide 7-/2-(2-aminothiazol-4-yl) 2-méthoxyimino acétamido/ 3-/(1-méthyl (1-H) tétrazol-5-yl) thio/ 8-oxo 4-thia 1-azabicyclo /4,2,0/ oct-2-ène 2-carboxylique isomère syn ;
- l'acide 7-/2-(2-aminothiazol-4-yl) 2-méthoxyimino acétamido/ 8-oxo 3-/(1-méthyl 1H-tétrazol-5-yl) thiométhyl/ 4-thia 1-azabicyclo /4,2,0/ oct-2-ène 2-carboxylique isomère syn ;
- le 1-//7-(2-amino thiazol-4-yl) (méthoxyimino) acétamido/ 2-carboxylate 8-oxo 4-thia 1-azabicyclo /4,2,0/ oct 2-ène 3-yl/ méthyl/ pyridinium isomère syn ;
- le 1-//7-(2-aminothiazol-4-yl) (méthoxyimino) acétamido/ 2-carboxylate 8-oxo 4-thia 1-azabicyclo /4,2,0/ oct-2-ène 3-yl/ méthyl/ (6,7-dihydro) 5-H/1/ pyridinium isomère syn ;
- le 6-/7 -/2-(2-amino thiazol-4-yl) 2-méthoxyimino acétamido/ 2-carboxy 8-oxo 4-thia 1-azabicyclo /4.2.0/ oct-2-èn-3-yl) méthyl thiéno /2,3-c/ pyridinium syn ;
- le 7-/2-(2-amino thiazol-4-yl) 2-méthoxyimino acétamido/ 8-oxo 2-carboxy 4-thia 1-azabicyclo /4.2.0/ oct-2-èn-3-yl/ méthyl/ triméthyl ammonium syn ;
- le //7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ 4-cyclopropyl/ pyridinium syn ;
- le 6-//7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ 7-méthyl thiéno /2,3-c/ pyridinium syn ;
- le 7-//7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ thiéno /2,3-b/ pyridinium syn ;
- le /7 -/2-(2-amino thiazol-4-yl) 2-(2-propényloxy) imino acétamido/ 2-carboxy 8-oxo 4-thia 1-azabicyclo /4.2.0/ oct-2-èn-3-yl/ méthyl pyridinium syn ;
- le 1-//7-/2-(2-amino thiazol-4-yl) 2-méthoxyimino acétamido/ 2-carboxy 8-oxo 4-thia 1-azabicyclo /4.2.0/ oct-2-èn-3-yl/ méthyl (2-méthylthio)/ pyridinium syn.

La présente invention a également pour objet un procédé de préparation des produits de formule (I') telle que décrite précédemment, caractérisé en ce que l'on traite un produit de formule (II') :

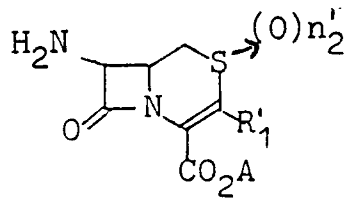

(II')

dans laquelle $R'_1$, A et $n'_2$ ont la signification précédente par un acide de formule (IV') :

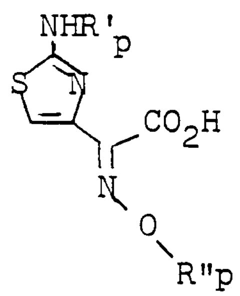

(IV')

ou un dérivé fonctionnel de cet acide, formule (IV') dans laquelle R'p représente un atome d'hydrogène ou un groupement protecteur du radical amino et R"p représente un groupement protecteur du radical hydroxyle ou R"p représente R' pour obtenir un produit de formule (V') :

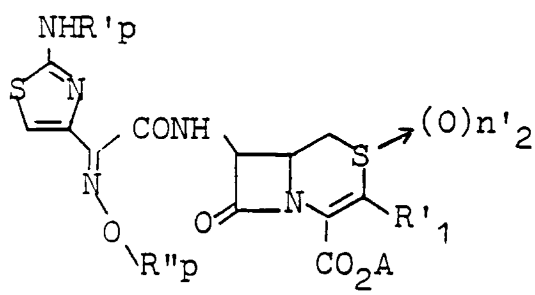

(V')

produit que l'on soumet, si nécessaire ou si désiré, à l'une ou plusieurs des réactions suivantes, dans un ordre quelconque :

a) coupure par hydrolyse, hydrogénolyse ou par action de la thiourée de tout ou partie du ou des groupements protecteurs ;
b) estérification ou salification par une base du ou des groupements carboxy ;
c) salification par un acide du ou des groupements amino ;
d) dédoublement de la molécule pour obtenir un produit optiquement actif ;
e) oxydation de l'atome de soufre en position 2 du cycle isocéphème.

En plus des groupements cités ci-dessus, le groupement ester facilement éliminable que peut représenter A peut être par exemple l'ester formé avec les radicaux butyle, isobutyle, tert-butyle, pentyle, hexyle, acétoxyméthyle, propionyloxyméthyle, butyryloxyméthyle, valéryloxyméthyle, pivaloyloxyméthyle, 2-acétoxyéthyle, 2-propionyloxyéthyle, 2-butyryloxyéthyle.

On peut également citer les radicaux 2-iodoéthyle, $\beta\beta\beta$-trichloroéthyle, vinyle, allyle, éthynyle, propynyle, benzyle, 4-méthoxybenzyle, 4-nitrobenzyle, phényléthyle, trityle, diphénylméthyle, 3,4-diméthoxyphényle, 2-triméthylsilyléthyle.

On peut également citer les radicaux phényle, 4-chlorophényle, tolyle, tert-butylphényle.

Le groupement protecteur du radical amino que peut représenter R'p peut être par exemple un radical alkyle de 1 à 6 atomes de carbone tel que, préférentiellement, tert-butyle ou tert-amyle. R'p peut également représenter un groupement acyle, aliphatique, aromatique ou hétérocyclique ou un groupe carbamoyle. On

peut citer les groupements alcanoyle inférieurs tels que par exemple formyle, acétyle, propionyle, butyryle, isobutyryle, valéryle, isovaléryle, oxalyle, succinyle, pivaloyle. R'p peut également représenter un groupe alkoxy ou cycloalkoxycarbonyle inférieur tel que par exemple, méthoxycarbonyle, éthoxycarbonyle, propoxycarbonyle, 1-cyclopropyléthoxycarbonyle, isopropyloxycarbonyle, butyloxycarbonyle, tert-butyloxycarbonyle, pentyloxycarbonyle, hexyloxycarbonyle, un groupe benzoyle, toluolyle, naphtoyle, phtaloyle, mésyle, phénylacétyle, phénylpropionyle ou un groupe aralcoxycarbonyle, tel que benzyloxycarbonyle.

Les groupements acyles peuvent être substitués par exemple par un atome de chlore, de brome, d'iode ou de fluor. On peut citer les radicaux chloroacétyle, dichloroacétyle, trichloroacétyle, bromoacétyle ou trifluoroacétyle.

R'p peut également représenter un groupement aralkyle inférieur tel que benzyle, 4-méthoxybenzyle, phénylbenzyle, trityle, 3,4-diméthoxybenzyle ou benzhydrile.

R'p peut également représenter un groupe haloalkyle tel que trichloroéthyle.

R'p peut également représenter un groupement chlorobenzoyle, paranitrobenzoyle, para-tert-butylbenzoyle, phénoxyacétyle, caprylyle, n-décanoyle, acryloyle, trichloroéthoxycarbonyle.

R'p peut également représenter un groupement méthylcarbamoyle, phénylcarbamoyle, naphtylcarbamoyle, ainsi que les thiocarbamoyles correspondants.

La liste ci-dessus n'est pas limitative, il est évident que d'autres groupements protecteurs des amines, groupements connus en particulier dans la chimie des peptides, peuvent également être utilisés.

Le groupement de protection du radical hydroxyle que peut représenter R''p, peut être choisi dans la liste ce-dessous : R''p peut représenter un groupe acyle tel que par exemple formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitrobenzoyle.

On peut citer également les groupements éthoxycarbonyle, méthoxycarbonyle, propoxycarbonyle, $\beta\beta\beta$ -trichloroéthoxycarbonyle, benzyloxycarbonyle, tert- butoxycarbonyle, 1-cyclo propyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-méthyl 1-méthoxyéthyle, phtaloyle.

On peut également citer d'autres acyles tels que propionyle, butyryle, isobutyryle, valéryle, isovaléryle, oxalyle, succinyle et pivaloyle.

On peut également citer les radicaux phénylacétyle, phénylpropionyle, mesyle, chlorobenzoyle, paranitrobenzoyle, para-tert-butylbenzoyle, caprylyle, acryloyle, méthylcarbamoyle, phénylcarbamoyle, naphtylcarbamoyle.

Dans un mode préférentiel d'exécution du procédé, on traite le produit de formule (II') par un dérivé fonctionnel d'un produit de formule (IV'). Ce dérivé fonctionnel peut être choisi par exemple un halogénure, un anhydride symétrique ou mixte, un amide ou un ester activé.

Comme exemple d'anhydride mixte, on peut citer par exemple celui formé avec le chloroformiate d'isobutyle et celui formé avec le chlorure de pivaloyle et les anhydrides mixtes carboxylique sulfonique formés par exemple avec le chlorure de paratoluènesulfonyle. Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

On peut citer également l'azide d'acide ou l'amide d'acide.

L'anhydride peut être formé in situ par action de carbodiimide NN'-disubstitué, par exemple la N,N-dicyclohexylcarbodiimide.

La réaction d'acylation est conduite de préférence dans un solvant organique tel que le chlorure de méthylène. On peut cependant utiliser d'autres solvants tels que le tétrahydrofuranne, le choroforme, le diméthylformamide, l'acétone ou le diméthylacétamide.

Lorsqu'on utilise un halogénure d'acide et de manière générale lorsqu'une molécule d'acide halohydrique est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

Dans un mode préféré d'éxécution du procédé, les produits de formule (IV') sont transformés en dérivés réactifs tels que des halogénures de chloroimminium, qui peuvent être préparés en faisant réagir les produits de formule (IV') avec des agents d'halogénation tels que le phosgène, le chlorure d'oxalyle, le chlorure de thionyle. On peut également préparer des complexes avec les sulfates de dialkyle de préférence le sulfate de diméthyle.

Les conditions opératoires pour de telles réactions sont connues de l'homme de métier. De telles réactions sont décrites par exemple dans le brevet français 2 073 338.

Selon les valeurs de R'p, R''p et A, les produits de formule (IV') peuvent ou non constituer des produits de formule (I').

Les produits de formule (IV') constituent des produits de formule (I') lorsque R'p représente un atome d'hydrogène, lorsque R''p ne représente pas un groupement protecteur du radical hydroxyle que l'on désire éliminer, par exemple, 1-méthoxyéthyle, et lorsque A ne représente pas, parmi les groupements esters facilement clivables, l'un de ceux que l'on désire éliminer.

Dans les autres cas, l'action sur le produit de formule (IV') d'un ou plusieurs agents d'hydrolyse, d'hydrogénolyse ou de la thiourée a pour but d'éliminer le radical R'p lorsque celui-ci représente un radical protecteur du radical amino, d'éliminer le radical R''p lorsque celui-ci est différent de R' et/ou d'éliminer le radical A lorsque celui-ci représente, parmi les groupements ester facilement clivables, l'un de ceux que l'on désire éliminer.

Cependant, il est bien entendu possible d'éliminer R'p sans toucher aux substituants R''p et A lorsque ceux-ci doivent être conservés. Il en est ainsi par exemple lorsque A représente un groupement ester que l'on souhaite conserver tel qu'un groupement propionyloxyméthyle.

La nature des réactifs à mettre en jeu dans tous ces cas est bien connue de l'homme de métier. Des exemples de telles réactions sont donnés plus loin dans la partie expérimentale.

On donne ci-après une énumération non exhaustive des moyens pouvant être mis en oeuvre pour éliminer les différents groupements.

L'élimination du groupe R'p peut être effectuée par hydrolyse, celle-ci étant acide, basique ou utilisant l'hydrazine.

On utilise préférentiellement l'hydrolyse acide pour éliminer les groupements alkoxy et cycloalkoxycarbonyle éventuellement substitués, tels que tert-pentyloxycarbonyle ou tert-butyloxycarbonyle, les groupements aralcoxycarbonyle éventuellement substitués tels que benzyloxycarbonyle, les groupements trityle, benzhydryle, tert-butyle ou 4-méthoxybenzyle.

L'acide que l'on utilise de préférence peut être choisi dans le groupe constitué par les acides chlorhydrique, benzène sulfonique ou para-toluène sulfonique, formique ou trifluoroacétique. On peut cependant utiliser d'autres acides minéraux ou organiques.

L'hydrolyse basique est utilisée préférentiellement pour éliminer les groupements acyle tels que trifluoroacétyle.

La base que l'on utilise de préférence est une base minérale telle que l'hydroxyde de sodium ou de potassium. On peut également utiliser la magnésie, la baryte ou un carbonate ou carbonate acide de métal alcalin tel que les carbonates et carbonates acides de sodium ou de potassium ou d'autres bases.

On peut également utiliser l'acétate de sodium ou de potassium.

L'hydrolyse utilisant l'hydrazine est utilisée de préférence pour éliminer des groupes tels que phtaloyle.

Le groupement R'p peut également être éliminé par le système zinc-acide acétique (pour le groupement trichloroéthyle), les groupements benzhydryle, benzyloxycarbonyle sont éliminés de préférence par l'hydrogène en présence d'un catalyseur.

Le groupement chloroacétyle est éliminé par action de la thiourée en milieu neutre ou acide selon le type de réaction décrit par MASAKI J.A.C.S., 90, 4508, (1968).

On peut également utiliser d'autres méthodes de déprotection connues dans la littérature.

Parmi les groupes préférés, on peut citer les groupements formyle, acétyle, éthoxycarbonyle, mésyle, trifluoroacétyle, chloroacétyle, trityle. On préfère particulièrement les radicaux trityle et chloroacétyle.

L'acide que l'on utilise de préférence est l'acide trifluoroacétique.

L'élimination du radical A ou R''p, lorsque celle-ci est nécessaire, est réalisée dans des conditions semblables à celles décrites précédemment pour l'élimination de R'p.

On peut utiliser, entre autres, l'hydrolyse acide pour éliminer les radicaux alkyle ou aralkyle éventuellement substitués.

On utilise préférentiellement un acide choisi dans le groupe formé par les acides chlorhydrique, formique, trifluoroacétique et paratoluène sulfonique.

Les autres valeurs des radicaux A ou R''p sont, lorsque ce la est désiré, éliminées selon les procédés connus de l'homme de métier. On opère de préférence dans des conditions modérées, c'est-à-dire, à température ambiante ou en chauffant légèrement.

Naturellement, on peut lorsque par exemple R'p et A ou R''p sont des groupements éliminables appartenant à des types différents, faire agir sur les produits (IV') plusieurs agents envisagés dans les énumérations précédentes.

La salification des produits peut être effectuée selon les méthodes usuelles.

La salification peut, par exemple, être obtenue par action sur un produit sous forme acide ou sur un solvat, par exemple le solvat éthanolique ou un hydrate de cet acide, d'une base minérale telle que

l'hydroxyde de sodium ou de potassium, le carbonate ou le carbonate acide de sodium ou de potassium. On peut également utiliser les sels d'acides minéraux tels que le phosphate tri-sodique. On peut également faire appel à des sels d'acides organiques.

Comme sels d'acides organiques, on peut mentionner, par exemple, les sels de sodium d'acides carboxyliques aliphatiques, linéaires ou ramifiés, saturés ou insaturés de 1 à 18 et de préférence de 2 à 10 atomes de carbone. Ces radicaux aliphatiques peuvent être interrompus par un ou plusieurs hétéroatomes tels que l'oxygène ou le soufre ou substitués par des radicaux aryle, comme par exemple : phényle, thiényle, furyle, ou par ou plusieurs radicaux hydroxyle ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques des acides aromatiques suffisamment solubles comme par exemple des acides benzoïques substitués, de préférence, par des radicaux alkyles inférieurs.

Comme exemples de tels acides organiques, on peut mentionner : les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthylique de l'acide adipique, les acides hexanoïque, heptanoïque, décanoïque, oléïque, stéarique, palmitique, 3-hydroxypropionique, 3-méthoxypropionique, 3-méthylthiobutyrique, 4-chloro-butyrique, 4-phénylbutyrique, 3-phénoxybutyrique, 4-éthylbenzoïque, 1-propylbenzoïque.

On utilise cependant de préférence comme sels de sodium l'acétate de sodium, le 2-éthyl hexanoate de sodium ou le diéthyl acétate de sodium.

La salification peut également être obtenue par action d'une base organique comme la triéthylamine, la diéthylamine, la triméthylamine, la propylamine, la N,N-diméthyl-éthanolamine, le tris(hydroxyméthyl) amino méthane, la méthylamine, l'éthanolamine, la pyridine, la picoline, la dicyclohexylamine, la morpholine et la benzylamine.

Elle peut également être obtenue par action de l'arginine, de la lysine de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opère en général en faisant réagir l'acide de formule (I') avec un dérivé de formule :

$Z\text{-}R_s$

dans laquelle Z représente un radical hydroxyle ou un atome d'halogène tel que le chlore, le brome, l'iode et $R_s$ désigne le groupement ester à introduire, groupement dont une liste non exhaustive figure ci-dessus. Dans certains cas, il peut être avantageux d'effectuer une estérification sur un produit dont l'amine est bloquée avant d'enlever le groupement protecteur de l'amine.

L'éventuel dédoublement des composés de formule générale (II') ou des composés de formule générale (V') peut être effectué au moyen d'un acide organique carboxylique ou sulfonique optiquement actif comme l'acide tartrique, dibenzoyltartrique, camphosulfonique ou glutamique, la décomposition du sel ainsi obtenue étant effectuée au moyen d'une base minérale telle que le bicarbonate de sodium ou d'une base organique telle qu'une amine tertiaire comme la triéthylamine. De plus, on peut éventuellement utiliser une base optiquement active.

L'oxydation éventuelle des produits de formule (IV') peut être effectuée à l'aide d'oxygène, de péroxydes, d'hydropéroxydes, de péracides ou d'eau oxygénée. La réaction est avantageusement sensibilisée à la lumière. Ces réactifs peuvent être en mélange avec des acides organiques ou minéraux. On utilise de préférence l'acide métachloro perbenzoïque. Les conditions réactionnelles sont connues de l'homme de métier. On trouve de telles conditions exposées par exemple dans le brevet français 2.387.234.

L'invention a plus spécialement pour objet un procédé de préparation des produits de formule (A') :

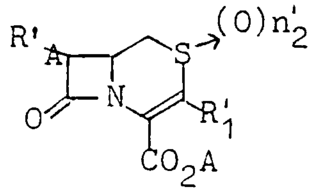

(A')

dans laquelle A, $R'_1$ et $n'_2$ ont la signification indiquée ci-dessus et $R'_A$ représente un radical amino libre ou protégé par un groupement protecteur mono ou divalent, ou $R'_A$ représente un radical :

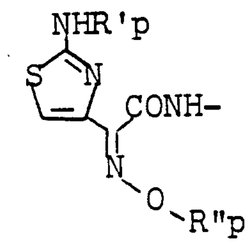

dans laquelle R'p et R"p ont la signification indiquée ci-dessus, caractérisé en ce que l'on traite un produit de formule ($II'_A$) :

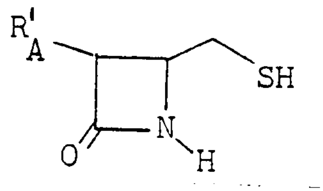

($II'_A$)

par un produit de formule (III'A) :

$$Hal - \underset{R'_1}{\underset{|}{CH}} - CO - CO_2A \qquad (III'_A)$$

dans laquelle Hal représente un atome d'halogène, $R'_1$ et A ont la signification précédente, pour obtenir un produit de formule (A'), produit que l'on soumet, si nécessaire ou si désiré, à l'une quelconque ou à plusieurs des réactions suivantes, dans un ordre quelconque :

a) coupure par hydrolyse, hydrogénolyse ou par action de la thiourée de tout ou partie du ou des groupements protecteurs ;

b) estérification ou salification par une base du ou des groupements carboxy ;

c) salification par un acide du ou des groupements amino ;

d) dédoublement de la molécule pour obtenir un produit optiquement actif ;

e) oxydation de l'atome de soufre en position 2 du cycle isocéphème.

Dans un mode préférentiel d'exécution du procédé ci-dessus, le radical amino que peut représenter $R'_A$ est protégé par un groupement protecteur tel que trityle ou phtalimido.

Hal représente de préférence un atome de chlore ou de brome, de préférence de chlore.

A représente de préférence un radical alkyle, très préférentiellement un radical tert-butyle.

L'action du produit de formule ($III'_A$) sur le produit de formule ($II'_A$) est effectuée de préférence en présence d'une base telle qu'une base aminée, la triéthylamine de préférence ou d'une base telle que le carbonate de lithium. On peut également opérer en présence de diazabicyclooctane.

On obtient intermédiairement les produits de formules (A'$_i$) :

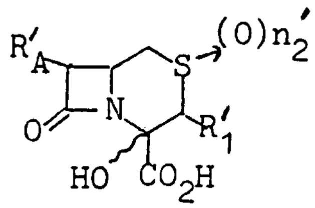

(A'$_1$)

qui peuvent ou non être isolés.

Le stade ultérieur consiste en une déshydratation qui peut être effectuée par exemple à l'aide d'un réactif tel que l'anhydride trifluoroacétique, le trichlorure ou le tribromure ou le triiodure de phosphore, le chlorure de méthanesulfonyle, le diisopropyl carbodiimide, le chlorure de sulfuryle, le chlorure de N,N-diméthyl-thiocarbamoyle, l'oxychlorure de phosphore, le tétraiodure de diphosphore.

On opère de préférence en présence d'une base telle que la pyridine.

Eventuellement, avant l'obtention de l'intermédiaire A'$_i$, on peut isoler un précurseur de formule :

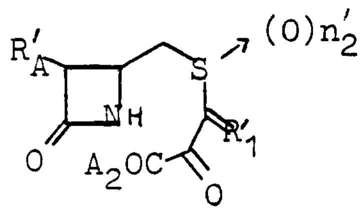

qui est généralement transformé in situ en produit de formule (A$_i$), lui-même conduisant au produit A.

Le procédé peut également être effectué au départ de produits de formule (II$_A$) optiquement actifs, préparés selon le procédé du brevet belge BB 887.428.

La présente invention a également pour objet un procédé de préparation des produits de formule (III'$_{A1}$) :

$$Hal - \underset{R'u}{\underset{|}{CH}} - CO - CO_2A \qquad (III'_{A1})$$

dans laquelle Hal représente un atome d'halogène, R'u représente les valeurs de R$_1$ liées par un atome de carbone, R$_1$ et A ayant la signification indiquée ci-dessus, caractérisé en ce que l'on traite un produit de formule R'uCHO par un produit de formule (Hal$_1$)$_2$ CHCO$_2$A en présence d'une base forte pour obtenir un produit de formule :

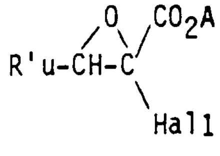

(III'$_{Ai}$)

formules dans lesquelles Hal$_1$ représente un atome d'halogène, produit que l'on traite par un halogénure pour obtenir le produit de formule (III'$_{A1}$) attendu.

Les valeurs de R'u correspondant aux valeurs de R$_1$ liées par un atome de carbone sont les valeurs Za-R$_3$, dans laquelle Za représente un radical -(CH$_2$)$_a$-, une simple liaison ou un radical -CH$_2$-S-, R$_3$ ayant les valeurs indiquées ci-dessus, les valeurs alkyle, alkényle ou alkynyle ayant de 2 à 8 atomes de carbone éventuellement substitué ou interrompu par un hétéroatome.

La base forte en présence de laquelle on fait agir les produits de formule R'u CHO avec les produits de formule (Hal$_1$)$_2$ CH CO$_2$A peut être de préférence le tert-butylate de potassium. On peut cependant utiliser

d'autres bases telles que le diisopropylamidure de lithium, l'hydrure de sodium ou le butyl lithium. On opère de préférence dans un solvant apolaire tel que le trétrahydrofuranne.

On fait ensuite agir sur le produit de formule ($III'_{Ai}$) un halogénure qui est de préférence un halogénure de métal alcalin tel que le bromure de lithium.

Une variante de ce procédé peut également être utilisée. Elle consiste en l'action d'un produit de formule :

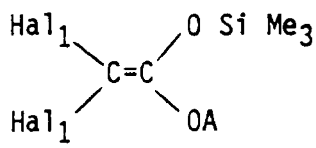

avec le produit de formule R'u CHO en présence d'un fluorure, par exemple un fluorure de tétralkylammonium ou un fluorure de métal alcalin. On obtient alors intermédiairement un produit de formule :

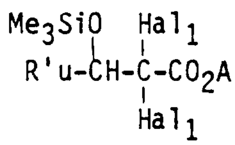

produit qui redonne le produit de formule ($III'_{Ai}$).

Les produits de formule ($III'_{Ai}$) peuvent également être utilisés directement à la place des produits de formule ($III_A$) dans la réaction avec les produits de formule ($II_A$). L'action des produits de formule ($III'_{Ai}$) avec les produits de formule ($II_A$) est effectuée de préférence en présence d'un carbonate de métal alcalin, de préférence le carbonate de lithium.

La présente invention a donc pour objet une variante du procédé décrit ci-dessus de préparation des produits de formule (A') :

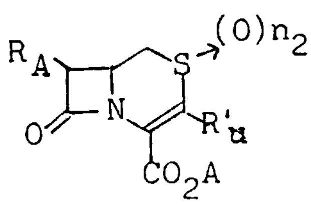

(A')

dans laquelle, $R_A$, $n_2$, A et R'u ont la signification indiquée ci-dessus, caractérisé en ce que l'on traite un produit de formule ($II_A$) tel que décrit ci-dessus par un produit de formule ($III'_{Ai}$) tel que décrit ci-dessus.

Les opérations subséquentes décrites ci-dessus (coupure des groupements protecteurs, estérification, salification, dédoublement ou oxydation) sont bien entendu effectuées comme précédemment.

La présente invention a également pour objet un procédé de préparation des produits de formule ($III''_{A1}$) :

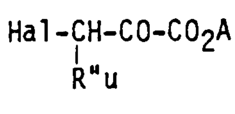

($III''_{A1}$)

dans laquelle Hal représente un atome d'halogène, R''u représente les valeurs de $R_1$ liées par un hétérotaome, $R_1$ et A ayant la signification indiquée ci-dessus, caractérisé en ce que l'on fait réagir un produit de formule :

$N_2 = CH\text{-}CO\text{-}CO_2A$ ($III''_{Ai}$)

avec un produit de formule R''u Hal pour obtenir le produit de formule ($III''_{A1}$) attendu.

Dans un mode préférentiel d'exécution du procédé, l'action des produits de formule ($III''_{Ai}$) avec les produits de formule R''u Hal est effectuée dans un solvant tel que le chlorure de méthylène, le tétrachlorure de carbone ou le benzène, préférentiellement le chlorure de méthylène à une température de l'ordre de 0 ° C à la température ambiante.

Parmi les produits de formule ($III''_{A1}$), on préfère ceux dans lesquels R''u représente les valeurs Z-$R_2$ et Za-$R_3$ dans laquelle Za représente un hétéroatome.

Il faut noter que les produits de formules ($III_A$), ($III'_A$), ($III'_{A1}$) et ($III''_{A1}$) peuvent se trouver sous deux formes tautomères différentes. Par exemple, ($III_A$) peut être également sous la forme :

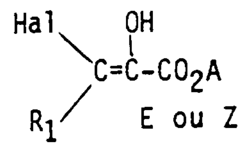

La présente invention a également pour objet un procédé de préparation des produits de formule (IX) :

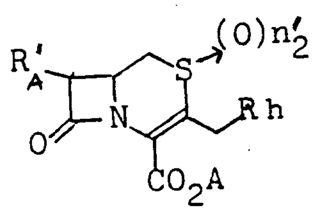

(IX)

dans laquelle $R'_A$, A et $n'_2$ on la signification indiquée ci-dessus et Rh représente un ammonium quaternaire éventuellement substitué ou un radical S-R'h dans lequel R'h représente un radical aryle carbocyclique ou hétérocyclique éventuellement substitué, correspondant à un produit de formule (I) lorsque $R_A$ représente le radical

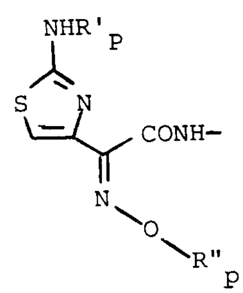

caractérisé en ce que, soit l'on fait agir un produit de formule R'h SH sur un produit de formule (VI) :

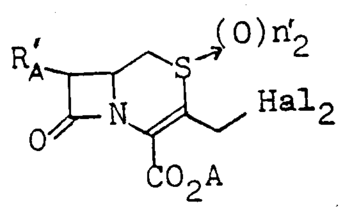

(VI)

formule dans laquelle $R_A$, $R_4$, A et $n_2$ ont la signification précédente et $Hal_2$ représente un atome d'halogène, soit l'on fait agir une amine ou une imine sur un produit de formule (VII) :

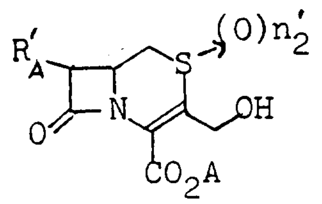

(VII)

pour obtenir un produit de formule (IX) que l'on soumet, si nécessaire ou si désiré, à l'une quelconque ou à plusieurs des réactions suivantes, dans un ordre quelconque :

a) coupure par hydrolyse, hydrogénolyse ou par action de la thiourée de tout ou partie du ou des groupements protecteurs ;

b) estérification ou salification par une base du ou des groupements carboxy ou sulfo ;

c) salification par un acide du ou des groupements amino ;

d) dédoublement de la molécule pour obtenir un produit optiquement actif ;

e) oxydation de l'atome de soufre en position 2 du cycle isocéphème.

f) déblocage de la fonction amine lorsque $R_A$ représente un radical amino protégé,

g) traitement pour un produit de formule ($IV_a$), ($IV_b$) ou ($IV_c$), dans les conditions décrites précédemment.

On opère de préférence avec un sel de sodium du produit de formule $R'_hSH$.

L'amine ou l'imine que l'on fait agir sur le produit de formule (VII) correspond à l'ammonium quaternaire que l'on veut former; notamment si l'on veut introduire un radical pyridinium, on opèrera avec la pyridine. On opère de préférence en présence d'un anhydride tel que l'anhydride trifluorométhanesulfonique.

La présente invention a également pour objet un procédé de préparation des produits de formule (VI) telle que définie ci-dessus, caractérisé en ce que l'on traite un produit de formule ($II_B$) :

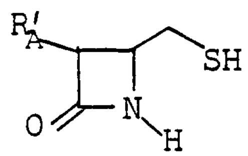

($II'_B$)

dans laquelle $R_A$ et $R_4$ ont la signification indiquée ci-dessus, par un produit de formule ($III_B$) :

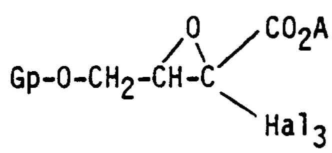

($III'_B$)

dans laquelle A a la signification indiquée ci-dessus, $Hal_3$ représente un atome d'halogène et Gp un groupement protecteur du radical hydroxyle pour obtenir un produit de formule (VIII) :

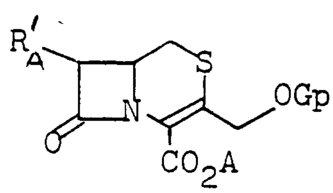

(VIII)

produit que l'on soumet à une réaction de déprotection pour obtenir le produit de formule (VII') :

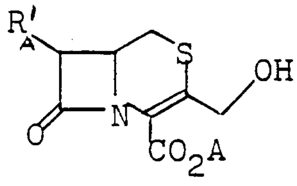

(VII')

produit que l'on soumet éventuellement d'abord à un réactif d'oxydation ou que l'on soumet à une réaction d'halogénation pour obtenir le produit de formule (VI) :

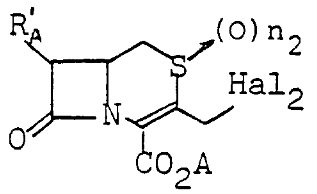

(VI )

produit que l'on soumet éventuellement à une réaction d'oxydatlon.

Le groupement protecteur préféré pour Gp est le triméthylsilyle.

$Hal_3$ représente de préférence un atome de chlore. Le groupement protecteur est clivé de préférence en milieu acide, par exemple, par l'acide chlorhydrique dilué après que la réaction des produits ($II_B$) et ($III_B$) soit effectuée dans les conditions précédemment décrites. Les réactions éventuelles d'oxydation sont également effectuées dans les conditions usuelles. Le réactif d'halogénation est de préférence le chlorure de tosyle en présence de diméthylaminopyridine.

L'invention a également pour objet un procédé de préparation des produits de formule générale (XI) :

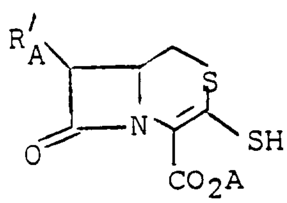

(XI)

dans laquelle $R_A$ et $CO_2A$ ont la valeur indiquée précédemment, caractérisé en ce que l'on transforme un produit de formule (XII) :

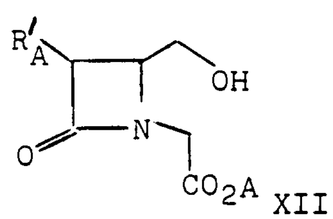

(XII)

dans laquelle $R_A$ est défini comme précédemment et $A_{XII}$ représente un atome d'hydrogène ou un groupement ester facilement clivable, en un dérivé comportant un groupement réactif du radical hydroxy de formule (XIII) :

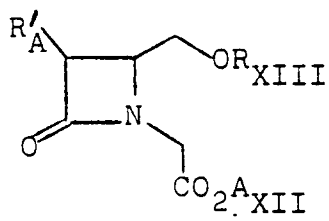

(XIII)

dans laquelle $R'_A$ et $A_{XII}$ sont définis comme précédemment et $R_{XIII}$ représente le reste d'un groupement réactif, que l'on traite par le sulfure de carbone en présence d'une base, pour obtenir le produit de formule (XI).

Dans un mode d'exécution préféré,

- le dérivé comportant un groupement réactif du radical hydroxy est le chlorure de tosyle ;
- la base en présence de laquelle on fait réagir le sulfure de carbone est la bis triméthylsilyl amidure de sodium, potassium ou lithium.

L'invention a notamment pour objet un procédé tel que défini précédemment, caractérisé en ce que l'on utilise au départ, un produit de formule (XII) dans laquelle $R_A$ représente un groupement amino protégé, notamment par un radical tert-butoxycarbonyl.

L'invention a notamment pour objet un procédé tel que défini précédemment, caractérisé en ce que sur le produit de formule (XIII), l'on effectue une ou plusieurs des réactions suivantes, dans un ordre quelconque :

- élimination du groupement protecteur du groupement amino protégé que représente $R_A$ pour obtenir un produit correspondant dans lequel $R_A$ représente une amine libre, que l'on transforme selon les méthodes décrites précédemment en produits de formule (XIII) dans laquelle $R_A$ représente R, R ayant la signification précitée ;
- clivage du radical $A_{XII}$ par les méthodes usuelles ;
- protection du groupement $-CO_2H$.

Les produits de formule générale (I')possèdent une très bonne activité antibiotique sur les bactéries gram (+) telles que les staphylocoques, les streptocoques et, notamment, sur les staphylocoques pénicillino-résistants. Leur efficacité sur les bactéries gram (-), notamment, sur les bactéries coliformes, les klebsiella, les salmonella et les proteus est particulièrement remarquable.

Ces propriétés rendent aptes lesdits produits à être utilisés comme médicaments dans le traitement des affections à germes sensibles et, notamment, dans celui des staphylococcies, telles que septicémies à staphycocoques, staphylococcies malignes de la face ou cutanée, pyodermites, plaies septiques ou suppurantes, anthrax, phlegmons, érésipèles, staphylococcies aigües primitives ou post grippales, bronchopneumonies, suppuration pulmonaire.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

La présente invention a donc également pour objet, à titre de médicaments et notamment de médicaments antibiotiques, les produits de formule (I'), tels que définis ci-dessus, ainsi que leurs sels d'acides, pharmaceutiquement acceptables.

Parmi ces produits de formule (I'), l'invention a également plus particulièrement pour objet, à titre de médicaments et notamment de médicaments antibiotiques, les produits dans lesquels R' représente un atome d'hydrogène ou un radical méthyl ou allyle et $R'_1$ représente un radical choisi dans le groupe formé par les radicaux $-S-CH_2CO_2H$ ; $-S-CH_2CO_2Et$ ; $-S-CH_2-CO_2tBu$ ;

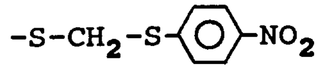 ;

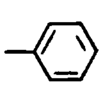 ; 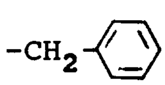 ; 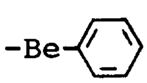 ; 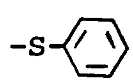 .

L'invention a également plus particulièrement pour objet à titre de médicaments et notamment de médicaments antibiotiques, les produits décrits dans les exemples et plus spécialement :

- l'acide 7-/2-(2-aminothiazol-4-yl) 2-méthoxy imino acétamido/ 3-(3-nitrophénylthio) 8-oxo 4-thia-1-azabicyclo /4,2,0/ oct-2-ène 2-carboxylique isomère syn ;
- l'acide 7-/2-(2-aminothiazol-4-yl) 2-méthoxyimino acétamido/ 3-(4-nitrophényl) 8-oxo 4-thia 1-azabicyclo /4,2,0/ oct-2-ène 2-carboxylique isomère syn ;
- l'acide 7-/2-(2-amniothiazol-4-yl) 2-méthoxyimino acétamido/ 3-/(1-méthyl (1-H) tétrazol-5-yl) thio/ 8-oxo 4-thia 1-azabicyclo /4,2,0/ oct-2-ène 2-carboxylique isomère syn ;
- l'acide 7-/2-(2-aminothiazol-4-yl) 2-méthoxyimino acétamido/ 8-oxo 3-/(1-méthyl 1H-tétrazol-5-yl) thiométhyl/ 4-thia 1-azablcyclo /4,2,0/ oct-2-ène 2-carboxylique isomère syn ;
- le 1-//7-(2-amino thiazol-4-yl) (méthoxyimino) acétamido/ 2-carboxylate 8-oxo 4-thia 1-azabicyclo /4,2,0/ oct 2-ène 3-yl/ méthyl/ pyridinium isomère syn ;
- le 1-//7-(2-aminothiazol-4-yl) (méthoxyimino) acétamido/ 2-carboxylate 8-oxo 4-thia 1-azabicyclo /4,2,0/ oct-2-ène 3-yl/ méthyl/ (6,7-dihydro) 5-H/1/ pyrindinium isomère syn,

plus particulièrement encore :

- l'acide 7-/2-(2-amino thiazol-4-yl) 2-méthoxyimino acétamido/ 3-/(1-méthyl (1-H) tétrazol-5-yl) thio/ 8-oxo 4-thia 1-azabicyclo /4,2,0/ oct-2-ène 2-carboxylique isomère syn ;
- l'acide 7-/2- (2-amino thiazol-4-yl) 2-méthoxyimino acétamido/ 8-oxo 3/(1-méthyl 1H-tétrazol-5-yl) thiométhyl /4-thia 1-azabicyclo /4,2,0/ oct-2-èn 2-carboxylique isomère syn ;
- le 1 //7-(2-aminothiazol-4-yl) (méthoxyimino) acétamido /2-carboxylate 8-oxo 4-thia 1-azabicyclo /4,2,0/ oct-2-ène 3-yl/ méthyl/ pyridinium isomère syn ;
- le 1 //7-(2-aminothiazol-4-yl) (méthoxyimino) acétylamino- 2-carboxylate 8-oxo 4-thia 1-azabicyclo /4,2,0/ oct-2-ène 3-yl/ méthyl/ (6,7-dihydro) 5H /1/ pyridinium isomère syn ;
- le 6-/7-/2-(2-amino thiazol-4-yl) 2-méthoxyimino acétamido/ 2-carboxy 8-oxo 4-thia 1-azabicyclo /4.2.0/ oct-2-èn-3-yl) méthyl thiéno /2,3-c/ pyridinium isomère syn ;
- le 7-/2-(2-amino thiazol-4-yl) 2-méthoxyimino acétamido/ 8-oxo 2-carboxy 4-thia 1-azabicyclo /4.2.0/ oct-2-èn-3-yl/ méthyl/ triméthyl ammonium isomère syn ;
- le //7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ 4-cyclopropyl/ pyridinium isomère syn ;
- le //7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ 7-méthyl 6-thiéno /2,3-c/ pyridinium isomère syn ;
- le //7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ thiéno /2,3-b/ pyridinium isomère syn ;
- le /7-/2-(2-amino thiazol-4-yl) 2-(2-propényloxy) imino acétamido/ 2-carboxy 8-oxo 4-thia 1-azabicyclo /4.2.0/ oct-2-èn-3-yl/ méthyl pyridinium isomère syn ;
- le 1-//7-/2-(2-amino thiazol-4-yl) 2-méthoxyimino acétamido/ 2-carboxy 8-oxo 4-thia 1-azabicyclo /4.2.0/ oct-2-èn-3-yl/ méthyl (2-méthylthio)/ pyridinium isomère syn.

L'invention s'étend aux compositions pharmaceutiques renfermant comme principe actif au moins un des médicaments définis ci-dessus.

Ces compositions peuvent être administrées par voie buccale, rectale, parentérale, intramusculaire ou par voie locale en application topique sur la peau et les muqueuses.

En particulier, les produits de formule (I) dans laquelle A représente un ester clivable tel que l'ester de propionyl oxyméthyle peuventêtre administrés par voie orale.

Elles peuvent être solides ou liquides et se présenter sous les formes pharmaceutiques couramment utilisées en médecine humaine comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent, notamment, se présenter sous forme d'une poudre destinées à être dissoute extemporanément dans un véhicule appropriés, par exemple de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec les produits décrits aux exemples 23, 27, 28, 29, 33, 34 ou 53 ou encore comprise entre 0,500 g et 1 g trois fois par jour, par voie intramusculaire.

Les produits de formule (I) peuvent également être utilisés comme désinfectants des instruments chirurgicaux.

L'invention a enfin pour objet, à titre de produits industriels nouveaux et les produits de formule générale (III'A) :

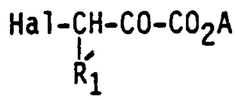

$(III'_A)$

dans laquelle $R_1$, A et Hal ont la signification indiquée ci-dessus, les produits de formule $(III'_{Ai})$ :

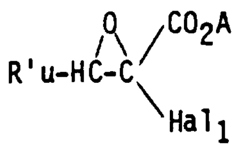

$(III'_{Ai})$

dans laquelle R'u, $Hal_1$ et A ont la signification indiquée ci-dessus, les produits de formule :

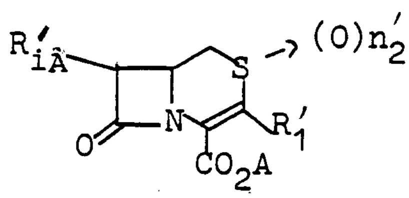

dans laquelle $R_1$, A et $n_2$ ont la signification indiquée ci-dessus et $R_{iA}$ représente un radical amino libre ou protégé par un groupement protecteur mono ou divalent, ainsi que les produits de formule (XI).

Les produits de formule $(IV_b)$ sont décrits ou peuvent être préparés selon le procédé du brevet français BF 2.073.338.

Les produits de formule $(IV_c)$ peuvent être préparés ou sont décrits dans la publicationJ. of Med. Chem. 1982, Vol 25, N° 4 p 457.

Les produits de formule $(II_A)$ sont décrits ou peuvent être préparés selon la méthode décrite dans le brevet belge 894.795.

Les produits de formule $(III'_{Ai})$ sont décrits ou peuvent être préparés selon la méthode décrite dans la publication J.O.C., 44, (25) 4741 (1983).

Les produits décrits ci-après dans les exemples illustrent l'invention sans toutefois la limiter.

EXEMPLE 1 : Acide 7-/2-(2-aminothiazol-4-yl) 2-méthoxyimino acétamido/8-oxo 4-thia 3-méthoxyméthyl 1-azabicyclo /4-2-0/ oct-2-ène 2-carboxylique syn cis racémique.

STADE A :méthoxyacétaldéhyde.

On porte au reflux 100 cm3 de diméthycétal du méthoxyacétal déhyde, 100 cm3 d'eau et 3,2 cm3 d'acide chlorhydrique concentré puis effectue plusieurs distillations fractionnées et obtient 8,7 g de produit attendu.

STADE B :2-chloro 3-méthoxyméthyl oxirane carboxylate de 1,1-diméthyl éthyle.

On refroidit à -20° C un mélange constitué de 2,106 g de méthoxyacétaldéhyde, 3,8 cm3 de dichloroacétate de terbutyle et 25 cm3 de tétrahydrofuranne. On introduit en 15 minutes 29 cm3 de terbutylate de potassium dans du tétrahydrofuranne (0,9 M/l) et laisse en contact 1 heure 20 minutes. On ajoute 25 cm3 d'éther et 25 cm3 d'eau et extrait à l'éther. On lave la phase organique à l'eau saturée en chlorure de sodium, sèche et concentre à sec sous pression réduite. On chromatographie sur silice le résidu obtenu, élue au chlorure de méthylène et obtient 1,44 g de produit attendu.

STADE C :7-/2-(2-tritylamino thiazol-4-yl) 2-méthoxyimino acétamido/ 8-oxo 2-hydroxy 3-méthoxyméthyl 4-thia 1-azabicyclo /4-2-0/ octane 2-carboxylate de 1,1-diméthyléthyle.

On mélange 1,38 g de 2-chloro 3-méthoxyméthyl oxirane carboxylate de 1,1-diméthyléthyle, 2,736 g de 4-mercaptométhyl 3-/2-(2-tritylaminothiazol-4-yl) 2-méthoxyimino acétamido/ 2-oxo 1-azétidine cis syn racémique préparé selon le procédé décrit dans le brevet belge 894.795 et 12 cm3 de diméthylformamide. Après 10 minutes de contact, on ajoute 458 mg de carbonate de lithium et agite pendant 2 heures 50 minutes. On verse le mélange réactionnel dans 100 cm3 d'eau et 60 cm3 d'acétate d'éthyle. On extrait à l'acétate d'éthyle, lave la phase organique à l'eau, sèche et concentre à sec sous pression réduite. On reprend le résidu à l'éther et obtient 3,058 g de produit attendu.

| Analyse : $C_{38}H_{41}O_7N_5S_2$ : 743,91 | | | | |
|---|---|---|---|---|
| Calculé<br>Trouvé | C% 61,35<br>61,1 | H% 5,56<br>5,7 | N% 9,41<br>8,9 | S% 8,62<br>8,4 |

STADE D : 7-/2-(2-tritylamino thiazol-4-yl) 2-méthoxy imino acétamido/ 8-oxo-3-méthoxy méthyl 4-thia 1-azabicyclo /4-2-0/ oct-2-ène 2-carboxylate de 1,1-diméthyléthyle.

On met en suspension 4,44 g de tétraiodure de diphosphore dans 35 cm3 de pyridine, agite 5 minutes et ajoute, en une seule fois, 3,058 g de 7-/2-(2-tritylamino thiazol-4-yl) 2-méthoxyimino acétamido/ 8-oxo 2-hydroxy 3-méthoxyméthyl 4-thia 1-azabicyclo/4-2-0/ octane 2-carboxylate de 1,1-diméthyléthyle et agite pendant 2 heures 40 minutes. On distille la pyridine, reprend le résidu avec 50 cm3 d'acétate d'éthyle, filtre, ajoute 50 cm3 d'acide chlorhydrique N au filtrat, agite vigoureusement et décante la phase organique, lave à l'eau, sèche, concentre à sec sous pression réduite. On chromatographie le résidu sur silice par un mélange chlorure de méthylène-acétate d'éthyle (85-15). On concentre à sec les fractions intéressantes, recristallise dans le méthanol et obtient 703mg de produit attendu.

| Analyse $C_{38}H_{39}O_6N_5S_2$ : 725,89 | | | | |
|---|---|---|---|---|
| Calculé<br>Trouvé | C% 62,88<br>62,7 | H% 5,41<br>5,4 | N% 9,65<br>9,6 | S% 8,83<br>8,8 |

STADE E :Acide 7/2-(2-aminothiazol-4-yl) 2-méthoxyimino acétamido/ 8-oxo 4-thia 3-méthoxyméthyl 1-azabicyclo /4-2-0/ oct-2-ène 2-carboxylique syn cis racémique.

On dissout 246 mg de 7-/2-(2-tritylaminothiazol-4-yl) 2-méthoxyimino acétamido 8-oxo 3-méthoxyméthyl 4-thia 1-azabicyclo /4-2-0/ oct-2-ène 2-carboxylate de 1,1-diméthyléthyle dans 1 cm3d'acide trifluoroacétique et laisse en contact pendant 50 minutes à température ambiante. On ajoute 12 cm3 d'éther isopropylique, filtre le précipité formé et obtient 176 mg de trifluoroacétate brut que l'on dissout dans l'éthanol. On ajoute 2 gouttes de pyridine, laisse cristalliser pendant 1 heure et obtient 88 mg de produit attendu.

| Analyse $C_{15}H_{17}O_6N_5S_2$ : 427,46 | | | | |
|---|---|---|---|---|
| Calculé<br>Trouvé | C% 42,15<br>42,2 | H% 4,01<br>4,0 | N% 16,38<br>16,2 | S% 15,00<br>15,0 |

EXEMPLE 2 : 4-oxyde de l'acide 7-/2-(2-aminothiazol-4-yl) 2-méthoxyimino acétamido/ 8-oxo 4-thia 3-méthoxyméthyl 1-azabicyclo /4-2-0/ oct-2-ène 2-carboxylique syn cis racémique.

STADE A :4-oxyde du 7-/2-(2-tritylamino thiazol-4-yl) 2-méthoxyimino acétamido/ 8-oxo-3-méthoxyméthyl 4-thia 1-azabicyclo /4-2-0/ oct-2-ène 2-carboxylate de 1,1-diméthyl-éthyle.

On dissout 660 mg de 7-/2-(2-tritylamino thiazol-4-yl) 2-méthoxyimino acétamido 8-oxo 3-méthoxy méthyl 4-thia 1-azabicyclo /4-2-0/ oct-2-ène 2-carboxylate de 1,1-diméthyléthyle et 257 mg d'acide métachloro perbenzoïque à 85 % dans 9 cm3 de chlorure de méthylène et agite pendant 1 heure. On ajoute de l'eau et 2 cm3 d'une solution de bicarbonate de sodium saturée. On extrait au chlorure de méthylène, sèche la phase organique et concentre à sec sous pression réduite. On chromatographie le résidu sur silice, élue par un mélange chlorure de méthylène-acétate d'éthyle (1-1) et obtient 515 mg de produit attendu isomère $\alpha$.

STADE B :4-oxyde de l'acide 7-/2-(2-aminothiazol-4-yl) 2-méthoxyimino acétamido/ 8-oxo 4-thia 3-méthoxyméthyl 1-azabicyclo /4-2-0/ oct-2-ène 2-carboxylique syn cis racémique.

On opère code au stade E de l'exemple 1 à partir de 515 mg d'isomère obtenu ci-dessus et 2,1 cm3 d'acide trifluoroacétique et obtient 168 mg de produit attendu.

| Analyse $C_{15}H_{17}O_7N_5S_2$ : 443,46 | | | | |
|---|---|---|---|---|
| Calculé | C% 40,63 | H% 3,86 | N% 15,79 | S% 14,46 |
| Trouvé | 40,7 | 3,9 | 15,5 | 14,2 |

EXEMPLE 3 : Acide 7-/2-(2-aminothiazol-4-yl) 2-méthoxyimino acétamido/ 8-oxo 3-/(2-pyridyl) thio/ 4-thia 1-azabicyclo /4-2-0/ oct-2-ène 2-carboxylique syn cis racémique.

STADE A :7-/2-(2-tritylamino thiazol-4-yl) 2-méthoxyimino acétamido/ 8-oxo 3-/ (2-pyridyl) thio/ 4-thia 1-azabicyclo /4-2-0/ octane-2-hydroxy 2-carboxylate de 1,1-diméthyléthyle.

Préparation du chlorure de 2-pyridyl sulfényle (selon DN Harp et coll Synthesis 181 (1979).

On refroidit à 0 ° C en agitant, 2,31 g de 2,2'-dipyridyl disulfure à 98 % dans 25 cm3 de tétrachlorure de carbone et 2 gouttes de triéthylamine, introduit en 1/2 heure, à 0 ° C, 1,489 g de chlorure de sulfuryle en solution dans 15 cm3 de tétrachlorure de carbone et agite 1/2 heure à 0 ° C. On décante la solution, évapore à sec sous pressionréduite et obtient 2,85 g de produit brut instable. On ajoute, en 1/4 heure, 1,60 g de chlorure de 2-pyridyl-sulfényle dans 25 cm3 de chlorure de méthylène à 1,70 g de 3-diazopyruvate de 1,1-diméthyléthyle dans 50 cm3 de chlorure de méthylène, agite pendant 45 minutes à température ambiante sous atmosphère inerte et obtient une solution de 3-chloro 3-(2-pyridyl)-thio-pyruvate de 1,1-diméthyléthyle à laquelle on ajoute en une fois 5,81 g de 4-mercaptométhyl 3-/2-(2-tritylamino thiazol-4-yl) 2-méthoxyimino acétamido/ 2-oxo 1-azétidine puis 1,5 cm3 de triéthylamine. On agite pendant 2 heures à température ambiante, essore l'insoluble et verse le filtrat sur 350 cm3d'eau et 10 cm3 d'acide chlorhydrique 2N. On décante, lave à l'eau la phase organique, traite au charbon actif et sèche, concentre à sec sous pression réduite. On chromatographie le résidu sur silice avec un mélange chlorure de méthylène-acétate d'éthyle (75-25) et obtient 4,22 g de produit attendu.

STADE B :7-/2-(2-tritylamino thiazol-4-yl) 2-méthoxy imino acétamido/ 8-oxo 3-/(2-pyridyl) thio/ 4-thia 1-azabicyclo /4-2-0/ oct-2-ène 2-carboxylate de 1,1-diméthyléthyle.

A 395 mg de produit obtenu ci-dessus, dissous dans 5 cm3 de pyridine, on ajoute 556 mg de tétraiodure de diphosphore et agite pendant 2 heures 30 minutes sous atmosphère inerte. On ajoute 5 cm3 d'acétate d'éthyle, filtre l'insoluble, évapore à sec le filtrat sous pression réduite et reprend le résidu par 25 cm3 de chlorure de méthylène. On lave à l'eau, sèche, concentre à sec sous pression réduite, chromatographie le résidu sur silice avec un mélange chlorure de méthylène-acétate d'éthyle et obtient 106,5 g de produit attendu F = 160 ° C.

STADE C : Acide 7-/2-(2-amino thiazol-4-yl) 2-méthoxy imino acétamido/ 8-oxo 3-/(2-pyridyl) thio/ 4-thia 1-azabicyclo /4-2-0/ oct-2-ène 2-carboxylique syn cis racémique.

On chauffe à 60°C pendant 2 heures 30 minutes 237,3 mg de produit obtenu au stade B et 3 cm3 d'acide formique à 66 %. On dilue avec 3 cm3 d'eau, essore l'insoluble et concentre à sec le filtrat à 60°C sous pression réduite. On reprend le résidu par 6 cm3 d'eau, évapore à nouveau et traite 2 fois par 6 cm3 du mélange d'acétonitrile-éthanol (1-1) en évaporant à chaque fois afin d'éliminer l'eau. On obtient 160 mg de produit que l'on reprend dans 7 cm3 d'éther anhydride et 0,3 cm3 d'acétonitrile, laisse reposer 1 heure, essore, lave à l'éther et obtient 120 mg de produit attendu F≃ 230°C (décomposition).
Spectre IR (Nujol)
Absence de trityle
Présence de lactame C=0 $1766^{cm-1}$
C=0 1663 $^{cm-1}$
Spectre UV
-dans EtOH
max1 : 335 nm
max : 299-300 nm
- dans Et OH - HCl 0,1N
infl : 310 nm

EXEMPLE 4 : Acide 7-/2-(2-amino thiazol-4-yl) 2-méthoxy imino acétamido/ 8-oxo 3-/(4-nitrophényl) méthyl thio/ 4-thia 1-azabicyclo /4-2-0/ oct-2-ène 2-caboxylique syn cis racémique.

STADE A :7-/2-(2-tritylamino thiazol-4-yl) 2-méthoxyimino acétamido/ 2-hydroxy 3-/(4-nitrophényl) méthyl thio/ 8-oxo 4-thia 1-azabicyclo /4-2-0/ octane 2-carboxylate de 1,1-diméthyléthyle syn cis racémique.

On opère comme au stade A de l'exemple 3 au départ de 925 mg de disulfure de 4,4'-dinitrobenzyle décrit dans Chem. Ber., 88,1995 (1955), 2,75 mmole de chlore, 1,12 g de diazopyruvate de 1,1-diméthyléthyle, 1,23 g de 3-/2-(2-tritylaminothiazol-4-yl) 2-méthoxyimino acétamido/ 2-oxo 1-azétidine cis syn racémique et 0,77 cm3 de triéthylamine. On obtient 1,07 g de produit attendu.

STADE B :7-/2-(2-tritylamino thiazol-4-yl) 2-méthoxyiminoacétamido/ 3-/(4 nitrophényl) méthyl thio/ 8-oxo 4-thia 1-azabicyclo/4-2-0/ oct-2-ène 2-carboxylate de 1,1-diméthyl éthyle syn cis racémique.

On opère comme au stade B de l'exemple 3 au départ de 922 mg de produit obtenu au stade A et 1,21 g de tétraiodure de diphosphore dans 10 cm3 de pyridine. On obtient, après chromatographie, 339 mg de produit attendu.

STADE C :Acide 7-/2-(2-amino thiazol-4-yl) 2-méthoxy imino acétamido/ 8-oxo 3-/(4-nitrophényl) méthyl thio/ 4-thia 1-azabicyclo /4-2-0/oct-2-ène 2-carboxylique syn cis racémique.

On agite 200 mg de produit obtenu au stade B dans 4 cm3 d'acide formique aqueux à 66 % pendant 3 heures. On refroidit, filtre et évapore sous pression réduite. On redissout dans 10 cm3 d'acétonitrile et 5 cm3 de méthanol. On évapore la solution, ajoute 5 cm3 de chlorure de méthylène et 1 cm3 de méthanol. On agite la suspension pendant la nuit, essore, sèche sous vide et obtient 103 mg de produit attendu.
Ultra-violet
- dans l'éthanol
infl : 233 nm $E_1^1$ = 434
infl : 257 nm $E_1^1$ = 356 $\epsilon$ =19600
max : 294 nm $E_1^1$ = 353 $\epsilon$ = 19400

EXEMPLE 5 : Acide 7-/2-(2-amino thiazol-4-yl) 2-méthoxy imino acétamido/ 8-oxo 3-(1-méthyl éthyle) 4-thia 1-azabicyclo /4-2-0/ oct-2-ène 2-carboxylique syn cis racémique.

STADE A :2-chloro 3-(1-méthyl éthyl) oxirane carboxylate de 1,1-diméthyl éthyle.

On refroidit à -20°C, 9,25 g de dichloroacétate de tertbutyle et 5 cm3 d'isobutyraldéhyde et ajoute en 20 minutes, 50 cm3 de terbutylate de potassium dans le tétrahydrofuranne 1M/l. On laisse remonter la

température du milieu pendant 1 h 25 et ajoute 50 cm3 d'eau et 50 cm3 d'éther. On extrait à l'éther, lave à l'eau saturée au chlorure de sodium la solution organique, sèche et concentre à sec sous pression réduite. On obtient 10,7 g de produit attendu.

STADE B :3-bromo 4-méthyl 2-oxo pentanoate de 1,1-diméthyléthyle.

On introduit 16,2 g de bromure de lithium anhydre dans 20,5 cm3 de tétrahydrofuranne. Après dissolution et refroidissement à 27° C, on ajoute 10,2 g de 2-chloro 3-(1-méthyléthyle) oxirane carboxylate de 1,1-diméthyl éthyle et laisse en contact 1/2 heure. On verse alors le mélange réactionnel dans 90 cm3 d'une solution aqueuse de chlorure de sodium à demi saturée et extrait à l'éther. On sèche la phase organique, concentre à sec sous pression réduite et obtient 8,9 g de produit attendu contenant 12 % d'homologue chloré.

STADE C :7-/2-(2-tritylaminothiazol-4-yl) 2-méthoxyimino acétamido/ 8-oxo 3-(1-méthyl éthyle) 4-thia-1-azabicyclo /4-2-0/ oct-2-ène-2-carboxylate de 1,1-diméthyléthyle.

On met en suspension et sous agitation 1,2 g de 4-mercaptométhyl 3-/2-(2-tritylamino thiazol-4-yl) 2-méthoxyimino acétamido/ 2-oxo 1-azétidine cis syn racémique, 15 cm3 de dichlorométhane et 636 mg de 3-bromo 4-méthyl 2-oxo pentanoate de 1,1-diméthyléthyle. On ajoute, goutte à goutte, 0,34 cm3 de triéthylamine. Après 1/2 heure, on ajoute 1,25 cm3 de pyridine puis, goutte à goutte, 2,67 cm3 d'une solution de tribromure de phosphore dans du chlorure de méthylène (0,48 cm3 $PBr_3$ dilué à 10 cm3 $Cl_2CH_2$) et laisse en contact 45 minutes. On ajoute 18 cm3 d'acide chlorhydrique N et extrait au chlorure de méthylène. On sèche la phase organique et concentre à sec sous pression réduite. Après chromatographie sur silice du résidu obtenu, en éluant au chlorure de méthylène à 10 % d'acétate d'éthyle, on recueille 374 mg de produit que l'on recristallise dans un mélange éther-éther isopropylique. On obtient 293 mg de produit attendu.

STADE D : Acide 7-/2-(2-aminothiazol-4-yl) 2-méthoxyimino acétamido/ 8-oxo 3-(1-méthyléthyle) 4-thia 1-azabicyclo /4-2-0/ oct-2-ène 2-carboxylique syn cis racémique.

On mélange 455 mg de produit obtenu au stade précédent et 1,82 cm3 d'acide trifluoroacétique et laisse au repos pendant 20 minutes. On ajoute 25 cm3 d'éther isopropylique et essore le précipité formé puis le dissout dans 2 cm3 d'eau contenant 1 cm3 de bicarbonate de sodium en solution saturée, agite, filtre l'insoluble et ajoute au filtrat de l'acide chlorhydrique 2N jusqu'à pH 4. Après 1 heure, on filtre, rince à l'eau puis à l'éther et obtient 73 mg de produit attendu.

| Analyse $C_{16}H_{19}O_5H_5S_2$ : 425,49 | | | | |
|---|---|---|---|---|
| Calculé<br>Trouvé | C% 45,17<br>44,6 | H% 4,50<br>4,4 | N% 16,46<br>16,1 | S% 15,07<br>14,2 |

Spectre UV
- dans EtOH
  max : 225 nm $E_1^1$ : 465 $\epsilon$ = 19800
  max : 296 nm $E_1^1$ : 396 $\epsilon$ = 16800

EXEMPLE 6 : 4-oxyde de l'acide 7-/2-(2-aminothiazol-4-yl) 2-méthoxyimino acétamido/ 8-oxo 4-thia 3-(1-méthyl éthyl) 1-azabicyclo /4-2-0/ oct-2-ène-2-carboxylique syn cis racémique.

STADE A :4-oxyde de l'acide 7-/2-(2-tritylaminothiazol-4-yl) 2-méthoxyimino acétamido/ 8-oxo 4-thia 3-(1-méthyl éthyl) 1-azablcyclo /4-2-0/ oct-2-ène 2-carboxylate de 1,1-diméthyl éthyle.

On opère comme à l'exemple 2en partant de 602 mg de 7-/2-(2-tritylamino thiazol-4-yl) 2-méthoxyimino acétamido/ 8-oxo 4-thia 3-(1-méthyléthyl) 1-azabicyclo /4-2-0/ oct-2-ène 2-carboxylate de 1,1-diméthyl éthyle et de 235 mg d'acide métachloro perbenzoïque et obtient 317 mg de produit attendu.

STADE B : 4-oxyde de l'acide 7-/2-(2-aminothiazol-4-yl) 2-méthoxyimino acétamido/ 8-oxo 4-thia 3-(1-méthyléthyl) 1-azabicyclo /4-2-0/ oct-2-ène 2-carboxylique syn cis racémique.

On opère comme au stade E de l'exemple 1 à partir de 307 mg de produit obtenu ci-dessus et de 1,3 cm3 d'acide trifluoroacétique. On obtient 148 mg de produit attendu.

| Analyse $C_{16}H_{19}O_6N_5S_2$ : 441,49 | | | | |
|---|---|---|---|---|
| Calculé<br>Trouvé | C% 43,53<br>43,9 | H% 4,34<br>4,4 | N% 15,86<br>15,5 | S% 14,52<br>14,1 |

EXEMPLE 7 : Acide 7-/2-(2-aminothiazol-4-yl) 2-méthoxyimino acétamido/ 8-oxo 4-thia 3-éthyl 1-azabicyclo /4-2-0/ oct-2-ène 2-carboxylique syn cis racémique.

STADE A :2-chloro 3-éthyl oxirane carboxylate de 1,1-diméthyl éthyle.

On mélange 4 cm3 de dichloroacétate de tertbutyle et 2 cm3 de propanol dilué dans 25 cm3 de tétrahydrofuranne, refroidit à -20 °C et introduit en 12 minutes à cette température 28 cm3 de terbutylate de potassium 0,9 M/1 dans le tétrahydrofuranne. On laisse remonter la température à 20 °C pendant 1 heure 20 minutes et ajoute 25 cm3 d'éther et 25 cm3 d'eau, agite, décante et réextrait à l'éther. On lave la phase organique à l'eau saturée en chlorure de sodium, sèche, concentre à sec et obtient 4,7 g de produit attendu.

STADE B :7-/2-(2-tritylaminothiazol-4-yl) 2-méthoxyimino acétamido/ 8-oxo 3-éthyl 2-hydroxy 4-thia 1-azabicyclo /4-2-0/ octane 2-carboxylatede 1,1-dyméthyl éthyle.

On agite 1,157 g de 2-chloro 3-éthyl oxirane carboxylate de 1,1-diméthyl éthyle et 2,39 g de 4-mercaptométhyl 3-/2-(2-tritylaminothiaxol-4-yl) 2-méthoxyimino acétamido/ 2-oxol-azétidine cis syn racémique dans 10 cm3 de diméthylformamide, ajoute 414 mg de carbonate de lithium et laisse en contact pendant 2 heures. On verse le mélange réactionnel dans 100 cm3 d'eau, extrait à l'acétate d'éthyle. On lave la phase organique à l'eau, sèche, concentre à sec sous pression réduite. On reprend le résidu à l'éther isopropylique et après quelques heures, filtre et isole 3,197 g de produit attendu.

| Analyse $C_{38}H_{41}O_6N_5S_2$ : 727,91 |
|---|

STADE C :7-/2-(2-tritylaminothiazol-4-yl) 2-méthoxyimino acétamido/ 8-oxo 3-éthyl 4-thia 1-azabicyclo /4-2-0/ oct-2-ène 2-carboxylate de 1,1-diméthyl éthyle.

On opère comme à l'exemple 1 en partant de 560 mg de tétraiodure de diphosphore, 4,5 cm3 de pyridine et 394 mg du produit obtenu ci-dessus et obtient 125 mg de produit attendu.

| Analyse $C_{38}H_{39}O_5N_5S_2$ : 709,89 | | | | |
|---|---|---|---|---|
| Calculé<br>Trouvé | C% 64,29<br>63,9 | H% 5,54N%<br>5,6 | 9,86<br>9,7 | S% 9,03<br>8,8 |

STADE D :Acide 7-/2-(2-aminothiazol-4-yl) 2-méthoxyimino acétamido/ 8-oxo 4-thia 3-éthyl 1-azabicyclo /4-2-0/ oct-2-ène 2-carboxylique syn cis racémique.

On obtient 155 mg de produit attendu en opérant comme au stade E de l'exemple 1 à partir de 489 mg de produit obtenu au stade C, 1,95 cm3 d'acide trifluoroacétique.

EXEMPLE 8 : 4-oxyde de l'acide 7-/2-(2-aminothiazol-4-yl) 2-méthoxyimino acétamido/ 3-éthyl 8-oxo 4-thia 1-azabicyclo /4-2-0/ oct-2-ène-2-carboxylique syn cis racémique.

STADE A :4-oxyde de 7-/2-(2-tritylaminothiazol-4-yl) 2-méthoxyimino acétamido/ 3-éthyl 8-oxo 4-thia 1-azabicyclo /4-2-0/ oct-2-ène 2-carboxylate de 1,1-diméthyl éthyle.

En utilisant la même technique qu'à l'exemple 2 à partir de 517 mg de 7-/2-(2-tritylamino thiazol-4-yl) 2-méthoxyimino acétamido/ 8-oxo 3-éthyl 4-thia 1-azabicyclo /4-2-0/ oct-2-ène 2-carboxylate de 1,1-diméthyl éthyle, on obtient 387 mg de produit attendu.

STADE B :4-oxyde de l'acide 7-/2-(2-aminothiazol-4-yl) 2-méthoxyimino acétamido/ 3-éthyl 8-oxo 4-thia 1-azabicyclo /4-2-0/ oct-2-ène-2-carboxylique syn cis racémique.

A partir de 366 mg de produit obtenu ci-dessus en opérant comme à l'exemple 1, stade E, on obtient 86 mg de produit attendu.

| Analyse $C_{15}H_{17}O_6N_5S_2$ : 427,46 | | | | |
|---|---|---|---|---|
| Calculé<br>Trouvé | C% 42,15<br>42,6 | H% 4,01<br>4,2 | N% 16,38<br>16 | S% 15,00<br>14,1 |

Spectre UV
- dans EtOH/HCl 0,1N
max : 272 nm $E_1^1$ : 527 $\epsilon$ = 22500

EXEMPLE 9 : Acide 7-/2-(2-aminothiazol-4-yl) 2-méthoxyimino acétamido/ 8-oxo 3-(2-pyridyl) 4-thia 1-azabicyclo /4-2-0/ oct-2-ène 2-carboxylique cis syn racémique.

STADE A :2-chloro 3-(2-pyridyl) oxirane carboxylate de 1,1-diméthyl éthyle.

On mélange 4 cm3 de dichloroacétate de tertbutyle dans 15 cm3 de tétrahydrofuranne et 2,6 cm3 de picolinaldéhyde, refoidit à -20°C/-25°C et introduit en 15 minutes à cette température 28 cm3 de terbutylate de potassium dans le tétrahydrofuranne à 0,9M/l. On laisse le mélange se rèchauffer à température ambiante. Après 1 heure 25 minutes, on ajoute 25cm3 d'eau, extraitau tétrahydrofuranne. On lave la phase organique à l'eau saturée en chlorure de sodium, sèche et conserve à -25°C la solution contenant le produit attendu utilisée pour la suite de la synthèse.

STADE B :3-/3-/(2-tritylaminothlazol-4-yl) 2-méthoxyimino acétamido/ 2-oxo 4-azétidinyl méthyl thio/ 3-(1,2-dihydro 2-pyridinylidène) pyruvate de 1,1-diméthyl éthyle.

On dissout 2,39 g de 4-mercaptométhyl 3-/2-(2-tritylaminothiazol-4-yl) 2-méthoxyimino acétamido/ 2-oxo 1-azétidine cis syn racémique dans 22 cm3 de diméthyl formamide, ajoute 12,8 cm3 de la solution obtenue ci-dessus puis 480 mg de carbonate de lithium et agite pendant 2 heures. On distille le solvant, ajoute 100 cm3 d'eau, extrait à l'acétate d'éthyle, lave la phase organique à l'eau, sèche et concentre à sec sous pression réduite. On reprend le résidu à l'éthanol, fait cristalliser pendant 2 heures et isole 2,357 g de produit attendu.

| Analyse $C_{41}H_{40}O_6N_6S_2$ : 776,94 | | | | |
|---|---|---|---|---|
| Calculé<br>Trouvé | C% 63,47<br>63,3 | H% 5,07<br>5,2 | N% 10,83<br>10,7 | S% 8,26<br>8,0 |

STADE C :7-/2-(2-tritylaminothiazol-4-yl) 2-méthoxyimino acétamido 8-oxo 2-hydroxy 3-(2-pyridyl) 4-thia 1-azabicyclo /4-2-0/ octane 2-carboxylate de 1,1-diméthyl éthyle.

On dissout 3,053 g de produit obtenu ci-dessus et 1,3 g de triéthylène diamine dans 30 cm3 de chlorure de méthylène, agite pendant 1 heure 30, ajoute 28 cm3 d'acide chlorhydrique N et extrait au chlorure de méthylène. On sèche la phase organique et concentre à sec sous pression réduite. On chromatographie le résidu sur silice avec un mélange chlorure de méthylèneacétate d'éthyle, recueille les fractions riches en produit attendu, chasse les solvants et reprend les cristaux formés dans l'éther. On obtient 1,885g de produit attendu.

STADE D :7-/2-(2-tritylaminothiazol-4-yl) 2-méthoxyimino acétamido/ 8-oxo 3-(2-pyridyl) 4-thia 1-azabicyclo /4-2-0/ oct-2-ène 2-carboxylate de 1,1-diméthyl éthyle.

On opère comme au stade D de l'exemple 1 à partir de 743 mg de produit obtenu précédemment et recristallise le produit brut obtenu dans l'acétate dans l'acétate d'éthyle. On obtient 284 mg de produit attendu.

| Analyse $C_{41}H_{38}O_5N_6S_2$ : 758,92 | | | | |
|---|---|---|---|---|
| Calculé<br>Trouvé | C% 64,88<br>64,5 | H% 5,05<br>5,1 | N% 11,07<br>10,9 | S% 8,45<br>8,2 |

STADE E : Acide 7-/2- (2-aminothiazol-4-yl) 2-méthoxyimino acétamido/ 8-oxo 3-(2-pyridyl) 4-thia 1-azabicyclo /4-2-0/ oct-2-ène 2-carboxylique.

On opère comme au stade E de l'exemple 1 à partir de 413 mg de produit obtenu au stade précédent et obtient 343 mg de trifluoroacétate que l'on dissout dans 1,5 cm3 d'une solution aqueuse saturée de bicarbonate de sodium. On filtre, acidifie avec de l'acide chlorhydrique 2N jusqu'à pH 4, distille une partie de l'eau et laisse cristalliser pendant 1 heure. On filtre, rince à l'eau et obtient 131 mg de produit attendu.

| Analyse $C_{18}H_{16}O_5N_6S_2$ : 460,49 | | | | |
|---|---|---|---|---|
| Calculé<br>Trouvé | C% 46,95<br>46,4 | H% 3,50<br>3,4 | N% 18,25<br>17,9 | S% 13,93<br>13,5 |

EXEMPLE 10 : 4-oxyde de l'acide 7-/2-(2-aminothiazol-4-yl) 2-méthoxyimino acétamido/ 8-oxo 3-(2-pyridyl 4-thia 1-azabicyclo /4-2-0/ oct-2-ène 2-carboxylique cis syn racémique.

STADE A :4-oxyde du 7-/2-(2-tritylaminothiazol-4-yl) 2-méthoxyimino acétmido/ 8-oxo 3-pyridyl 4-thia 1-azabicyclo /4-2-0/ oct-2-ène 2-carboxylate de 1,1-diméthyl éthyle.

On opère comme à l'exemple 2 à partir de 537 mg de produit obtenu à l'exemple 9 et obtient 243 mg de produit attendu correspondant à l'isomère .

STADE B :4-oxyde de l'acide 7-/2-(2-aminothiazol-4-yl) 2-méthoxyimino acétamido/ 8-oxo 3-(2-pyridyl) 4-thia 1-azabicyclo /4-2-0/ oct-2-ène 2-carboxylique cis syn racémique.

On opère comme au stade E de l'exemple 1, on dissout le trifluoroacétate obtenu dans le méthanol ajoute de la triéthylamine jusqu'à pH 4, laisse cristalliser pendant 15 minutes et obtient 92 mg de produit attendu.

| Analyse $C_{18}H_{16}O_6N_6S_2$ : 476,49 | | | | |
|---|---|---|---|---|
| Calculé<br>Trouvé | C% 45,37<br>45,3 | H% 3,38<br>3,6 | N% 17,64<br>16,9 | S% 13,46<br>12,9 |

EXEMPLE 11 : Acide 7-/2-(2-aminothiazol-4-yl) 2-méthoxyimino acétamido/ 3-phényl 8-oxo 4-thia 1-azabicyclo /4-2-0/ oct-2-ène 2-carboxylique syn cis racémique.

STADE A :Acide 3-bromo 3-phényl pyruvique.

On mélange 12,31 g d'acide phényl pyruvique et 50 cm3 d'acide acétique, ajoute goutte a goutte en 1 heure une solution de 12,34 g de brome dans 4 cm3 d'acide acétique. On agite encore 15 minutes après la fin d'introduction et concentre à sec sous pression réduite. On reprend le résidu par 40 cm3 de dichloroéthane, concentre à sec sous pression réduite, ajoute 30 cm3 de dichloroéthane et laisse cristalliser pendant 2 heures à 20 ° C. On obtient 12,6 g de produit attendu f = 110 ° C.

STADE B :3-bromo 3-phényl pyruvate de 1,1-diméthyl éthyle.

On refroidit à 0 + 5 ° C 12,05 g d'acide 3-bromo 3-phényl pyruvique dans 120 cm3 d'acétate d'éthyle, introduit en 20 minutes 14,9 g de O-tertbutyle isourée dans 10 cm3 d'acétate d'éthyle et agite pendant 1 heure à 0 + 5 ° C puis 3 heures à 20 ° C. On filtre l'urée formée, lave la solution d'acétate d'éthyle par une solution aqueuse saturée en bicarbonate de sodium puis à l'eau, sèche et concentre à sec sous pression réduite. On empâte le résidu à l'éther, amène à sec et chromatographie sur silice le résidu obtenu par un mélange chlorure de méthylène-acétate d'éthyle (70-30) et isole 3,14 g de produit attendu.

| Analyse $C_{13}H_{15}BrO_3$ : 299,172 | | | |
|---|---|---|---|
| Calculé<br>Trouvé | C% 52,15<br>53,4 | H% 5,05<br>5,4 | Br% 26,71<br>23,6 |

Spectre IR

Présence de $\phi CH_3$
C = 0 1746 $cm^{-1}$
1723 $cm^{-1}$
Ester terbutylique 1372 $cm^{-1}$
1155 $cm^{-1}$

STADE C :7-/2-(2-tritylaminothiazol-4-yl) 2-méthoxyimino acétamido/ 3-phényl 8-oxo 4-thia 1-azabicyclo /4-2-0/ oct-2-ène 2-carboxylate de 1,1-diméthyl éthyle.

Dans une solution constituée par 6,13 g de 4-mercapto méthyl 3-/2-(2-tritylaminothiazol-4-yl) 2-méthoxyimino acétamido/ 2-oxo 1-azétidine(cis syn racémique) et 120 cm3 de chlorure de méthylène, on introduit sous agitation 3,94 g de 3-bromo 3-phényl pyruvate de 1,1-diméthyl éthyle et 1,93 cm3 de triéthylamine. On agite pendant 20 minutes à 20 ° C, ajoute 7,5 cm3 de pyridine pure, puis en 15 minutes, 2,98 g de tribromure de phosphore dans 20 cm3 de chlorure de méthylène et agite encore pendant 20 minutes. On verse le mélange réactionnel dans 1 litre d'eau et de glace et extrait au chlorure de méthylène. On lave la phase organique à l'eau, sèche et concentre à sec sous pression réduite. Le résidu est purifié par chromatographie en éluant par un mélange chlorure de méthylène-acétate d'éthyle (85-15). On isole 1,14 g de produit attendu.

Spectre UV

- dans l'éthanol ( + 2 cm3 DMSO pour dissoudre)
max : 310 nm $E_1^1$ : 203 $\epsilon$ = 15300

- dans l'éthanol HCl 0,1N
infl : 284 nm $E_1^1$ : 246 $\epsilon$ = 18500
max : 293 nm $E_1^1$ : 256 $\epsilon$ = 19200
infl : 302 nm $E_1^1$ : 246 $\epsilon$ = 18500
infl: 315 nm $E_1^1$ : 187 $\epsilon$ = 14000

STADE D :Acide 7-/2-(2-aminothiazol-4-yl) 2-méthoxyimino acétamido/ 3-phényl 8-oxo 4-thia 1-azabicyclo /4-2-0/ oct-2-ène 2-carboxylique syn cis racémique.

On agite pendant 20 minutes à 20°C 400 mg de produit obtenu au stade précédent et 2 cm3 d'acide trifluoroacétique et ajoute goutteà goutte en 10 minutes, 20 cm3 d'éther isopropylique, agite encore 20 minutes, essore, sèche et obtient 297 mg de trifluoroacétate. Celui-ci est repris par 2 cm3 d'éthanol, on ajoute 0,046 cm3 de pyridine puis 2 cm3 d'éthanol et agite 30 minutes. On obtient 138 mg de produit attendu F 220°C (décomposition). Spectre UV

1) dans l'éthanol (+1 cm3 DMSO pour dissoudre)
max : 308 nm $E_1^1$ : 276 $\epsilon$ = 14200
2) dans l'éthanol HCl 0,1N
max : 248 nm $E_1^1$ : 378 $\epsilon$ = 19500
infl : 254 nm $E_1^1$ : 370
max : 283 nm $E_1^1$ : 296 $\epsilon$ = 15300
infl : 309 nm $E_1^1$ : 234 $\epsilon$ = 12000

EXEMPLE 12 : 4-oxyde de l'acide 7-/2-(2-aminothiazol-4-yl) 2-méthoxyimino acétamido/ 3-phényl 8-oxo 4-thia 1-azabicyclo /4-2-0/ oct-2-ène 2-carboxylique syn cis racémique.

STADE A :4-oxyde de 7-/2-(2-tritylaminothiazol-4-yl) 2-méthoxyimino acétamido/ 3-phényl 8-oxo 4-thia 1-azabicyclo /4-2-0/ oct-2-ène 2-carboxylate de 1,1-diméthyl éthyle.

On dissout 600 mg de 7-/2-(2-tritylaminothiazol-4-yl) 2-méthoxyimino acétamido/ 8-oxo 3-phényl 4-thia 1-azabicyclo /4-2-0/ oct-2-ène 2-carboxylate de 1,1-diméthyl éthyle dans 20 cm3 de chlorure de méthylène, ajoute en une fois 193 mg d'acide métachloro perbenzoïque à 85 % et agite pendant 40 minutes. On ajoute 10 cm3 d'une solutiond'hyposulfite de sodium 0,5M, agite 10 minutes, décante et lave la phase organique avec une solution saturée de bicarbonate de sodium, sèche et concentre à sec sous pression réduite. On chromatographie sur silice le résidu, élue par un mélange chlorure de méthylène-acétate d'éthyle (60-40) et obtient 59 mg d'isomère et 364 mg d'isomère $\beta$ F≃200°C (décomposition).

| Analyse $C_{42}H_{39}H_5O_6S_2$ : 773,936 | | | | |
|---|---|---|---|---|
| Calculé<br>Trouvé | C% 65,18<br>64,6 | H% 5,08<br>5,2 | N% 9,05<br>8,6 | S% 8,29<br>8,1 |

Spectre UV

1) dans EtOH
infl : 224 nm $E_1^1$ : 403 $\epsilon$ = 19200
maxi : 299 nm $E_1^1$ : 322 $\epsilon$ = 15300
2) dans EtOH/HCl 0,1N
infl : 221 nm $E_1^1$ : 310
maxi : 283 nm $E_1^1$ : 370 $\epsilon$ = 17600
infl : 309 nm $E_1^1$ : 243 $\epsilon$ = 11500

STADE B : 4-oxyde de l'acide 7-/2-(2-aminothiazol-4-yl) 2-méthoxyimino acétamido/ 3-phényl 8-oxo 4-thia 1-azabicyclo /4-2-0/ oct-2-ène 2-carboxylique syn cis racémique.

On opère comme au stade D de l'exemple 11 à partir de 325 mg de produit obtenu ci-dessus et 1,7 cm3 d'acide trifluoroacétique, ajoute 15 cm3 d'éther isopropylique, agite pendant 20 minutes à 15-20°C pour précipiter 176 mg de produit attendu brut. On empâte celui-ci à l'acétate d'éthyle, chromatographie

sur silice par un mélange chlorure de méthylène-méthanol (92-8) et concentre à sec la fraction intéressante. On reprend le résidu à l'acétate d'éthyle, essore et obtient 55 mg de produit attendu.

Spectre UV

1) dans EtOH
infl : 224 nm $E_1^1$ : 403 $\epsilon$ = 19200
max : 299 nm $E_1^1$ : 322 $\epsilon$ = 15300
2) dans EtOH/HCl 0,1N
infl : 221 nm $E_1^1$ : 310
max : 283 nm $E_1^1$ : 370 $\epsilon$ = 17600
infl : 309 nm $E_1^1$ : 243 $\epsilon$ = 11500

EXEMPLE 13 : Acide 7/2-(2-aminothiazol-4-yl) 2-méthoxyiminoacétamido 3-(4-nitrophényl) 8-oxo 4-thia 1-azabicyclo /4-2-0/ oct-2-ène 2-carboxylique cis syn racémique.

STADE A :2-chloro 3-(4-nitrophényl) oxirane carboxylate de 1,1-diméthyl éthyle.

On refroidit à -20°C, 5,55 g de dichloroacétate de tertbutyle dans 30 cm3 de tétrahydrofuranne et 4,99 g de 4-nitrobenzaldéhyde dans 30 cm3 de tétrahydrofuranne et ajoute, goutte à goutte à -20°C et en 20 minutes, 30 cm3 d'une solution molaire de terbutylate de potassium dans le tétrahydrofuranne. On agite en laissant remonter la température à 20°C et verse le mélange réactionnel dans 100 cm3 d'eau et de glace, extrait à l'éther. On lave la phase organique à l'eau saturée de chlorure de sodium puis à l'eau, sèche et concentre à sec sous pression réduite. On chromatographie le résidu sur silice au chlorure de méthylène et isole 6,44 g de produit F=60°C.

STADE B :7-/2-(2-tritylaminothiazol-4-yl) 2-méthoxyimino acétamido/ 2-hydroxy 3-(4-nitrophényl) 8-oxo 4-thia 1-azabicyclo /4-2-0/ octane 2-carboxylatede 1,1-diméthyl éthyle.

On dissout 4,32 g de produit obtenu ci-dessus dans 130 cm3 de diméthyl formamide, ajoute 7,24 g de 4-mercaptométhyl 3-/(2-tritylaminothiazol-4-yl) 2-méthoximino acétamido/ 2-oxo 1-azétidine cis syn racémique, agite 5 minutes et introduit en une seule fois 1,06 g de carbonate de lithium. On agite pendant 1 heure, verse le mélange réactionnel dans 1,2 litre d'eau et de glace, extrait à l'acétate d'éthyle puis lave à l'eau, sèche et concentre à sec sous pression réduite. On chromatographie le résidu sur silice avec un mélange chlorure de méthylène-acétate d'éthyle (85-15) et isole 7,4 g de produit. On dissout celui-ci dans un mélange de 30 cm3 d'éther isopropylique et 15 cm3 de chlorure de méthylène au reflux et évapore le chlorure de méthylène. Le produit attendu cristallise à chaud. Après 1 heure à 20°C, on essore et obtient 6,074 g de produit attendu cristallisé F=265°C.

STADE C :7-/2-(2-tritylaminothiazol-4-yl) 2-méthoxyimino acétamido 3-(4-nitrophényl) 8-oxo 4-thia 1-azabicyclo /4-2-0/ oct-2-ène 2-carboxylate de 1,1-diméthyléthyle.

On dissout 7,41 g de produit obtenu comme ci-dessus dans 74 cm3 de pyridine puis introduit en 2 fractions sensiblement égales, à 10 mn d'intervalle, 10,28 g de tétraiodure de diphosphore. On refroidit à 20°C et agite pendant 50 minutes. On verse le mélange réactionnel dans 1 litre d'eau et glace, amène le pH à 4 par addition lente et 260 cm3 d'acide chlorhydrique 2N et extrait à l'acétate d'éthyle. On lave la phase organique à l'eau, sèche et concentre à sec sous pression réduite. On chromatographie le résidu sur silice par un mélange chlorure de méthylène-acétate d'éthyle (85-15). Après isolement du produit intéressant, on le reprend au reflux par un mélange de 30 cm3 d'éther isopropylique et 20 cm3 de chlorure de méthylène, puis distille le chlorure de méthylène et obtient la cristallisation à chaud. On laisse revenir à température ambiante et après une heure de repos à 20°C on essore le produit attendu, soit 3,97 g F=197°C.

STADE D :Acide 7-/2-(2-aminothiazol-4-yl) 2-méthoxyimino acétamido 3-(4-nitrophényl) 8-oxo 4-thia 1-azabicyclo /4-2-0/ oct-2-ène 2-carboxylique cis syn racémique.

On agite sous atmosphère inerte pendant 50 minutes 800 mg de produit obtenu ci-dessus dans 4 cm3 d'acide trifluoroacétique puis on ajoute lentement 50 cm3 d'éther-isopropylique. On essore le précipité

formé, ajoute 3,5 cm3 d'acide trifluoroacétique et agite pendant 3 heures. On rajoute 50 cm3 d'étherisopropylique, agite pendant 10 minutes. On essore le trifluoroacétate, le reprend par 10 cm3 d'éthanol, agite 10 minutes, ajoute 0,2 cm3 de pyridine et agite encore 15 minutes. On essore le produit puis le dissout dans 15 cm3 d'eau saturée en bicarbonate de sodium. On amène le pH à 2 par addition d'acide chlorhydrique 2N. Après 15 minutes, on essore, lave à l'eau et obtient 200 mg de produit attendu F>260 ° C.

EXEMPLE 14 : 4-oxyde de l'acide 7-/2-(2-aminothiazol-4-yl) 2-méthoxyimino acétamido 3(4-nitrophényl) 8-oxo 4-thia 1-azabicyclo /4-2-0/ oct-2-ène 2-carboxylique cis syn racémique.

STADE A : 4-oxyde de 7-/2-(2-tritylaminothiazol-4-yl) 2-méthoxyimino acétamido/ 3-(4-nitrophényl) 8-oxo 4-thia 1-azabicyclo /4-2-0/ oct-2-ène 2-carboxylate de 1,1-diméthyl éthyle.

On agite 1,9 g de 7/2-(2-tritylamino thiazol-4-yl) 2-méthoxyimino acétamido/ 3-(4-nitrophényl) 8-oxo 4-thia 1-azabicyclo /4-2-0/ oct-2-ène 2-carboxylate de 1,1-diméthyl éthyle dans 57 cm3 de chlorure de méthylène et 750 mg d'acide métachloroperbenzoïque à 85 % pendant 2 heures. On dilue au chlorure de méthylène et ajoute 5 cm3 d'une solution aqueuse à 0,5 M/l d'hyposulfite de sodium, agite 5 minutes et décante. On lave la phase organique dans 15 cm3 d'une solution aqueuse saturée en bicarbonate de sodium puis à l'eau, sèche et concentre à sec sous pression réduite. Le résidu est chromatographie sur silice. On élue par un mélange de chlorure de méthylène-acétate d'éthyle (75-25). Chacun des 2 isomères isolés est recristallisé dans l'éther isopropylique. On obtient 110 mg d'isomère $\beta$ F≃180 ° C (fusion pâteuse) et 592 mg d'isomère $\alpha$ F = 210 ° C (décomposition).

STADE B :Acide 7-/2-(2-aminothiazol-4-yl) 2-méthoxyimino acétamido 3-(4-nitrophényl 8-oxo 4-thia 1-azabicyclo /4-2-0/ oct-2-ène 2-carboxylique cis syn racémique.

On agite 200 mg de produit obtenu ci-dessus (isomère $\alpha$ ) avec 1 cm3 d'acide trifluoroacétique pendant 2 heures puis précipite le trifluoroacétate par addition lente de 20 cm3 d'éther isopropylique. Après 20 minutes, on essore et reprend le trifluoroacétate dans 2 cm3 d'éthanol et 8 cm3 de méthanol. On ajoute en 2 fois 0,04 cm3 de pyridine, laisse cristalliser pendant 1 heure et obtient 82 mg de produit attendu.

| Analyse $C_{19}H_{16}N_6O_8N_2$ : 520,503 | | | | |
|---|---|---|---|---|
| Calculé<br>Trouvé | C% 43,84<br>C% 44,3 | H% 3,10<br>H% 3,1 | N% 16,15<br>N% 15,7 | S% 12,32<br>S% 11,6 |

Spectre UV

1) dans EtOH
maxi : 234 nm $E_1^1$ : 369 $\epsilon$ = 19200
infl : 308 nm $E_1^1$ : 264
maxi : 327 nm $E_1^1$ : 283 $\epsilon$ = 14700
2) dans EtOH/HCl 0,1N
maxi : 270 nm $E_1^1$ : 361 $\epsilon$ = 18800
infl : 282 nm $E_1^1$ : 352 $\epsilon$ = 18300
maxi : 322 nm $E_1^1$ : 279 $\epsilon$ = 14500

EXEMPLE 15 : Acide 7/2-(2-aminothiazol-4-yl) 2-méthoxyimino acétamido/ 3-(4-chlorophényl) 8-oxo 4-thia 1-azabicyclo /4-2-0/ oct-2-ène 2-carboxylique syn cis racémique.

STADE A :2-chloro 3-(4-chlorophényl) oxirane carboxylate de 1,1-diméthyl éthyle.

On agite 5,55 g de dichloroacétate de terbutyle et 30 cm3 de tétrahydrofuranne, ajoute 4,64 g de 4-chlorobenzaldéhyde puis refroidit à -20 ° C pour ajouter 30 cm3 de solution N de terbutylate de potassium dans le tétrahydrofuranne. On laisse remonter la température à 20 ° C et après 20 minutes, on ajoute 50 cm3 d'éther et 40 cm3 d'eau saturée en chlorure de sodium, agite 5 minutes et décante. On lave la phase organique à l'eau saturée en chlorure de sodium, sèche et concentre à sec sous pression réduite. On chromatographie le résidu sur silice au chlorure de méthylène et obtient 1,90 g de produit attendu.

| Analyse $C_{13}H_{14}Cl_2O_3$ | | | |
|---|---|---|---|
| Calculé<br>Trouvé | C% 54,00<br>52,8 | H% 4,88<br>4,9 | Cl% 24,52<br>24,6 |

STADE B :7-/2-(2-tritylaminothiazol-4-yl) 2-méthoxyimino acétamido/ 2-hydroxy 3-(4-chlorophényl) 8-oxo 4-thia 1-azabicyclo /4-2-0/ octane2-carboxylate de 1,1-diméthyl éthyle.

On opère comme au stade de l'exemple 13 à partir de 4,86 g de produit obtenu précédemment et obtient 8,37 g de produit attendu cristallisé F=191°C après purification par chromatographie sur silice (éluant : $CH_2Cl_2$-ACOEt 85-15).

STADE C :7-/2-(2-tritylaminothiazol-4-yl) 2-méthoxyimino acétamido/ 3-(4-chlorophényl) 8-oxo 4-thia 1-azabicyclo /4-2-0/ oct-2-ène 2-carboxylate de 1,1-diméthyléthyle syn cis racémique.

On opère comme au stade C de l'exemple 13 à partir de 8,060 g de produit obtenu ci-dessus et obtient 3,680 g de produit attendu F=183°C.

STADE D :Acide 7-/2-(2-aminothiazol-4-yl) 2-méthoxyimino acétamido/ 3-(4-chlorophényl) 8-oxo 4-thia 1-azabicyclo /4-2-0/ oct-2-ène 2-carboxylique syn cis racémique.

Comme indiqué au stade D de l'exemple 11, on prépare 238 mg de produit attendu F>260°C.

| Analyse $C_{19}H_{16}ClN_5O_5S_2$ | | | | |
|---|---|---|---|---|
| Calculé<br>Trouvé | C% 46,20<br>45,7 | H% 3,27<br>3,3 | N% 14,18<br>13,5 | Cl% 7,18<br>6,9 |

Spectre IR

C = 0 1760 $cm^{-1}$ ($\beta$lactame)
C = 0 1706 $cm^{-1}$ (acide)
C = 0 1650 $cm^{-1}$ (amide)
amide II
hétérocycle 1525-1540 $cm^1$
aromatique 1605 $cm^{-1}$
1583 $cm^{-1}$
1490 $cm^{-1}$

EXEMPLE 16 : 4-oxyde de l'acide 7-/2-(2-aminothiazol-4-yl) 2-méthoxyimino acétamido/ 3-(4-chlorophényl) 8-oxo 4-thia 1-azabicyclo /4-2-0/ oct-2-ène 2-carboxylique syn cis racémique.

STADE A :4-oxyde de 7-/2-(2-tritylamino thiazol-4-yl) 2-méthoxyimino acétamido/ 3-(4-chlorophényl)8-oxo 4-thia 1-azabicyclo /4-2-0/ oct-2-ène 2-carboxylate de 1,1-diméthyl éthyle.

On opère comme au stade A de l'exemple 14 à partir de 2,4 g de 7-/2-(2-tritylaminothiazol-4-yl) 2-méthoxyimino acétamido/ 3-(4-chlorophényl) 8-oxo 4-thia 1-azabicyclo /4-2-0/ oct-2-ène 2-carboxylate de 1,1-diméthyl éthyle et obtient, après cristallisation dans l'éther isopropylique, 173 mg d'isomère$\beta$ F=185-190°C et 1,27 g d'isomère $\alpha$ F=185-190°C après cristallisation dans l'éther.

STADE B :4-oxyde de l'acide 7-/2-(2-aminothiazol-4-yl) 2-méthoxyimino acétamido/ 3-(4-chlorophényl) 8-oxo 4-thia 1-azabicyclo /4-2-0/ oct-2-ène 2-carboxylique syn cis racémique.

On opère comme au stade B de l'exemple 14 à partir de 300 mg de produit obtenu au stade précédent. On obtient 119 mg F≃260°C (décomposition).

EXEMPLE 17 : Acide 7-/2-(2-aminothiazol-4-yl) 2-méthoxyimino acétamido/ 8-oxo 3-(phénylséléno) 4-thia 1-azabicyclo /4-2-0/ oct-2-ène 2-carboxylique syn cis racémique.

STADE A : Chlorure de l'acide terbutyloxalique.

On refroidit à -60°C 84 cm3 de chlorure d'oxalyle dans 800 cm3 d'éther et ajoute en 1/2 heure, une solution de 94 cm3 de terbutanol, 80 cm3 de pyridine et 200 cm3 d'éther. On laisse remonter la température à 0°C et agite encore pendant 1 heure. On filtre et concentre à sec le filtrat, distille le résidu sous pression réduite et obtient 93 g de produit attendu Eb 13 mm Hg = 45-46°C.

STADE B :diazopyruvate de tertbutyle.

On refroidit à O°/5°C 16 g de produit obtenu ci-dessus dans 400 cm3 d'éther et ajoute 350 cm3 de diazométhane en solution chlorométhylénique (25,5 g/litre). On agite encore 1/2 heure à 0°C après l'introduction puis concentre à sec sous pression réduite, reprend le résidu avec très peu de n-pentane, essore et obtient 11 g de produit attendu F = 100°C.

STADE C :7-/2-(2-tritylamino thiazol-4-yl) 2-méthoxyimino acétamido/ 8-oxo 4-thia 2-hydroxy 3-phényl séléno 1-azabicyclo /4-2-0/ octan-2-carboxylate de tertbutyle.

On met en suspension 557 mg de 4-mercaptométhyl 3-/2-(2-tritylamino thiazol-4-yl) 2-méthoxyimino acétamido/ 2-oxo 1-azétidine dans 4 cm3 de chlorure de méthylène, ajoute en une seule fois la solution de phényl séléno pyruvate préparée ci-dessous, puis 0,2 cm3 de triéthylamine. On agite 1 heure à température ambiante sous atmosphère inerte et verse dans 20 cm3 d'un mélange eau et glace puis extrait au chlorure de méthylène. On lave à l'eau la phase organique, sèche et concentre à sec sous pression réduite et obtient 950 mg de produit attendu brut. On chromatographie sur silice le résidu obtenu par un mélange chlorure de méthylène-acétate d'éthyle (70-30) et isole 280 mg de produit attendu F 230°C (décomposition).

Préparation du 3-chloro 3-phénylséléno pyruvate de tertbutyle.

On dissout 170 mg de diazopyruvate de tertbutyle dans 2 cm3 de chlorure de méthylène puis ajoute 191 mg de chlorure de phénylsélényle et agite le mélange à température ambiante jusqu'à fin de dégagement gazeux.

STADE D :7-/2-(2-tritylamino thiazol-4-yl) 2-méthoxyimino acétamido/ 8-oxo 4-thia 3-(phénylséléno) 1-azabicyclo /4-2-0/ oct-2-ène 2-carboxylate de tertbutyle.

On agite 4 g de produit brut obtenu ci-dessus et 40 cm3 de pyridine et ajoute en 2 fois, en 10 minutes, 5,24 g de tétraiodure de diphosphore et laisse sous agitation à température ambiante pendant 1 heure. On verse le mélange dans 500 cm3 d'eau, 200 cm3 d'acétate d'éthyle et 200 cm3 d'acide chlorhydrique N, agite 1/4 d'heure, décante et réextrait la phase aqueuse à l'acétate d'éthyle. Les phases organiques réunies sont lavées à l'eau, séchées et concentrées à sec sous pression réduite. On chromatographie le résidu sur silice avec un mélange chlorure de méthylène-acétate d'éthyle (9-1) et obtient 370 mg de produit que l'on reprend dans l'éther isopropylique pour isoler 300 mg de produit attendu F≃150°C (pâteuse).

STADE E :Acide 7-/2-(2-aminothiazol-4-yl) 2-méthoxyimino acétamido/ 8-oxo 3-(phénylséléno) 4-thia 1-azabicyclo /4-2-0/ oct-2-ène 2-carboxylique syn cis racémique.

On chauffe à 60°C pendant 1 heure 1/4 300 mg du produit obtenu ci-dessus dans 6 cm3 d'acide formique à 66 %. On essore le triphénylcarbinol formé et concentre à sec sous pression réduite le filtrat. On reprend le résidu par 15 cm3 d'éther isopropylique et obtient 190 mg de produit attendu F 190°C.

| Analyse $C_{18}H_{17}N_5O_5S_2Se$ : 538,46 | | | | |
|---|---|---|---|---|
| Calculé | C% 42,38 | H% 3,18 | N% 13,00 | S% 11,90 |
| Trouvé | 42,9 | 3,3 | 12,5 | 13,4 |

Spectre UV

1) dans EtOH
infl : 216 nm $E_1{}^1$ : 505
infl : 233 nm $E_1{}^1$ : 399 = 21500
max : 310 nm $E_1{}^1$ : 244 = 13100
2) dans EtOH/HCl 0,1N
infl : 216 nm $E_1{}^1$ : 430
max : 276 nm $E_1{}^1$ : 318 = 17100
infl : 283 nm $E_1{}^1$ : 315
max : 322 nm $E_1{}^1$ : 212 = 11400

EXEMPLE 18 : Acide 7-/2-(2-aminothiazol-4-yl) 2-méthoxyimino acétamido/ 3-(3-nitrophénylthio) 8-oxo 4-thia 1-azabicyclo /4-2-0/ oct-2-ène 2-carboxylique cis racémique.

STADE A :7-/2-(2-tritylaminothiazol-4-yl) 2-méthoxyimino acétamido/ 2-hydroxy 3-(3-nitrophénylthio) 8-oxo 4-thia 1-azabicyclo /4-2-0/ octane 2-carboxylate de 1,1-diméthyl éthyle.

On refroidit à 0°C une solution de 678 mg de di-(3-nitrophényl) di-sulfure dans 5 cm3 de chlorure de méthylène, ajoute goutte à goutte une solution de 142 mg de chlore dans 1,12 cm3 de tétrachlorure de carbone et agite pendant 10 minutes à 0°C et 20 minutes à température ambiante. On refroidit à 0°C et ajoute lentement une solution de 681 mg de diazopyruvate de tertbutyle dans 5 cm3 de chlorure de méthylène. On agite pendant 30 minutes à température ambiante puis ajoute 1,6 g de 4-mercaptométhyl 3-/2-(2-tritylamino thiazol-4-yl) 2-méthoxyimino acétamido/ 2-oxo 1-azétidine et 0,56 cm3 de triéthylamine et maintient l'agitation encore 1 heure. On filtre, lave le filtrat avec de l'acide chlorhydrique 0,1N, sèche et concentre à sec puis chromatographie le résidu sur silice en éluant par un mélange chloroforme-acétone (9-1). On obtient 800 mg de produit attendu.

Spectre UV

1) dans EtOH
maxi : 248 nm $E_1{}^1$ : 373 $\epsilon$ = 31800
infl : 295 nm $E_1{}^1$ : 90 $\epsilon$ = 7700
2) dans EtOH/HCl 0,1N
maxi : 252 nm $E_1{}^1$ : 310 $\epsilon$ = 26400
infl : 290 nm $E_1{}^1$ : 189 $\epsilon$ = 16100
infl : 300 nm $E_1{}^1$ : 133 $\epsilon$ = 11300

STADE B :7-/2-(2-tritylaminothiazol-4-yl) 2-méthoxyimino acétamido/ 3-(3-nitrophénylthio) 8-oxo 4-thia 1-azabicyclo /4-2-0/ oct-2-ène 2-carboxylate de 1,1-diméthyl éthyle racémique.

On agite la suspension de 677 mg de tétraiodure de diphosphore dans 7 cm3 de pyridine sous atmosphère inerte pendant 5 minutes, ajoute 780 mg du produit obtenu ci-dessus et agite pendant 2 heures. On ajoute à nouveau 363 mg de tétraiodure de diphosphore et maintient l'agitation pendant 1 h. On verse le mélange réactionnel dans 50 cm3 d'acétate d'éthyle, filtre puis évapore à sec sous pression réduite. On dissout le résidu dans le chlorure de méthylène, lave avec de l'acide chlorhydrique 0,1N, puis à l'eau. On sèche les phases organiques et concentre à sec. On chromatographie le résidu sur silice par un mélange chloroforme-acétone (10-0,5) et obtient 348 mg de produit cristallisé avec l'éther.

Spectre UV

1) dans EtOH
maxi : 241 nm $E_1{}^1$ : 470 $\epsilon$ = 39200
maxi : 309 nm $E_1{}^1$ : 227 $\epsilon$ = 19000
2) dans EtOH/HCl 0,1N
infl : 242 nm $E_1{}^1$ : 425 $\epsilon$ = 35500
maxi : 270 nm $E_1{}^1$ : 309 $\epsilon$ = 25800
infl : 291 nm $E_1{}^1$ : 290

infl : 303 nm $E_1^1$ : 265 $\epsilon$ = 22100
infl : 317 nm $E_1^1$ : 203 $\epsilon$ = 16900

STADE C :Acide 7-/2-(2-aminothiazol-4-yl) 2-méthoxyiminoacétamido/ 3-(3-nitrophénylthio) 8-oxo 4-thia 1-azabicyclo /4-2-0/ oct-2-ène 2-carboxylique cis racémique.

On dissout à 50°C, 286 mg de produit obtenu au stade précédent dans 3 cm3 d'acide formique à 66 %, agite pendant 4 heures, filtre et concentre à sec le filtrat. On dissout le résidu dans un mélange acétonitrile éthanol puis évapore les solvants. On recristallise le résidu dans l'isopropanol et obtient 82 mg de produit attendu. On récupère 65 mg du même produit des liqueurs mères F=180°-200°C (décomposition).

Spectre UV
- dans EtOH/HCl 0,1N
infl : 219 nm $E_1^1$ : 439
maxi : 250 nm $E_1^1$ : 495 $\epsilon$ = 26500
infl : 280 nm $E_1^1$ : 386
infl : 291 nm $E_1^1$ : 352
infl : 310 nm $E_1^1$ : 289 $\epsilon$ = 15500

EXEMPLE 19 : Acide 7-/2-(2-aminothiazol-4-yl) 2-méthoxyimino acétamido/ 8-oxo 3-(4-nitrophényl thio) 4-thia 1-azabicyclo /4-2-0/ oct-2-ène 2-carboxylique syn racémique.

STADE A :7-/2-(2-tritylaminothiazol-4-yl) 2-méthoxyimino acétamido/ 2-hydroxy 3-(4-nitrophénylthio) 8-oxo 4-thia 1-azabicyclo /4-2-0/ octane 2-carboxylate de 1,1-diméthyl éthyle.

On refroidit à 5°/10°C sous atmosphère inerte, 13,4 gde N-chlorosuccinimide dans80 cm3 de benzène. En 30 minutes, on introduit 15,5 g de 4-nitro thiophénol à moins de 10°C et agite pendant 16 heures à température ambiante. On filtre, évapore les solvants et sèche sous pression réduite à température ambiante. On reprend 1,33 g du résidu dans 10 cm3 de chlorure de méthylène et ajoute goutteà goutte, en 15 minutes, cette solution à une solution refroidie à 0°/5°C de 0,8 g de diazopyruvate de terbutyle dans 15 cm3 de chlorure de méthylène sous atmosphère inerte. On agite 30 minutes puis ajoute 2,62 g de 4-mercaptométhyl 3-/2-(2-tritylaminothiazol-4-yl) 2-méthoxyimino acétamido/ 2-oxo 1-azétidine et 0,505 g de triéthylamine. On agite pendant 1 heure, lave avec de l'acide chlorhydrique 0,1N, de l'eau et concentre à sec. On chromatographie le résidu sur silice avec un mélange chloroforme-acétone (9-1) et obtient 2,5 g de produit attendu.

Spectre UV

1) dans EtOH
infl : 236 nm $E_1^1$ : 317 $\epsilon$ = 27000
infl : 259 nm $E_1^1$ : 185 $\epsilon$ = 15800
infl : 266 nm $E_1^1$ : 163
maxi : 316 nm $E_1^1$ : 164 $\epsilon$ = 14000
2) dans EtOH/HCl 0,1N
infl : 271 nm $E_1^1$ : 194
maxi : 280 nm $E_1^1$ : 206 $\epsilon$ = 17600
infl : 289 nm $E_1^1$ : 205
infl : 301 nm $E_1^1$ : 190 $\epsilon$ = 16200
infl : 321 nm $E_1^1$ : 148 $\epsilon$ = 12600

STADE B :7-/2-(2-tritylaminothiazol-4-yl) 2-méthoxyimino acétamido/ 3-(4-nitrophénylthio) 8-oxo 4-thia 1-azabicyclo /4-2-0/ oct-2-ène 2-carboxylate de 1,1-diméthyl éthyle.

On opère comme au stade B de l'exemple 18 à partir de 1,79 g de produit obtenu précédemment et obtient 500 mg de produit attendu.

Spectre UV

- dans EtOH/HCl 0,1N
maxi : 292 nm $E_1^1$ : 280 $\epsilon$ = 23400
infl: 319 nm $E_1^1$ : 246 $\epsilon$ = 20500

STADE C :Acide 7-/2-(2-aminothiazol-4-yl) 2-méthoxyimino acétamido/ 8-oxo 3-(4-nitrophénylthio) 4-thia 1-azabicyclo /4-2-0/ oct-2-ène 2-carboxylique syn racémique.

On traite comme à l'exemple 18, stade C, 200 mg de produit obtenu ci-dessus. On reprend le résidu à l'acétonitrile, évapore le solvant puis cristallise le produit avec l'éther. On obtient 80 mg de produit F = 190-200 ° C (décomposition).

Spectre UV

- Ethanol
maxi : 223 nm $E_1^1$ : 481
maxi : 307 nm $E_1^1$ : 363
- Ethanol/HCl 0,1N
maxi : 222 nm $E_1^1$ : 400
infl : 265-274 nm
maxi : 290 nm $E_1^1$ : 361
infl : 310 nm $E_1^1$ : 331

EXEMPLE 20 : Acide 7-/2-(2-aminothiazol-4-yl) 2-méthoxyimino acétamido/ 8-oxo 3-(4-méthoxyphénylthio) 4-thia 1-azabicyclo /4-2-0/ oct-2-ène 2-carboxylique syn racémique.

STADE A : 7-/2-(2-tritylamlnothiazol-4-yl) 2-méthoxyimino acétamido/ 2-hydroxy3-(4-méthoxyphénylthio) 8-oxo 4-thia 1-azabicyclo /4-2-0/ octane 2-carboxylate de 1,1-diméthyl éthyle.

On opère comme au stade A de l'exemple 19 à partir de 14,0 g de 4-méthoxy thiophénol et obtient après distillation du résidu 5,16 g de chlorure de 4-méthoxyphényl sulfényle Eb 0,12 mm Hg = 78 ° C. On refroidit à 0 ° /5 ° C, 1 g de diazopyruvate de tertbutyle dans 10 cm3 de chlorure de méthylène et ajoute goutte à goutte une solution de 1,12 g de chlorure de 4-méthoxy phényl sulfényle dans 10 cm3 de chlorure de méthylène et agite le mélange. Après 30 minutes, on ajoute 3,27 g de 4-mercaptométhyl 3-/2-(2-tritylaminothiazol-4-yl) 2-méthoxyimino acétamido/ 2-oxo 1-azétidine et 0,82 cm3 de triéthylamine puis agite pendant 1 heure. On lave le mélange réactionnel à l'acide chlorhydrique 0.1N à l'eau, le sèche et concentre à sec. On obtient 3,7 g de produit attendu après cristallisation avec de l'éther.

Spectre UV

1) dans EtOH
infl : 227 nm $E_1^1$ : 430 $\epsilon$ = 36000
infl : 241 nm $E_1^1$ : 355 = 29700
infl : 280 nm $E_1^1$ : 124
infl : 296 nm $E_1^1$ : 84 $\epsilon$ = 7000
2) EtOH/HCl 0,1N
maxi : 252 nm $E_1^1$ : 237 $\epsilon$ = 19900
maxi : 273 nm $E_1^1$ : 211 $\epsilon$ = 17700
infl : 289 nm $E_1^1$ : 187 $\epsilon$ = 15700
infl : 301 nm $E_1^1$ : 132

STADE B :7-/2-(2-tritylaminothiazol-4-yl) 2-méthoxyimino acétamido/ 3-(4-méthoxyphénylthio) 8-oxo 4-thia 1-azabicyclo /4-2-0/ oct-2-ène 2-carboxylate de 1,1-diméthyl éthyle cis racémique.

On opère comme au stade B de l'exemple 18 à partir de 2 g de produit obtenu ci-dessus, évapore la pyridine sous pression réduite à moins de 40 ° C, ajoute de l'eau, acidifie à pH 2 et extrait à l'acétate d'éthyle. On sèche les phases organiques, concentre à sec sous pression réduite et chromatographie le

résidu sur silice en éluant avec un mélange chlorure de méthylène-acétone (10-0,5). On obtient 841 mg de produit attendu cristallisé avec l'éther F> 215 ° C (décomposition).

Spectre UV

1) dans EtOH
maxi : 238 nm $E_1^1$ : 446 $\epsilon$ = 36600
infl : 267 nm $E_1^1$ : 227
maxi : 315 nm $E_1^1$ : 215 $\epsilon$ = 17600
2) dans EtOH/HCl 0,1N
infl : 235 nm $E_1^1$ : 371
infl : 271 nm $E_1^1$ : 250
maxi : 281 nm $E_1^1$ : 255 $\epsilon$ = 20900
maxi : 293 nm $E_1^1$ : 253 $\epsilon$ = 20700
infl : 303 nm $E_1^1$ : 240
infl : 320 nm $E_1^1$ : 198 $\epsilon$ = 16200

STADE C :7-/2-(2-aminothiazol-4-yl) 2-méthoxyimino acétamido/ 3-(4-méthoxyphénylthio) 8-oxo 4-thia 1-azabicyclo /4-2-0/ oct-2-ène 2-carboxylate de 1,1-diméthyl éthyle racémique.

On dissout 810 mg de produit obtenu précédemment dans 10 cm3 d'acide formique à 66 %, agite 1 heure à température ambiante, filtre et concentre à sec sous pression réduite à moins de 30 ° C. On dissout le résidu dans un mélange acétonitrile-méthanol, évapore les solvants et cristallise le résidu avec l'éther. On obtient 516 mg de produit attendu.

Spectre UV

1) dans EtOH
maxi : 236 nm $E_1^1$ : 496 $\epsilon$ = 28700
maxi : 310 nm $E_1^1$ : 292 $\epsilon$ = 16900
2) dans EtOH/HCl 0,1N
maxi : 240 nm $E_1^1$ : 416 $\epsilon$ = 24000
infl : 259 nm $E_1^1$ : 352
infl : 281 nm $E_1^1$ : 316 $\epsilon$ = 18200
maxi : 317 nm $E_1^1$ : 259 $\epsilon$ = 15000

STADE D :Acide 7-/2-(2-aminothiazol-4-yl) 2-méthoximino acétamido/ 8-oxo 3-(4-méthoxyphénylthio) 4-thia 1-azabicyclo /4-2-0/ oct-2-ène 2-carboxylique syn racémique.

On dissout 108 mg du produit obtenu au stade précédent dans 2 cm3 d'acide formique à 66 % et chauffe à 50 ° C pendant 3 heures sous agitation puis concentre à sec sous pression réduite. On reprend le résidu à l'acétonitrile, évapore le solvant et cristallise le produit brut dans l'éther. On le dissous dans du chlorure de méthylène à 10 % de méthanol, traite au charbon actif, filtre, concentre à sec le filtrat et cristallise le résidu obtenu avec l'éther. On obtient 80 mg de produit attendu F=185-190 ° C (décomposition).

Spectre UV

- dans EtOH/HCl 0,1N
maxi : 242 nm $E_1^1$ : 458 $\epsilon$ = 23900
infl : 250 nm $E_1^1$ : 358
infl : 282 nm $E_1^1$ : 319
maxi : 316 nm $E_1^1$ : 279 $\epsilon$ = 14500

EXEMPLE 21 : Acide 7-/2-(2-aminothiazol-4-yl) 2-méthoxyimino acétamido/ 8-oxo 3-phénylthio 4-thia 1-azabicyclo /4-2-0/ oct-2-ène 2-carboxylique syn racémique.

STADE A :7-/2-(2-tritylaminothiazol-4-yl) 2-méthoxyimino acétamido/ 2-hydroxy 8-oxo 3-phénylthio 4-thia 1-azabicyclo /4-2-0/ octane 2-carboxylate de 1,1-diméthyl éthyle.

On opère comme au stade A de l'exemple 19 à partir de 11 g de thiophénolet obtient après distillation sous pression réduite du résidu, 10,9 g de chlorure de phénylsulfényle Eb 4 mm Hg = 68 ° C. On en dissout 0,17 g dans 2 cm3 de chlorure de méthylène, ajoute 0,15 g de diazopyruvate de tertbutyle dans 0,5 cm3 de chlorure de méthylène, agite 15 minutes puis ajoute 5 cm3 de chlorure de méthylène, 0,56 g de 4-mercaptométhyl 3-/2-(2-tritylaminothiazol-4-yl) 2-méthoxyimino acétamido/ 2-oxo 1-azétidine et 0,1 g de triéthylamine. On agite pendant 30 minutes, lave à l'eau, sèche et concentre à sec sous pression réduite, chromatographie le résidu sur silice avec un mélange chloroforme-acétone (8-2) et obtient 545 mg de produit attendu.

Spectre UV

1) dans EtOH
infl : 237 nm $E_1^1$ : 310 $\epsilon$ = 25000
infl : 246 nm $E_1^1$ : 301 $\epsilon$ = 24300
infl : 270 nm $E_1^1$ : 151
infl : 295 nm $E_1^1$ : 75 $\epsilon$ = 6000
2) dans EtOH/HCl 0,1N
maxi : 252 nm $E_1^1$ : 230 $\epsilon$ = 18600
maxi : 274 nm $E_1^1$ : 194 $\epsilon$ = 15700
infl : 288 nm $E_1^1$ : 170 $\epsilon$ = 13700
infl : 300 nm $E_1^1$ : 124

STADE B :7-/2-(2-tritylaminothiazol-4-yl) 2-méthoxyimino acétamido/ 8-oxo 3-phénylthio 4-thia 1-azabicyclo /4-2-0/ oct-2-ène 2-carboxylate de 1,1-diméthyl éthyle.

On opère comme au stade B de l'exemple 20 à partir de 1,5 g de produit obtenu ci-dessus et obtient 580 mg de produit attendu F=200-202 ° C (décomposition).

Spectre UV

1) dans EtOH
infl : 235 nm $E_1^1$ : 410
infl : 273 nm $E_1^1$ : 190
maxi : 310 nm $E_1^1$ : 216
2) dans EtOH/HCl 0,1N
maxi : 281-282 nm $E_1^1$ : 266
maxi : 292 nm $E_1^1$ : 274
infl : 304 nm$E_1^1$ : 255
infl : 320 nm $E_1^1$ : 191

STADE C :7-/2-(2-aminothiazol-4-yl) 2-méthoxyimino acétamido/ 8-oxo 3-phénylthio 4-thia 1-azabicyclo /4-2-0/ oct-2-ène 2-carboxylate de 1,1-diméthyl éthyle.

On dissout 200 mg de produit obtenu au stade précédent dans 5 cm3 d'acide formique à 66 %, agite pendant 1 heure à température ambiante et concentre à sec sous pression réduite à moins de 30 ° C. On dissout le résidu dans un mélange acétonitrile-méthanol, évapore le solvant et cristallise avec l'éther pour obtenir 110 mg de produit attendu

Rf : 0,42 ($CH_2Cl_2$-

MeOH (9-1)).

STADE D :Acide 7-/2-(2-aminothiazol-4-yl) 2-méthoxyimino acétamido/ 8-oxo 3-phénylthio 4-thia 1-azabicyclo /4-2-0/ oct-2-ène 2-carboxylique syn racémique.

On refroidit à -20 ° C, 0,8 cm3 d'acide trifluoroacétique, 0,2 cm3 de chlorure de méthylène. On ajoute 168 mg du produit obtenu comme précédemment, agite à -20 ° C pendant 1 heure puis à 0 ° C pendant 1 heure. On évapore les solvants à 0 ° C par courant d'azote. On ajoute 5 cm3 d'éther isopropylique, agite à température ambiante pendant 10 minutes et on essore le sel de trifluoroacétate du produit attendu. On le dissous dans 1 cm3 d'éthanol. On ajoute de l'acétate d'éthyle et 3 gouttes de pyridine. On cristallise le produit. On purifie ce produit impur par chromatographie sur silice avec un mélange d'éluant chlorure de méthylène-méthanol (7:3). On évapore les fractions intéressantes et on cristallise le produit avec le chlorure de méthylène contenant quelques gouttes de méthanol. On obtient 55 mg de produit attendu.

Spectre UV

1) dans EtOH
maxi : 304 nm $E_1^1$ : 314 $\epsilon$ = 15400
infl : 221 nm $E_1^1$ : 457
infl : 231 nm $E_1^1$ : 430
2) dans EtOH/HCl 0,1N
infl : 220 nm $E_1^1$ : 381 $\epsilon$ = 16500
infl : 225 nm $E_1^1$ : 329
maxi : 263 nm $E_1^1$ : 336
infl : 280 nm $E_1^1$ : 323
infl : 291 nm $E_1^1$ : 308
infl : 331 nm $E_1^1$ : 258

EXEMPLE 22 : Acide 7-/2-(2-aminothiazol-4-yl) 2-méthoxyimino acétamido/ 3-(éthoxy carbonyl méthyl thio) 8-oxo 4-thia 1-azabicyclo /4-2-0/ oct-2-ène 2-carboxylique racémique.

STADE A :7-/2-(2-tritylaminothiazol-4-yl) 2-méthoxyimino acétamido/ 3-(éthoxy carbonyl méthyl thio) 2-hydroxy 8-oxo 4-thia 1-azabicyclo /4-2-0/ octane 2-carboxylate de 1,1-diméthyl éthyle.

On met en suspension 1,47 g de N-chlorosuccinimide dans 6 cm3 de tétrachlorure de carbone sous atmosphère inerte et ajoute goutte à goutte 1,2 g de mercapto acétate d'éthyle dans 2 cm3 de tétrachlorure de carbone, en agitant fortement pendant 1 heure. Après 10 minutes, on filtre la solution de chlorure d'éthoxy carbonyl méthyl sulfényle obtenu et l'ajoute goutte à goutte à une solution de 1,7 g de diazopyruvate de tertbutyle dans 5 cm3 de tétrachlorure de carbone. On agite 10 minutes puis évapore les solvants. On chromatographie le résidu sur silice par un mélange hexane-acétate d'éthyle (6-4) et obtient 1,48 g de 3-chloro 3-(éthoxy carbonyl méthyl thio) pyruvate de tertbutyle. On dissout ce dernier dans 10 cm3 de chlorure de méthyle, ajoute 2,23 g de 4-mercaptométhyl 3-/2-(2-tritylamino thiazol-4-yl) 2-méthoxyimino acétamido/ 2-oxo 1-azétidine et 0,7 cm3 de triéthylamine, agite pendant 30 minutes, concentre à sec et chromatographie sur silice le résidu obtenu en éluant par un mélange chlorure de méthylène-acétone (8,5-1,5). Après cristallisation avec l'éther, on obtient 1,55 g de produit attendu F = 190 ° /195 ° C (décomposition).

Spectre UV

1) dans EtOH
infl : 265 nm $E_1^1$ : 155 $\epsilon$ = 12700
infl : 271 nm $E_1^1$ : 135
infl : 293 nm $E_1^1$ : 82 $\epsilon$ = 6700
2) dans EtOH/HCl 0,1N
maxi : 280 nm $E_1^1$ : 201 $\epsilon$ = 16400
infl : 290 nm $E_1^1$ : 184

STADE B : 7-/2-(2-tritylaminothiazol-4-yl) 2-méthoxyimino acétamido/ 3-(éthoxy carbonyl méthyl thio) 8-oxo 4-thia 1-azabicyclo /4-2-0/ oct-2-ène 2-carboxylatede 1,1-diméthyl éthyle racémique.

On dissout 1,55 g de produit obtenu ci-dessus dans 30 cm3 de pyridine, ajoute sous atmosphère inerte 1,4 g de tétraiodure de diphosphore et agite pendant 30 minutes. On ajoute encore 0,76 g de tétraiodure de diphosphore et agite pendant 1 heure. On ajoute100 cm3 d'acétate d'éthyle, filtre, concentre à sec sous pression réduite à une température inférieure à 30° C. On dissout le résidu dans l'acétate d'éthyle, filtre, lave avec de l'acide chlorhydrique 0,1N, avec une solution de bicarbonate de sodium, avec une solution de chlorure de sodium, sèche et concentre à sec. On chromatographie le résidu sur silice par un mélangechlorure de méthylène-acétone (10-0,75) et obtient, après cristallisation à l'éther, 675 mg de produit attendu F=212°/214°C (décomposition).

Spectre UV

1) dans EtOH
infl : 227 nm $E_1^1$ : 407
infl : 265 nm $E_1^1$ : 195 $\epsilon$ = 15600
maxi : 306 nm $E_1^1$ : 203 $\epsilon$ = 16200
2) dans EtOH/HCl 0,1N
infl : 285 nm $E_1^1$ : 260
maxi : 292 nm $E_1^1$ : 270 $\epsilon$ = 21600
infl : 300 nm $E_1^1$ : 257
infl : 315 nm $E_1^1$ : 180 $\epsilon$ = 14400

STADE C :Acide 7-/2-(2-aminothiazol-4-yl) 2-méthoxyimino acétamido/ 3-(éthoxy carbonyl méthyl thio) 8-oxo 4-thia 1-azabicyclo /4-2-0/ oct-2-ène 2-carboxylique racémique.

On dissout à 50°C, 200 mg de produit obtenu au stade B dans 4 cm3 d'acide formique à 66 %, agite pendant 2 heures. On refroidit, filtre la solution et concentre à sec le filtrat sous pression réduite. On redissout le résidu dans l'éthanol puis évapore le solvant. On ajoute 20 cm3 d'éther, agite 30 minutes, essore le produit brut et le chromatographie sur silice par un mélange chlorure de méthylène-méthanol (8-2). Après cristallisation avec l'éther, on obtient 70 mg de produit attendu F> 210°C (décomposition).

Spectre UV

1) dans EtOH
maxi : 221-222 nm $E_1^1$ : 327 $\epsilon$ = 16400
maxi : 301 nm $E_1^1$ : 251 $\epsilon$ = 12600
2) dans EtOH/HCl 0,1N
maxi : 225 nm $E_1^1$ : 246 $\epsilon$ = 12500
maxi : 262 nm $E_1^1$ : 264 $\epsilon$ = 13200
infl : 290 nm $E_1^1$ : 258
infl : 310 nm $E_1^1$ : 190 $\epsilon$ = 9800

EXEMPLE 23 :Acide 7-/2-(2-aminothiazol-4-yl) 2-méthoxyimino acétamido/ 8-oxo 3-/(1-méthyl (1H) tétrazol-5-yl) thio/ 4-thia 1-azabicyclo /4-2-0/ oct-2-ène 2-carboxylique syn cis racémique.

STADE A :7-/2-(2-tritylaminothiazol-4-yl) 2-méthoxyimino acétamido/ 8-oxo 3-/(1-méthyl (1H) tétrazol-5-yl) thio/ 4-thia 1-azabicyclo /4-2-0/ octane-2-hydroxy 2-carboxylate de 1,1-diméthyl éthyle.

Le disulfure de bis (1-méthyl (1H) tétrazol-5-yl) utilisé au début de l'exemple a été préparé comme suit :
On dissout 1,26 g de sel de sodium de 1-méthyl 1,2-dihydro (5H) tétrazol-5-thione dans 25 cm3 d'eau, ajoute 5 cm3 d'acide chlorhydrique 2N. On introduit alors, par petites portions, en 5 minutes, 2 cm3 d'eau oxygénée à 30 % puis on chauffe pendant 1/4 d'heure entre 20° et 45°C. On refroidit à 0°/56C, essore les cristaux formés que l'on recristallise dans 9 cm3 d'éthanol. On obtient 446 mg de produit attendu F=114°C.
On met en suspension 437 mg de disulfure de bis (1-méthyl (1H) tétrazol-5-yl) dans 7,5 cm3 de chlorure de méthylène, refroidit à -20°Cet ajoute, sous agitation et sous atmosphère inerte, une solution de

124 mg de chlore dans 0,975 cm3 de tétrachlorure de carbone. On laisse remonter la température à 0 ° C et agite 5 minutes à 0 ° C. A la solution de chlorure de 1-méthyl (1H) tétrazol-5-yl sulfényle obtenue, on ajoute en 10 minutes 595 mg de diazopyruvate de tertbutyle en solution dans 5 cm3 de chlorure de méthylène et agite 1/4 d'heure à 0 ° C et laisse remonter à température ambiante. On n'isole pas le 3-chloro 3-/(1-méthyl (1H) tétrazol-5-yl) thio/ pyruvate de tertbutyle formé, mais ajoute à la solution, en une seule fois, 2,16 g de 4-mercapto méthyl 3-/2-(2-tritylaminothiazol-4-yl) 2-méthoxyimino acétamido/ 2-oxo 1-azétidine, 0,61 cm3 de triéthylamine et 10 cm3 de chlorure de méthylène. Après une agitation de 3 heures, on filtre l'insoluble, lave le filtrat avec 50 cm3 d'eau additionnée de 2 cm3 d'acide chlorhydrique 2N, puis à l'eau, sèche et concentre à sec sous pression réduite. On chromatographie le résidu sur silice par un mélange chlorure de méthylène-acétate d'éthyle (75-25) et obtient 1,393 g de produit attendu.

| Analyse $C_{38}H_{39}N_9O_6S_3$ PM : 813,983 | | | | |
|---|---|---|---|---|
| Calculé<br>Trouvé | C% 56,07<br>56,1 | H% 4,83<br>5,0 | N% 15,49<br>14,5 | S% 11,82<br>11,8 |

Spectre UV

1) dans EtOH
infl : 240 nm
infl : 265 nm
infl : 278 nm
infl : 298 nm
2) dans EtOH/HCl 0,1N
infl : 270 nm
infl : 289 nm
maxi : 278 nm

STADE B :7-/2-(2-tritylamino thiazol-4-yl) 2-méthoxyimino acétamido/ 8-oxo 3/(1-méthyl (1H) tétrazol-5-yl) thio/ 4-thia 1-azabicyclo /4-2-0/ oct-2-ène 2-carboxylate de 1,1-diméthyléthyle syn cis racémique.

A une solution de 1,135 g de produit obtenu ci-dessus dans 14 cm3 de pyridine, on ajoute d'abord 1,19 g de tétraiodure de diphosphore puis, 1/2 heure plus tard, 0,4 g. On agite ensule pendant 3 heures. On verse le mélange réactionnel sur 400 cm3 d'eau, 76 cm3 d'acide chlorhydrique 2N et 100 cm3 d'acétate d'éthyle. On décante et extrait à l'acétate d'éthyle. On lave à l'eau les phases organiques réunies, sèche et concentre à sec sous pression réduite. On chromatographie le résidu sur silice par un mélange chlorure de méthylène-acétate d'éthyle (85-15) et obtient 3,544 g de produit attendu F≃150 ° C.

Spectre UV

1) dans EtOH
infl : 230 nm
maxi : 310 nm
2) dans EtOH/HCl 0,1N
infl : 284 nm
infl : 302 nm
infl : 315 nm
maxi : 292 nm

STADE C :Acide 7-/2-(2-aminothiazol-4-yl) 2-méthoxyimino acétamido/ 8-oxo 3-/(1-méthyl (1H) tétrazol-5-yl) thio/ 4-thia 1-azabicyclo /4-2-0/ oct-2-ène 2-carboxylique syn cis racémique.

On dissout 248 mg de produit obtenu au stade précédent dans 3,5 cm3 d'acide formique à 66 %, agite et chauffe à 55 ° /60 ° C pendant 7 heures. On dilue avec 3,5 cm3 d'eau, essore l'insoluble, concentre à sec le filtrat sous pression réduite. On reprend le résidu 3 fois consécutives par 5 cm3 d'éthanol en évaporant à chaque fois. On triture dans 10 cm3 d'éther les 152 mg de résine obtenus, essore après 16 heures et obtient 128 mg de produit attendu F≃210 ° C (décomposition).

Spectre UV

1) dans EtOH
maxi : 231 nm
infl : 250 nm
maxi : 305 nm
2) dans EtOH/HCl 0,1N
maxi : 229 nm
maxi : 260 nm
maxi : 284 nm
infl : 291 nm
infl : 311 nm

EXEMPLE 24 : Acide 7-/2-(2-aminothiazol-4-yl) 2-méthoxyimino acétamido/ 3-(carboxy méthyl thio) 8-oxo 4-thia 1-azabicyclo /4-2-0/ oct-2-ène 2-carboxylique racémique.

STADE A :7-/2-(2-tritylaminothlazol-4-yl) 2-méthoxyimino acétamido/ 3-//2-(1,1-diméthyl éthoxy) 2-oxo éthyl/ thio/2-hydroxy 8-oxo 4-thia 1-azabicyclo /4-2-0/ octane carboxylate de 1,1-diméthyl éthyle.

On opère comme au stade A de l'exemple 23 en partant de 1,48 g de mercaptoacétate de 1,1-diméthyl éthyle. On refroidit à -10 °C la solution de chlorure de 2-(1,1-diméthyl éthoxy) 2-oxo éthyl sulfényle obtenue, ajoute goutte à goutte une solution de 1,7 g de diazopyruvate dans 5 cm3 de chlorure de méthylène, laisse remonter la température à ≃ 20 °C, maintient l'agitation pendant 20 minutes, filtre et obtient une solution de 3-chloro 3-/2-(1,1-diméthyl éthoxy) 2-oxo éthyl thio/ pyruvate de tertbutyle. A celle-ci, on ajoute 20 cm3 de chlorure de méthylène, 2,78 g de 4-mercaptométhyl 3-/2-(2-tritylaminothiazol-4-yl) 2-méthoxyimino acétamido/ 2-oxo 1-azétidine et 1,68 cm3 de triéthylamine. Après une heure d'agitation, on filtre le mélange réactionnel, lave le filtrat avec de l'acide chlorhydrique N, une solution de bicarbonate de sodium, de l'eau saturée en chlorure de sodium, sèche et concentre à sec. On chromatographie sur silice le résidu par un mélange chlorure de méthylène-acétone (9-1) et obtient 1,07 g de produit attendu.

Spectre UV

1) dans EtOH
infl : 236 nm $E_1^1$ : 273 $\epsilon$ = 23000
infl : 258 nm $E_1^1$ : 167
infl : 266 nm $E_1^1$ : 144 $\epsilon$ = 12200
infl : 296 nm $E_1^1$ : 74 $\epsilon$ = 6300
2) dans EtOH/HCl 0,1N
maxi : 279 nm $E_1^1$ : 182 $\epsilon$ = 15400
infl : 289 nm $E_1^1$ : 164 $\epsilon$ = 13900

Le mercapto acétate de 1,1-diméthyl éthyle utilisé au début de l'exemple a été préparé de la manière suivante :

On refroidit à 0 °/2 °C un mélange de 8,81 g de O-éthyl dithiocarbonate de potassium dans 20 cm3 d'acétone, ajoute sous agitation, 9,79 g de bromoacétate de 1,1-diméthyl éthyle en 10 minutes. Après 1 heure d'agitation à température ambiante, on verse le mélange réactionnel dans 200 cm3 d'éther, filtre et concentre à sec. On reprend le résidu dans 100 cm3 d'éther, filtre, évapore à nouveau et obtient 11,6 g de (éthoxy thio carbonyl) thio acétate de 1,1-diméthyl éthyle. On refroidit celui-ci à 0 °C sous atmosphère inerte et sous agitation, ajoute goutte à goutte, 1,62 g de 1,2-diaminoéthane et agite pendant 2 heuresà température ambiante. On ajoute alors 50 cm3 d'hexane, agite fortement pendant 10 minutes, sépare la phase hexanique et extrait le résidu à l'hexane. On lave la solution hexanique avec de l'acide chlorhydrique 0,1N, une solution de bicarbonate de sodium, sèche et concentre à sec. On distille le résidu sous pression réduite et sous atmosphère inerte et obtient 4,5 g de produit attendu $Eb_{21\ mm/Hg}$ = 72 °C.

STADE B :7-/2-(2-tritylaminothiazol-4-yl) 2-méthoxyimino acétamido/ 3-//2-(1,1-diméthyl éthoxy) 2-oxo éthyl/ thio/ 8-oxo 4-thia 1-azabicyclo /4-2-0/ oct-2-ène 2-carboxylate de 1,1-diméthyl éthyle.

On dissout 675 mg de produit obtenu ci-dessus dans 13,5 cm3 de pyridine, ajoute sous atmosphère inerte 592 mg de tétraiodure de diphosphore, agite pendant 1/2 heure puis ajoute encore 320 mg de ce

dernier et maintient l'agitation pendant 2 heures. On ajoute alors 50 cm3 d'acétate d'éthyle, filtre, concentre à sec sous pression réduite. On dissout le résidu dans du chlorure de méthylène, lave avec de l'acide chlorhydrique N, une solution de bicarbonate de sodium, à l'eau saturée en chlorure de sodium, sèche et concentre à sec. On chromatographie le résidu sur silice par un mélange chlorure de méthylène-acétone (10-0,75) et obtient, après cristallisation dans l'éther, 312 mg de produit attendu.

Spectre UV

1) dans EtOH
infl : 233 nm $E_1^1$ : 351
infl : 263 nm $E_1^1$ : 190 $\epsilon$ = 15500
infl : 271 nm $E_1^1$ : 166
maxi : 308-309 nm $E_1^1$ : 191 $\epsilon$ = 15600
2) dans EtOH/HCl 0,1N
maxi : 282 nm $E_1^1$ : 240
maxi : 292 nm $E_1^1$ : 246 $\epsilon$ = 20100
infl : 301 nm $E_1^1$ : 232
infl : 312 nm $E_1^1$ : 185 $\epsilon$ = 15100

STADE C : Acide 7-/2-(2-aminothiazol-4-yl) 2-méthoxyimino acétamido/ 3-(carboxy méthyl thio) 8-oxo 4-thia 1-azabicyclo /4-2-0/ oct-2-ène 2-carboxylique racémique.

On agite à 50°C pendant 2 heures et demie unesolution de 200 mg de produit obtenu au stade B dans 4 cm3 d'acide formique aqueux a 66 %. On opère comme suit : on filtre la solution refroidie puis l'évapore. On redissout le résidu dans un mélange méthanol-acétonitrile que l'on évapore. On répète le traitement. On redissout le résidu dans 5 cm3 de méthanol, ajoute du noir, agite pendant 2 heures puis filtre. On ajoute sous agitation 30 cm3 d'éther goutte à goutte pendant 1 heure puis essore le précipité blanc. On sèche à 50°C et obtient 53 mg de produit attendu. F=190°-200°C avec décomposition
Ultra-violet (dans l'éthanol + HCl 0,1N)
maxi : 266 nm $E_1^1$ : 308 = 14600
maxi : 213 nm $E_1^1$ : 308 = 14600
infl : 310 nm $E_1^1$ : 230 = 10900

EXEMPLE 25 : Acide 7-/2-(2-aminothiazol-4-yl) 2-méthoxyimino acétamido/ 3-/(2-aminoéthyl) thio/ 8-oxo 4-thia 1-azabicyclo /4-2-0/ oct-2-èn 2-carboxylique isomère syn racémique.

STADE A :7-/2-(2-tritylamino thiazol-4-yl) 2-méthoxyimino acétamido/ 3-/2-//(1,1-diméthyl éthoxy) carbonyl/amino/ éthyl/ thio 2-hydroxy 8-oxo 4-thia 1-azabicyclo /4-2-0/ octane 2-carboxylate de 1,1-diméthyl éthyle isomère syn racémique.

a) On ajoute sous agitation et sous azote à -20°C, une solution de 1,3 cm3 de chlore dans le tétrachlorure de carbone (titre 127,5 mg/l) à une solution de 881,3 mg de disulfure de bis //(1,1-diméthyl éthoxy) carbonyl/ amino/ éthyle dans 15 cm3 de chlorure de méthylène. On laisse remonter la température à 0°C. On agite 5 minutes.
b) On ajoute à cette solution à +5°C une solution de 783 mg de diazopyruvate de tertbutyle en solution dans 10 cm3 de chlorure de méthylène. On laisse remonter à température ambiante.
c) A ce mélange réactionnel, on ajoute successivement 2,85 g de 4-mercaptométhyl 3-/2-(2-tritylaminothiazol-4-yl) 2-méthoxyimino acétamido/ 2-oxo 1-azétidine solvaté à 11 % de chlorure de méthylène et 581,8 mg de triéthylamine. On opère ensuite comme au stade A de l'exemple 23 et isole 3,857 g de produit brut que l'on chromatographie sur silice (éluant : chlorure de méthylène-acétate d'éthyle 75:25). On isole finalement 1,539 g de produit pur F=#210°C.

| Analyse $C_{43}H_{50}N_6O_8S_3$ : 875,1 | | | | |
|---|---|---|---|---|
| Calculé<br>Trouvé | C% 59,02<br>59,2 | H% 5,76<br>5,8 | N% 9,60<br>9,5 | S% 10,99<br>10,7 |

Le disulfure de bis //(1,1-diméthyl éthoxy) carbonyl/ amino/ éthyle utilisé au départ du stade A a été préparé comme suit :

On ajoute 3,04 g de triéthylamine à une suspensionde 3,38 g de dichlorhydrate de cystamine dans 30 cm3 de méthanol puis, en 5 minutes, une solution de 7,25 g de carbonate de ditertbutyle dans 10 cm3 de méthanol. On agite 45 minutes à température ambiante, évapore à sec, reprend par 75 cm3 d'acétate d'éthyle, essore le chlorhydrate de triéthylamine. Le filtrat est évaporé sous pression réduite. On obtient 5,64 g d'un solide incolore F = 104°-106°C.

STADE B :7-/2-(2-tritylamino thiazol-4-yl) 2-méthoxyimino acétamido/ 8-oxo 3-//2-//(1,1-diméthyl éthoxy) carbonyl/ amino/ éthyl/ thio/ 4-thia 1-azabicyclo /4-2-0/ oct-2-èn 2-carboxylate de 1,1-diméthyl éthyle isomère syn racémique.

On opère comme au stade B de l'exemple 23 au départ de 1,321 g de produit obtenu au stade A en solution dans 13 cm3 de pyridine et 1,719 g de tétraiodure de diphosphore. On obtient 1,08 g de produit brut. On joint 135 mg de produit obtenu dans une autre manipulation et chromatographie sur silice (éluant : chlorure de méthylène-acétate d'éthyle 85:15). On obtient 539 mg de produit attendu.

| Analyse $C_{43}H_{48}N_6O_7S_3$ : 857,09 |
|---|

STADE C :Acide 7-/2-(2-aminothiazol-4-yl) 2-méthoxyimino acétamido/ 3-/(2-amino éthyl) thio/ 8-oxo 4-thia 1-azabicyclo /4-2-0/ oct-2-èn 2-carboxylique isomèresyn racémique.

On dissout 428,5 mg de produit obtenu au stade B dans 5 cm3 d'acide formique à 66 %. On porte la solution en bain d'huile à 50°-55°C. On chauffe pendant 5 heures, ajoute 5 cm3 d'eau, essore l'insoluble, le lave avec 0,2 cm3 d'eau (104 mg de triphénylcarbinol). On évapore à sec et obtient 277 mg de résine. On triture dans 5 cm3 de méthanol. On laisse une nuit et le lendemain, on essore un solide ocre : poids 162 mg F#270°C.

Spectre UV

1) dans EtOH + diméthyl sulfoxyde
maxi : 298 nm $E_1^1$ : 345 nm $\epsilon$ = 15800
2) dans EtOH/HCl N/10
2 inflexions : 275 nm $E_1^1$ : 327
310 nm $E_1^1$ : 256 $\epsilon$ = 11700
1 maximum : 285 nm $E_1^1$ : 342 $\epsilon$ = 15700

EXEMPLE 26

En opérant comme dans les exemples décrits précédemment, on obtient les produits suivants :

a) acide 7-/2-(2-aminothiazol-4-yl) 2-méthoxyimino acétamido/ 3-/(5-méthyl 1,3,4-thiadiazol 2-yl) thio/ 8-oxo 4-thia 1-azabicyclo /4-2-0/ oct-2-ène 2-carboxylique syn cis racémique ;

Spectre UV

- dans EtOH + HCl N/10
max : 228 nm $E_1^1$ : 340 $\epsilon$ = 17500
max : 262 nm $E_1^1$ : 363 $\epsilon$ = 18600
inflexion : 281 nm $E_1^1$ : 336 $\epsilon$ = 17300
inflexion : 291 nm $E_1^1$ : 314
inflexion : 309 nm $E_1^1$ : 250 $\epsilon$ = 12800

b) acide 7-/2-(2-amino thiazol-4-yl) 2-méthoxyimino acétamido/ 8-oxo 3-/ (5-/méthylthio/ 1,3,4-thiadiazol-2-yl) thio/ 4-thia 1-azabicyclo /4-2-0/ oct-2-ène 2-carboxylique syn cis racémique ;

Spectre UV

1) dans EtOH
max : 225 nm $E_1^1$ : 331 $\epsilon$ = 18000
max : 299-300 nm $E_1^1$ : 305 $\epsilon$ = 16600
2) dans EtOH + HCl N/10
maxi : 224 nm $E_1^1$ : 245 $\epsilon$ = 13400
inflexion : 264 nm $E_1^1$ : 275
inflexion : 275 nm $E_1^1$ : 305
max : 286 nm $E_1^1$ : 333 $\epsilon$ = 18200
inflexion : 310 nm $E_1^1$ : 254 $\epsilon$ = 13900

c) trifluoro acétate de l'acide 7-/2- (2-aminothiazol-4-yl) 2-méthoxyimino acétamido/ 3-/(5-méthyl 1,3,4-oxadiazol-2-yl) thio/ 8-oxo 4-thia 1-azabicyclo /4-2-0/ oct-2-ène 2-carboxylique syn cis racémique ;

Spectre UV

1) dans EtOH
max : 228 nm $E_1^1$ : 343 $\epsilon$ = 21000
max : 306 nm $E_1^1$ : 221 $\epsilon$ = 13500
2) dans EtOH + HCl N/10
inflexion : 222 nm $E_1^1$ : 274
max : 261-262 nm $E_1^1$ : 245 $\epsilon$ = 15000
max : 284 nm $E_1^1$ : 238 $\epsilon$ = 14500
inflexion : 310 nm $E_1^1$ : 190 $\epsilon$ = 11600

EXEMPLE 27 :Acide 7-/2-(2-aminothiazol-4-yl) 2-méthoxyimino acétamido/ 8-oxo 3-/(1-méthyl 1H tétrazol-5-yl) thio méthyl/ 4-thia 1-azabicyclo /4-2-0/ oct-2-ène 2-carboxylique syn cis racémique.

STADE A :2-(1,1-diméthyl éthyl) diméthyl silyloxy/ éthanol.

A 20 ° C, on mélange sous azote :
- 18 g de chlorure de tertbutyl de méthyl silyle,
- 150 cm3 de dichloro méthane,
- 17,5 cm3 de diméthyl formamide,
- 33,6 cm3 d'éthylène glycol.

Après dissolution complète, on ajoute en 5 minutes, 20,1 cm3 de triéthylamine puis ajoute 1,8 g de 4-diméthyl amino pyridine. Après 2 h 45 d'agitation, on verse la solution dans 120 cm3 d'eau et neutralise avec de l'acide chlorhydrique N (environ 40 cm3) de manière à obtenir un pH de 3. On décante, réextrait la phase aqueuse avec 20 cm3 de pentane, puis on lave la phase organique avec 60 cm3 d'eau qu'on réextrait avec 20 cm3 de pentane. La phase organique est séchée et distillée sous pression réduite. Après rectification, on isole 13,9 g de produit attendu. Eb 16 mm/Hg = 82 ° -86 ° C

STADE B :2-/(1,1-diméthyl éthyl) diméthyl silyloxy/ acétaldéhyde.

Sous agitation et azote, on dissout 4,71 cm3 de chlorure d'oxalyle dans 120 cm3 de dichlorométhane. On refroidit à -70 ° C et introduit en 12 minutes en maintenant la température à -65 ° C, une solution comprenant 8,6 cm3 de diméthylsulfoxyde et 26 cm3 de dichlorométhane. Puis après 10 minutes de contact à cette température, on inroduit en 12 minutes la solution suivante à -65 ° C qui comprend 8,81 g de 2/(1,1-diméthyl éthyl) diméthyl silyloxy/ éthanol obtenu au stade A, 50 cm3 de dichlorométhane et 8,86 cm3 de pyridine. Après 15 minutes de contact à cette température, on ajoute en 8 minutes à -65 ° C, 35 cm3 de triéthylamine. A + 13 ° C, on neutralise par de l'acide chlorhydrique N, de manière à obtenir un pH de 4. On décante, réextrait avec 50 cm3 de dichlorométhane, sèche la phase organique et distille sous pression réduite. Le produit brut est chromatographié sur silice en éluant au dichlorométhane. On isole finalement 7,95 g de produit attendu.

STADE C : 2-chloro 3-(tertbutyl diméthyl silyloxy méthyl) oxirane carboxylate de 1,1-diméthyl éthyle.

On opère comme au stade B de l'exemple 1 en prenant la précaution d'introduire en même temps les solutions de terbutylate de potassium et 7,95 g d'aldéhyde obtenu au stade B à -20 ° C, dans une solution de dichloroacétate de 1,1-diméthyl éthyle et obtient, après chromatographie sur silice (éluant : hexane-dichlorométhane 6-4), 9,4 g de produit attendu.

STADE D :7-/2-(2-tritylaminothiazol-4-yl) 2-méthoxyimino acétamido/ 8-oxo 2-hydroxy 3-/(1,1-diméthyl éthyl) diméthyl silyloxy méthyl/ 4-thia 1-azabicyclo /4-2-0/ octane-2-carboxylate de 1,1-diméthyl éthyle.

On opère comme au stade C de l'exemple 1, avec un temps de réaction de 16 heures. Après chromatographie sur silice et élution au dichlorométhaneacétate d'éthyle(75-25), on isole à partir de 8,31 g de 4-mercaptométhyl 3 /2-(2-tritylamino thiazol-4-yl) 2-méthoxyimino acétamido/ 2-oxol-azétidine cis syn racémique et du produit obtenu ci-dessus, 9,09 g de produit cyclisé.

STADE E :7-/2-(2-tritylaminothiazol-4-yl) 2-méthoxyimino acétamido/ 8-oxo 3-/(1,1-diméthyl éthyl) diméthyl silyloxy méthyl/ 4-thia 1-azabicyclo /4-2-0/ oct-2-ène 2-carboxylate de 1,1-diméthyl éthyle.

On opère comme précédemment dans l'exemple 1, stade D. Le contact est réduit à 55 mm. Le mélange réactionnel est versé dans l'eau, acidifié avec de l'acide chlorhydrique 2N jusqu'à pH 1,4 et extrait à l'acétate d'éthyle. En partant de 9,09 g de produit obtenu ci-dessus, on isole, après chromatographie, 4 g de produit attendu.

Spectre UV

- dans l'éthanol
infl : 233 nm $E_1^1$ : 364
infl : 265 nm $E_1^1$ : 173
max : 302 nm $E_1^1$ : 229 $\epsilon$ = 18900

STADE F :7-/2-(2-tritylamino thiazol-4-yl) 2-méthoxyimino acétamido/ 8-oxo 3-hydroxy méthyl 4-thia 1-azabicyclo /4-2-0/ oct-2-ène 2-carboxylate de 1,1-diméthyl éthyle.

On met en suspension 2,595 g de dérivé silylé obtenu au stade E dans 30 cm3 d'acétone et 4,7 cm3 d'acide chlorhydrique N. La solution devient limpide peu à peu et après 3 heures d'agitation, on ajoute 7,7 cm3 d'eau bicarbonatée saturée et distille l'acétone sous pression réduite. On ajoute 20 cm3 de dichlorométhane, agite, décante, réextrait et sèche la phase organique et distille sous pression réduite. La gomme résiduelle est dissoute avec 5,5 cm3 d'acétate d'éthyle, auquel on ajoute 43 cm3 d'éther. Après 3 h 15 d'agitation, on filtre les cristaux formés, rince et sèche. On isole ainsi 2,232 g de produit attendu.

STADE G : 7-/2-(2-tritylaminothiazol-4-yl) 2-méthoxyimino acétamido/ 8-oxo 3-chlorométhyl 4-thia 1-azabicyclo /4-2-0/ oct-2-ène 2-carboxylate de 1,1-diméthyl éthyle.

On dissout 623 mg d'alcool obtenu au stade F dans 8 cm3 de dichlorométhane avec 834 mg de chlorure de tosyle et introduit en 20 minutes, la solution suivante comprenant 534 mg de 4-diméthyl amino pyridine et 5 cm3 de dichlorométhane. Après 1 heure d'agitation, on ajoute 2,2 cm3 d'acide chlorhydrique N, agite, décante, sèche la phase organique. Après distillation sous pression réduite, le résidu est chromatographié sur silice én éluant avec du dichlorométhane-acétate d'éthyle (9-1). Le produit attendu cristallise dans l'éther ; on en obtient 245 mg.

Spectre UV

- dans EtOH
infl : 224 nm $E_1^1$ : 441 $\epsilon$ = 38200
infl : 264 nm $E_1^1$ : 179
infl : 271 nm $E_1^1$ : 164
max : 306 nm $E_1^1$ : 222 $\epsilon$ = 19200

STADE H : 7-/2-(2-tritylamino thiazol-4-yl) 2-méthoxyimino acétamido/ 8-oxo 3-/(1-méthyl 1H tétrazol-5-yl) thiométhyl/ 8-oxo 4-thia 1-azabicyclo /4-2-0/ oct-2-ène 2-carboxylate de 1,1-diméthyl éthyle.

On dissout 301 mg du produit obtenu au stade G, 114 mg du sel de sodium du 1-méthyl 5-mercapto 1,2,3,4-tétrazole dans 3 cm3 de diméthylformamide. Après 1 h 40 d'agitation, on verse la solution dans 30 cm3 d'eau et extrait à l'acétate d'éthyle. La phase organique est séchée et distillée sous pression réduite. La gomme obtenue est dissoute dans de l'acétate d'éthyle (2 cm3) et on ajoute de l'éther jusqu'à la limite de la solubilité. On filtre les cristaux obtenus après 45 minutes, rince à l'éther, sèche et obtient en 2 jets, 283 mg du dérivé cherché.

STADE I : Acide 7-/2-(2-aminothiazol-4-yl) 2-méthoxyimino acétamido/ 8-oxo 3-/(1-méthyl 1H tétrazol 5-y1) thiométhyl/ 4-thia 1-azabicyclo /4-2-0/ oct-2-ène 2-carboxylique.

On opère comme dans l'exemple 1, stade E, mais le produit brut est dissout dans l'eau bicarbonatée et acidifiée à pH 4, puis filtré après avoir distillé une grande partie de l'eau. A partir de 371 mg de produit obtenu au stade H, on isole 61 mg de produit attendu.

Spectre UV

1) dans EtOH
max : 302 nm $E_1^1$ : 295 $\epsilon$ = 15100
2) dans EtOH/HCl N/10
infl : 273 nm $E_1^1$ : 304
max : 285 nm $E_1^1$ : 318 $\epsilon$ = 16300
infl : 292 nm $E_1^1$ : 312 $\epsilon$ = 16000
infl : 309 nm $E_1^1$ : 245 $\epsilon$ = 12500

EXEMPLE 28 : trifluorométhane sulfonate de 1-//7-/2-(2-aminothiazol-4-yl)2-méthoxyimino acétamido/ 2-carboxy 8-oxo 4-thia 1-azabicyclo /4-2-0/ oct-2-ène 3-yl/ méthyl/ pyridinium.

STADE A : trifluorométhane sulfonate de 1/7-/2-(2-tritylaminothiazol-4-yl) 2-méthoxyimino acétamido/ 2-carboxylate de 1,1-diméthyl éthyle 8-oxo 4-thia 1-azabicyclo /4-2-0/ oct-2-ène 3-yl/ méthyl pyridinium.

On dissout 356 mg d'alcool obtenu au stade F de l'exemple 27 dans 4 cm3 d'une solution contenant 0,4 cm3 de pyridine dilués à 10 cm3 dans du dichlorométhane. On refroidit sous azote à -70 ° C et introduit, en 5 minutes, 2,6 cm3 d'une solution comprenant 0,84 cm3 d'anhydride trifluorométhane sulfonique étendu à 20 cm3 avec du dichlorométhane (130 %). La solution est abandonnée au réchauffement spontané pendant 1 h 05 puis le solvant est évaporé sous vide. On dissout le résidu dans l'éthanol et laisse cristalliser le sel durant 17 heures. On filtre, rince et sèche 215 mg de produit attendu.

STADE B : trifluorométhane sulfonate de 1-//7-/2-(2-aminothiazol-4-yl) 2-méthoxyimino acétamido/ 2-carboxy 8-oxo 4-thia 1-azabicyclo /4-2-0/ oct-2-ène 3-yl/ méthyl/ pyridinium.

On dissout les 215 mg d'ester tritylé obtenus au stade A dans 0,9 cm3 d'acide trifluoroacétique et abandonne le milieu en atmosphère close pendant 50 minutes puis dilue avec 10 cm3 d'éther isopropylique, filtre le précipité, rince et sèche. Le précipité est dissout dans de l'éthanol auquel est ajouté 2 gouttes de pyridine. Après 15 minutes, on filtre les cristaux formés, rince, sèche et obtient 65 mg de produit attendu.

Spectre UV

- dans EtOH
infl : 215 nm $E_1^1$ : 410
infl : 256 nm $E_1^1$ : 276 $\epsilon$ = 17200
infl : 264 nm $E_1^1$ : 251
max : 293 nm $E_1^1$ : 279 $\epsilon$ = 17400

EXEMPLE 29 : trifluorométhane sulfonate de 1-//7-(2-aminothiazol-4-yl) méthoxyimino acétamido/ 2-carboxy 8-oxo 4-thia 1-azabicyclo /4-2-0/ oct-2-ène-3) yl/ mèthyl/ (6,7-dihydro) 5H 1-pyrindinium.

STADE A :trifluorométhane sulfonate de 1-//7-(2-triphényl méthyl amino thiazol-4-yl) méthoxyimino acétamido/ 2-carboxylate de 1,1-diméthyl éthyle 8-oxo 4-thia 1-azabicyclo /4-2-0/ oct-2-ène-3-yl/ méthyl/ (6,7-dihydro) 5H 1-pyrindinium.

On dissout 356 mg d'alcool obtenu au stade F de l'exemple 27 dans 2 cm3 de dichlorométhane et 4 cm3 d'une solution comprenant 0,58 cm3 de 6,7-dihydro 5H 1-pyrindane ajustés à 10 cm3 dans du dichlorométhane, puis on refroidit à -70 ° C et introduit en 2 minutes, 3 cm3 d'une solution comprenant 0,84 cm3 d'anhydride trifluorométhane sulfonique étendu à 20 cm3 dans le dichlorométhane. Ensuite, la solution est réchauffée à la température ambiante pendant 2 heures et distillée sous pression réduite. On empâte la résidu à l'éther, filtre, dissout l'insoluble dans l'éthanol et filtre les cristaux formés après 1 heure de repos. On isole ainsi 281 mg de produit attendu.

STADE B : trifluorométhane sulfonate de 1-//7-(2-aminothiazol-4-yl) méthoxyimino acétamido/ 2-carboxy 8-oxo 4-thia 1-azabicyclo /4-2-0/ oct-2-ène-3)yl/ méthyl/ (6,7-dihydro) 5H 1-pyrindinium.

On procède comme à l'exemple 28, à partir de 281 mg du produit obtenu au stade A. On isole 93 mg de produit attendu.

Spectre UV

- dans EtOH/HCl N/10
max : 223 nm $E_1^1$ : 315 $\epsilon$ = 20900
max : 282 nm $E_1^1$ : 394 $\epsilon$ = 26200
infl : 310 nm $E_1^1$ : 200 $\epsilon$ = 13300

EXEMPLE 30 : Acide 7-/2-(2-aminothiazol-4-yl) 2-méthoxyimino acétamido/ 8-oxo 3-/(1-carboxy méthyl 1H-tétrazol-5-yl) thio/ 4-thia 1-azabicyclo /4-2-0/ oct -2-ène 2-carboxylique syn cis racémique.

En opérant comme à l'exemple 3 au départ d'un produit dont le groupement carboxylique est protégé sous forme d'ester diphénylméthylique, on obtient le produit attendu.

Spectre UV

1) dans l'éthanol
2 max : 229 nm $E_1^1$ : 393 $\epsilon$ = 21300
305 nm $E_1^1$ : 261 $\epsilon$ = 14100
2) dans l'éthanol HCl N/10
2 inflexions : 220 nm $E_1^1$ : 306
310 nm $E_1^1$ : 228 $\epsilon$ = 12300
2 maximum : 263 nm $E_1^1$ : 293 $\epsilon$ = 15900
284 nm $E_1^1$ : 287 $\epsilon$ = 15500

EXEMPLE 31 : Acide 7-/2-(2-aminothiazol-4-yl) 2-méthoxyimino acétamido/ 8-oxo 3-/(phényl méthoxy méthyl)/ 4-thia 1-azabicyclo /4-2-0/ oct-2-ène 2-carboxylique syn cis racémique.

En opérant comme à l'exemple 2, on obtient le produit attendu.

| Analyse | | | | |
|---|---|---|---|---|
| Calculé | C% 50,09 | H% 4,20 | N% 13,91 | S% 12,73 |
| Trouvé | 50,0 | 4,2 | 13,6 | 12,4 |

Exemple 32 : Lactone de l'acide 7-/2-(2-amino thiazol-4-yl) 2-méthoxyimino acétamido 8-oxo 3-hydroxy méthyl 4-thia 1-azabicyclo /4.2.0/ oct-2-èn 2-carboxylique.

On dissout 356 mg de produit obtenu à l'exemple 27, stade F, dans 1,4 cm3 d'acide trifluoroacétique et laisse 50 minutes dans un récipient bouché. On ajoute 15 cm3 d'éther isopropylique, essore le trifluoroacétate formé et obtient 246 mg de produit brut. On le dissout dans l'éthanol, agite une demi-heure, filtre, sèche et obtient 151 mg de produit attendu.

| Analyse : $C_{14}H_{13}O_5S_2$ = 395,42 | | | | |
|---|---|---|---|---|
| Calculé : | C% 42,52 | H% 3,31 | N% 17,71 | S% 16,22 |
| Trouvé : | 42,5 | 3,3 | 17,3 | 15,9. |

Exemple 33 : Trifluoroacétate de trifluorométhane sulfonate de 6-/7-/2-(2-amino thiazol-4-yl) 2-méthoxy imino acétamido/ 2-carboxy 8-oxo 4-thia 1-azabicyclo /4.2.0/ oct-2-èn 3-yl/ méthyl thiéno /2,3-c/ pyridinium syn cis, racémique.

On refroidit à -70° C, 0,214 g de produit obtenu à l'exemple 27 stade F, 4 cm3 de chlorure de méthylène et 203 g de thiéno /2,3-c/ pyridine, ajoute 0,1 cm3 d'anhydride trifluorométhane sulfonique et laisse revenir à température ambiante, sous agitation.

On verse le mélange réactionnel dans 40 cm3 d'eau, agite, décante et extrait au chlorure de méthylène. On sèche les phases organiques, concentre à sec sous pression réduite à 35° C environ. On chromatographie le résidu sur silice élue avec un mélange chlorure de méthylène - méthanol (92-8) et isole 0,104 g de produit. On dissout ce dernier dans 0,45 cm3 d'acide trifluoroacétique, agite 50 minutes à température ambiante et ajoute 4,5 cm3 d'éther éthylique. On agite 5 minutes, essore, rince à l'éther et obtient 75 mg de produit attendu.

Spectre RMN (DMSO)

6,82 pppm $H_5$ du thiazole
7,94 - 8,01 ppm $H_2$ du thiényle
8,91 - 8,96 ppm $H_3$ du thiényle
8,55 - 8,63 ppm H en $\beta$ du $N^+$
8,88 ppm H en 5 du thiéno pyridine
10,0 ppm H en 7 du thiéno pyridine.

Exemple 34 : Trifluorométhane sulfonate de //7-/2-(2-amino thiazol 4-yl 2-méthoxyimino acétamido/ 8-oxo 2-carboxy 4-thia 1-azabicyclo /4.2.0/ 2-èn 3-yl/ méthyl/ triméthyl ammonium syn, cis, racémique.

Stade A : Trifluorométhane sulfonate de //7-2-(2-trityl amino thiazol-4-yl) 2-méthoxyimino acétamido/ 8-oxo 2-(1,1-diméthyl éthyloxy) carbonyl 4-thia 1-azabicyclo /4.2.0/ oct-2-èn 3-yl/ méthyl/ triméthyl ammonium, syn, cis, racémique.

On dissout 392 mg du produit obtenu à l'exemple 27, stade F, dans 8,8 cm3 de chlorure de méthylène, refroidit à -70° C et ajoute 2,75 cm3 d'une solution 0,74M fraîchement préparée de triméthylamine dans le chlorure de méthylène, puis toujours à cette température, goutte à goutte, en 5 minutes, 3,3 cm3 d'une solution d'anhydride trifluorométhane sulfonique fraîchement préparée dans le chlorure de méthylène (0,42 cm3 q.s.p. 10 cm3).

Après 15 minutes à -70° C, on ajoute 2 gouttes de solution de triméthylamine pour amener le pH à 4 et distille le solvant. On ajoute de l'eau au résidu et extrait à l'acétate d'éthyle, sèche la phase organique et concentre à sec sous pression réduite. On triture le résidu dans l'éther, filtre et obtient 481 mg de produit attendu.

Stade B : Trifluorométhane sulfonate de //7-/2-(2-amino thiazol-4-yl) 2-méthoxyimino acétamido/ 8-oxo 2-carboxy 4-thia 1-azabicyclo /4.2.0/ oct-2-èn 3-yl/ méthyl/ triméthyl ammonium syn, cis, racémique.

On opère comme au stade E de l'exemple 1 à partir de 481 mg de produit obtenu ci-dessus et obtient 84 mg de produit attendu.

Spectre RMN (DMSO)

6,83 ppm $H_5$ du thiazole
4,61 - 4,77 ppm
4,2 - 4,36 ppm } H du $CH_2$ en α du $N^+$
3,14 ppm H du triméthylamino.

Exemple 35 : Trifluorométhane sulfonate de //7-/2-(2-amino thiazol-4)yl) 2-méthoxyimino acétamido/ 8-oxo 2-carboxy 4-thia 1-azabicyclo /4.2.0/ oct-2-èn 3-yl/ méthyl/ triéthyl ammonium syn, cis, racémique.

On opère comme à l'exemple 34 à partir de triéthylamine et obtient 61 mg de produit attendu.

Spectre RMN (DMSO)

4,2 - 4,37 ppm
4,64 - 4,7 ppm H en du $N^+$
3,86 ppm H du méthoxy
5,66 ppm H en 7.

Exemple 36 : Trifluorométhane sulfonate de //7-/2-(2-aminothiazol-4-yl) 2-méthoxyimino acétamido/ 8-oxo 2-carboxy 4-thia 1-azabicyclo /4.2.0/ oct-2-ène 3-yl/ méthyl/ diméthyl 2-propynyl ammonium syn cis racémique.

On opère comme à l'exemple 34 à partir de 2-diméthylaminopropyne et obtient 90 mg de produit attendu.

Spectre RMN (DMSO)

6,81 ppm $H_5$ du thiazole
3,14 ppm H du diméthyl ammonium
2,84 ppm H en 3 du propynyle.

Exemple 37 : Trifluorométhane sulfonate de /7-(2-(2-amino thiazol-4-yl) 2-méthoxyimino acétamido/ 2-carboxy 8-oxo 4-thia 1-azabicyclo /4.2.0/ oct-2-ène 3-yl/ méthyl 4-méthyl morpholinium.

On opère comme à l'exemple 34 à partir de 4-méthyl morpholine et obtient 115 mg de produit attendu.

Spectre RMN (DMSO)

6,82 ppm $H_5$ du thiazole
3,22 ppm H du $CH_3$ en 1 du morpholinium
4,33 - 4,5 ppm
4,7 - 4,9 ppm H du $CH_2$ en du morpholinium.

Exemple 38 : Trifluoroacétate de trifluorométhane sulfonate de l'acide 7-/2-(2-amino thiazol-4-yl) 2-méthoxy imino acétamido/ 8-oxo 4-thia 3-(diméthylaminométhyl) 1-azabicyclo /4.2.0/ oct-2-ène 2-carboxylique, syn cis racémique.

On opère comme à l'exemple 34 à partir de 3-diméthylamino propionitrile. Après traitement à l'acide trifluoroacétique, on obtient 289 mg de produit 3-diméthylamino méthylé sous forme de trifluoroacétate.

Spectre RMN (DMSO)

6,84 ppm H en 5 du thiazole
2,81 ppm H du diméthylamino
Les produits ci-après ont été préparés comme indiqué à l'exemple 38 à partir des produits correspondants.

Exemple 39 : Trifluoroacétate de trifluorométhane sulfonate de //7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ 1-méthyl pyrrolidinium, syn cis racémique.

Spectre RMN (DMSO)

6,88 ppm $H_5$ du thiazole
3,03 ppm H du $CH_3$-$N^+$.

Exemple 40 :Trifluoroacétate de trifluorométhane sulfonate de //7-/2-(2-amino thiazol-4-yl) 2-méthoxy imino acétamido/ 8-oxo 4-thia 2-carboxy 1-azabicyclo /4.2.0/ oct-2-èn-3-yl/ méthyl/ 4-diméthylamino pyridinium syn cis, racémique.

6,81 ppm $H_5$ du thiazole
3,2 ppm H de $(CH_3)_2N$-
5 - 5,16 ppm
5,35-5,41 ppm } H du $-CH_2-$ en 3.

Exemple 41 : Trifluoroacétate de trifluorométhane sulfonate de //7-(2-(2-amino thiazol-4-yl) 2-méthoxy imino acétamido/ 8-oxo 4-thia 2-carboxy 1-azabicyclo /4.2.0/ oct-2-èn-3-yl/ méthyl/ 5-éthyl 2-méthyl pyridinium syn cis, racémique.

6,87 ppm $H_5$ du thiazole
2,77 ppm H du $CH_3$- en 2 du pyridinium
1,17 - 1,25 - 1,33 ppm
2,68 - 2,76 - 2,84 - 2,92 ppm } H de l'éthyle en 5 du pyridinium.

Exemple 42 : Trifluoroacétate de trifluorométhane sulfonate de //7-/2-(2-amino thiazol-4-yl) 2-méthoxy imino acétamido/ 8-oxo 4-thia 2-carboxy 1-azabicyclo /4.2.0/ oct-2-èn-3-yl/ méthyl/ 3-méthyl 1H-imidazolium. syn cis, racémique.

6,83 ppm $H_5$ du thiazole
3,85 et 3,51 ppm H du $CH_3$-N.

Exemple 43 : Trifluoroacétate de trifluorométhane sulfonate de //7-/2-(2-amino thiazol-4-yl)2-méthoxy imino acétamido/ 8-oxo 4-thia 2-carboxy 1-azabicyclo /4.2.0/ oct-2-èn-3-yl/ méthyl/ 2-(1-méthyl éthyl) pyridinium syn cis, racémique.

6,83 ppm $H_5$ du thiazole
1,31 - 1,38
1,35 - 1,42 ppm H des méthyles géminés.

Exemple 44 : Trifluoroacétate de trifluorométhane sulfonate de //7-/2-(2-amino thiazol-4-yl) 2-méthoxy imino acétamido/ 8-oxo 4-thia 2-carboxy 1-azabicyclo /4.2.0/ oct-2-èn-3-yl/ méthyl/ 4-cyclopropyl pyridinium syn cis, racémique.

6,81 ppm $H_5$ du thiazole
1,05 à 1,58 ppm H des $-CH_2$ du cyclopropyle
2,27 ppm H angulaire du cyclopropyle.

Exemple 45 : Trifluoroacétate de trifluorométhane sulfonate de //7-/2- (2-amino thiazol-4-yl)2-méthoxy imino acétamido/ 8-oxo 4-thia 2-carboxy 1-azabicyclo /4.2.0/ oct-2-èn-3-yl/ méthyl/ 1-pyrazinium syn cis, racémique.

6,82 ppm $H_5$ du thiazole
9,3 et 9,61 ppm H aromatiques.

Exemple 46 :Trifluoroacétate de trifluorométhane sulfonate de 6-//7-/2-(2-amino thiazol-4-yl) 2-méthoxy imino acétamido/ 8-oxo 4-thia 2-carboxy 1-azabicyclo /4.2.0/ oct-2-èn-3-yl/ méthyl/ 7-méthyl 6-thiéno /2,3-c/ pyridinium syn cis, racémique.

6,82 ppm $H_5$ du thiazole
5,93 ppm H du $-CH_2-N^+$

Exemple 47 : Trifluoroacétate de trifluorométhane sulfonate de //7-/2-(2-amino thiazol-4-yl) 2-méthoxy imino acétamido/ 8-oxo 4-thia 2-carboxy 1-azabicyclo /4.2.0/ oct-2-èn-3-yl/ méthyl/ 3-méthoxy pyridinium syn cis, racémique.

6,82 ppm $H_5$ du thiazole
4,02 ppm du méthoxy
5,4 - 5,56 ppm }
5,77- 5,94 ppm } H du $-CH_2-N^+$

Exemple 48 : Trifluoroacétate de trifluorométhane sulfonate de //7-/2-(2-amino thiazol-4-yl) 2-méthoxy imino acétamido/ 8-oxo 4-thia 2-carboxy 1-azabicyclo /4.2.0/ oct-2-èn-3-yl/ méthyl/ 4-éthoxy pyridinium syn cis, racémique.

6,82 ppm $H_5$ du thiazole
1,34 - 1,42 - 1,51 }
4,31 - 4,39 - 4,48 - 4,56 ppm } H de l'éthoxy.

Exemple 49 : Trifluoroacétate de trifluorométhane sulfonate de //7-/2-(2-amino thiazol-4-yl) 2-méthoxy imino acétamido/ 8-oxo 4-thia 2-caboxy 1-azabicyclo /4.2.0/ oct-2-èn-3-yl/ méthyl/ 4-méthoxy méthyl pyridinym syn cis, racémique.

6,83 ppm $H_5$ du thiazole
4,82 - 4,71 ppm H du $O-CH_2-$
3,44 - 3,42 ppm H du $CH_3$ du $CH_3-O-CH_2$

Exemple 50 : Trifluoroacétate de trifluorométhane sulfonate de 7-//7-/2-(2-amino thiazol-4-yl) 2-méthoxy imino acétamido/ 8-oxo 4-thia 2-carboxy 1-azabicyclo /4.2.0/ oct-2-èn-3-yl/ méthyl/ thiéno /2,3-b/ pyridinium syn cis, racémique.

6,87 ppm $H_5$ du thiazole
5,94 - 6,11
6,17 - 6,34 ppm } H du $-CH_2-N^+$

7,91 - 7,97
8,3 - 8,35 ppm } H aromatiques du thiophène.

Exemple 51 : Trifluoroacétate de trifluorométhane sulfonate de //7-/2-(2-amino thiazol-4-yl) 2-méthoxy imino acétamido/ 8-oxo 4-thia 2-carboxy 1-azabicyclo /4.2.0/ oct-2-èn-3-yl/ méthyl/ 3-(1-méthyléthyloxy) pyridinium syn cis, racémique.

6,84 ppm $H_5$ du thiazole
4,91 ppm H du $O-CH-(CH_3)_2$
1,34 - 1,41 ppm H des $CH_3$ du $O-CH-(CH_3)_2$

Exemple 52 : Trifluoroacétate de 2-amino (méthoxyimino N-(1,7-dioxo 1,3,5,5a 6,7-hexahydro 3-hydroxy azéto /1,2-d/ furo /3,4-b/ /1,4/ thiazin-6-yl)4-thiazol acétamide syn cis, racémique

Stade A : 7-/2-(2-tritylamino thiazol-4-yl) 2-méthoxy imino acétamido/ 3-formyl 8-oxo 4-thia 1-azabicyclo /4.2.0/ oct-2-èn 2-carboxylate de 1,1-diméthyl éthyle.

On refroidit à -70 ° C, 8,2 cm3 d'une solution de chlorure d'oxalyle (1 cm3 q.s.p. 100 cm3 de chlorure de méthylène). On ajoute 8,2 cm3 d'une solution de diméthyl sulfoxyde (1,7 cm3 q.s.p. 100 cm3 de chlorure de méthylène), agite 5 minutes et ajoute, goutte à goutte, 356 mg du produit obtenu au stade F de l'exemple 27 dans 10 cm3 de chlorure de méthylène en maintenant l'agitation. En 2 minutes et à -70 ° C, on ajoute 5 cm3 d'une solution de triéthylamine (2 cm3 q.s.p. 20 cm3 de chlorure de méthylène). On amène à -45 ° C et laisse 10 minutes, ajoute de l'eau et 2,5 cm3 d'acide chlorhydrique N, agite , décante et réextrait au chlorure de méthylène, sèche et concentre à sec sous pression réduite. On dissout le résidu dans l'acétate d'éthyle, ajoute de l'éther, filtre après 15 minutes et isole 256 mg de produit attendu.

Stade B : Trifluoroacétate de 2-amino $\alpha$-(méthoxyimino) N-(1,7-dioxo 1,3,5,5a 6,7-hexahydro 3-hydroxy azéto /1,2-d/ furo /3,4-b/ /1,4/ thiazin-6-yl) 4-thiazol acétamide syn cis, racémique.

On opère comme à l'exemple 34, stade B, à partir de 85 mg de produit obtenu au stade A ci-dessus et obtient 50 mg de produit attendu.

Spectre RMN (DMSO)

6,9 ppm $H_5$ du thiazole
3,91 ppm H du $N-O-CH_3$
6,28 ppm H du -CH-OH

Exemple 53 : Trifluorométhane sulfonate de 7-//7-/2-(2-amino thiazol-4-yl) 2-méthoxy imino acétamido/ 2-carboxy 8-oxo 4-thia 1-azabicyclo /4.2.0/ oct-2-èn 3-yl/ méthyl/ thiéno /2,3-b/ pyridinium syn 6S, 7S.

On opère comme à l'exemple 34 à partir du 7-/2-(2-tritylamino thiazol-4-yl) 2-méthoxy imino acétamido/ 8-oxo 3-hydroxyméthyl 4-thia 1-azabicyclo /4.2.0/ oct-2-èn 2-carboxylate de 1,1-diméthyl éthyle syn 6S,7S obtenu par le même procédé que le produit de l'exemple 27, stade F et à partir du thiéno /2,3-b/ pyridine.

Spectre RMN (DMSO)

6,8 ppm $H_5$ du thiazole
7,87 - 7,94
8,25 - 8,33 ppm H du thiophène
5,68 ppm $H_7$

Exemple 54 : Trifluoroacétate du trifluorométhane sulfonate de /7-/2-(2-amino thiazol-4-yl) 2-éthoxy imino acétamido/ 8-oxo 4-thia 2-carboxy 1-azabicyclo /4.2.0/ oct-2-èn 3-yl/ méthyl/ pyridinium syn cis, racémique.

Stade A : 7-/2-(2-tritylamino thiazol-4-yl) 2-éthoxy imino acétamido/ 3-hydroxy méthyl 8-oxo 4-thia 1-azabicyclo /4.2.0/ oct-2-èn 2-carboxylate de 1,1-diméthyl éthyle syn cis, racémique.

On dissout 549 mg d'acide 2-/2-tritylamino thiazol-4-yl/ 2-éthoxy imino acétique syn dans 5,5 cm3 d'acétone et 0,18 cm3 de triéthylamine et ajoute aussitôt 228 mg de chlorure de tosyle puis agite 45 minutes.

On ajoute au mélange réactionnel une solution comprenant 285 mg de produit obtenu à la préparation 1 dans 1 cm3 d'eau, 1,5 cm3 d'une solution 1M de bicarbonate de sodium et 2 cm3 d'acétone et agite 35 minutes. On distille l'acétone sous pression réduite et extrait au chlorure de méthylène. On sèche les phases organiques, concentre à sec, chromatographie le résidu sur silice et sous pression en éluant par un mélange chlorure de méthylène - acétate d'éthyle (75-25). On isole 602 mg de produit attendu.

Stade B : Trifluorométhane sulfonate de /7-/2-(2-tritylamino thiazol-4-yl) 2-éthoxy imino acétamido/ 8-oxo 4-thia 2-/(1,1-diméthyl éthoxy) carbonyl/ 1-azabicyclo /4.2.0/ oct-2-èn-3-yl/ méthyl pyridinium syn cis, racémique.

On opère comme à l'exemple 34 à partir de 218 mg de 7-/2-(2-tritylamino thiazol-4yl) 2-éthoxy imino acétamido/ 3-hydroxy méthyl 8-oxo 4-thia 1-azabicyclo /4.2.0/ oct-2-èn 2-carboxylate de 1,1-diméthyl éthyle obtenu ci-dessus et à partir de 3,4 cm3 d'une solution de pyridine à 0,4 cm3 pour 10 cm3 de chlorure de méthylène. On obtient 158 mg de produit attendu.

Stade C : Trifluoroacétate du trifluorométhane sulfonate de //7-/2-(2-amino thiazol-4-yl) 2-éthoxy imino acétamido/ 8-oxo 4-thia 2-carboxy 1-azabicyclo /4.2.0/ oct-2-èn-3-yl/ méthyl pyridinium syn cis, racémique.

On opère comme à l'exemple 37, stade B et obtient 160 mg de produit attendu.

Spectre RMN (DMSO)

6,83 ppm $H_5$ du thiazole
1,12 - 1,20 - 1,28
4,01 - 4,09 - 4,17 - 4,26 ppm H du N-O-éthyle

Préparation I : 7-amino 3-hydroxyméthyl 8-oxo 4-thia 1-azabicyclo /4.2.0/ oct-2-èn 2-carboxylate de 1,1-diméthyl éthyle cis, racémique.

Stade A : 3-(1,3-dihydro 1,3-dioxo 2H-iso indol-2-yl) 4-oxo 2-mercaptométhyl azétidine cis.

On mélange à chaud et sous agitation 10,6 g de 3-(1,3-dihydro 1,3-dioxo 2H-isoindol 2-yl) 4-oxo 2-chlorométhyl azétidine cis (brevet belge 894.785), 48 g d'iodure de sodium et 125 cm3 de diméthyl formamide et maintient les conditions pendant 6 heures. Après refroidissement à température ambiante, on ajoute 520 cm3 d'eau et extrait à l'acétate d'éthyle. On sèche les phases organiques, concentre à sec sous pression réduite, reprend le résidu cristallisé avec très peu d'acétate d'éthyle, essore, rince à l'acétate d'éthyle, empâte à l'éther et obtient 9,75 g de dérivé iodé.

On prépare une solution de 2,1 g d'hydrogénosulfure de sodium dans 40 cm3 de diméthyl formamide, refroidit à 0-5°C et ajoute sous atmosphère inerte, goutte à goutte, 8,9 g de dérivé iodé puis agite 10 minutes. On acidifie avec 1,5 cm3 d'acide acétique, ajoute 220 cm3 d'eau et extrait à l'acétate d'éthyle.

On sèche les phases organiques, concentre à sec sous pression réduite, chromatographie sur silice le résidu en éluant à l'acétate d'éthyle. On évapore le solvant, reprend le résidu cristallisé à l'éther et obtient 4 g de produit attendu..

Stade B : 7-(1,3-dihydro 1,3-dioxo 2H-iso indol 2-yl) 8-oxo 3-/(1,1-diméthyl éthyl) diméthyl silyloxy méthyl/ 4-thia 1-azabicyclo /4.2.0/ oct-2-èn 2-carboxylate de 1,1-diméthyl éthyle cis, racémique.

On opère comme aux stades D et E de l'exemple 27 à partir du produit obtenu au stade ci-dessus et obtient le produit attendu.

Stade C : 7-amino 3-hydroxy méthyl 8-oxo 4-thia 1-azabicyclo /4.2.0/ oct-2-èn 2-carboxylate de 1,1-diméthyl éthyle cis, racémique.

On dissout 1,592 g de produit obtenu ci-dessus dans 4 cm3 de diméthyl formamide et ajoute, goutte à goutte en 12 minutes, 3,3 cm3 d'une solution d'hydrazine M dans le diméthyl formamide. On distille le diméthyl formamide à 45 °C sous pression réduite, ajoute 10 cm3 d'eau puis 3,3 cm3 d'acide chlorhydrique N et 8 cm3 d'éthanol et agite pendant 2 heures. On filtre, distille l'éthanol, ajoute de l'acétate d'éthyle, agite, décante, alcalinise la phase aqueuse avec du bicarbonate de sodium et extrait le précipité à l'acétate d'éthyle. On sèche les phases organiques et concentre à sec sous pression réduite. On reprend le résidu à l'acétate d'éthyle, ajoute de l'éther, triture, filtre et obtient 604 mg de produit attendu.

Les composés ci-après ont été préparés comme indiqué à l'exemple 54 à partir des produits correspondants.

Exemple 55 : Trifluoroacétate de trifluorométhane sulfonate de /7-/2-(2-amino thiazol-4-yl) 2-carboxy méthoxy imino acétamido/ 2-carboxy 8-oxo 4-thia 1-azabicyclo /4.2.0/ oct-2-èn-3-yl/ méthyl pyridinium syn cis, racémique.

Spectre RMN (DMSO)

6,83 ppm $H_5$ du thiazole
4,6 ppm H du

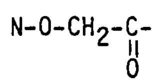

Exemple 56 : Trifluoroacétate de trifluorométhane sulfonate de /7-/2-(2-amino thiazol-4-yl) 2-carboxy méthoxy imino acétamido/2-carboxy 8-oxo 4-thia 1-azabicyclo /4.2.0/ oct-2-èn-3-yl/ méthyl triméthylammonium syn cis, racémique.

Spectre RMN (DMSO)

6,87 ppm $H_5$ du thiazole
3,15 ppm H du triméthyle $N^+$
4,64 ppm H du

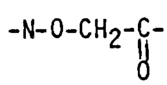

Exemple 57 : Trifluoroacétate de trifluorométhane sulfonate de /7-/2-(2-amino thiazol-4-yl) 2-(1-carboxy 1-méthyl éthoxy) imino acétamido/ 2-carboxy 8-oxo 4-thia 1-azabicyclo /4.2.0/ oct-2-èn-3-yl/ méthyl pyridinium syn cis, racémique.

Spectre RMN (DMSO)

6,78 ppm $H_5$ du thiazole
1,4 ppm H des méthyles géminés

Exemple 58 : Trifluoroacétate de trifluorométhane sulfonate de /7-/2-(2-amino thiazol-4-yl) 2-(1-carboxy 1-méthyl éthoxy) imino acétamido/ 2-carboxy 8-oxo 4-thia 1-azabicyclo /4.2.0/ oct-2-èn-3-yl/ méthyl triméthylammonium syn cis, racémique.

Spectre RMN (DMSO)

6,78 ppm $H_5$ du thiazole
3,14 ppm H du triméthyl $N^+$
1,44 ppm H des méthyles géminés.

Exemple 59 :Trifluoroacétate de trifluorométhane sulfonate de /7-/2-(2-amino thiazol-4-yl) 2-phénoxy imino acétamido/ 2-carboxy 8-oxo 4-thia 1-azabicyclo /4.2.0/ oct-2-èn-3-yl/ méthyl pyridinium syn cis, racémique.

Spectre RMN (DMSO)

7,06 ppm $H_5$ du thiazole
7 à 7,44 ppm H du 0-Φ

Exemple 60 : Trifluoroacétate de trifluorométhane sulfonate de /7-/2-(2-amino thiazol-4-yl) 2-difluorométhoxy imino acétamido/ 2-carboxy 8-oxo 4-thia 1-azabicyclo /4.2.0/ oct-2-èn-3-yl/ méthyl pyridinium syn cis, racémique.

Spectre RMN (DMSO)

7,08 ppm $H_5$ du thiazole
6,37 - 7,14 - 7,92 ppm H du $-CHF_2$

Exemple 61 : Trifluoroacétate de trifluorométhane sulfonate de /7-/2-(2-amino thiazol-4-yl) 2-difluorométhoxy imino acétamido/ 2-carboxy 8-oxo 4-thia 1-azabicyclo /4.2.0/ oct-2-èn-3-yl/ méthyl triméthylammonium syn cis, racémique.

Spectre RMN (DMSO)

7,11 ppm $H_5$ du thiazole
3,15 ppm H du triméthyl $N^+$

Exemple 62 : Trifluoroacétate de trifluorométhane sulfonate de /7-/2-(2-amino thiazol-4-yl) 2-(2-propényloxy) imino acétamido/ 2-carboxy 8-oxo 4-thia 1-azabicyclo /4.2.0/ oct-2-èn-3-yl/ méthyl pyridinium syn cis, racémique.

Spectre RMN (DMSO)

6,8 ppm $H_5$ du thiazole
5,11 - 6 ppm H du $CH_2$ = CH et $H_7$ $-CH_2O$

Exemple 63 : Trifluoroacétate de trifluorométhane sulfonate de /7-/2-(2-amino thiazol-4-yl) 2-(2-propényloxy) imino acétamido/ 2-carboxy 8-oxo 4-thia 1-azabicyclo /4.2.0/ oct-2-èn-3-yl/ méthyl triméthylammonium syn cis, racémique.

Spectre RMN (DMSO)

6,83 ppm $H_5$ du thiazole
3,14 ppm H du triméthyl $N^+$

Exemple 64 : Trifluoroacétate de trifluorométhane sulfonate de 1-/7-/2-(2-amino thiazol-4-yl) 2-cyano méthoxy imino acétamido/ 2-carboxy 8-oxo 4-thia 1-azabicyclo /4.2.0/ oct-2-èn 3-yl/ méthyl pyridinium syn cis, racémique.

Stade A : 7-/2-(2-tritylamino thiazol-4-yl) 2-cyano méthoxy imino acétamido/ 3-hydroxy méthyle 8-oxo 4-thia 1-azabicyclo /4.2.0/ oct-2-èn 2-carboxylate de 1,1-diméthyl éthyle.

On opère comme au stade A de l'exemple 54 à partir de l'acide 2-(2-tritylamino thiazol-4-yl) 2-cyano méthoxy imino acétique et obtient le produit attendu.

Stade B : Trifluorométhane sulfonate de 1-/7-/2-(2-tritylamino thiazol-4-yl) 2-cyano méthoxy imino acétamido/ 2-/1,1-diméthyl éthoxy carbonyl/ 8-oxo 4-thia 1-azabicyclo /4.2.0/ oct-2-èn 3-yl/ méthyl pyridinium.

On opère comme à l'exemple 34 stade A à partir du produit obtenu ci-dessus et de pyridine et obtient le produit attendu.

Stade C : Trifluoroacétate de trifluorométhane sulfonate de 1-/7-/2-(2-amino thiazol-4-yl) 2-cyano méthoxy imino acétamido/ 2-carboxy 8-oxo 4-thia 1-azabicyclo /4.2.0/ oct-2-èn 3-yl/ méthyl pyridinium.

On opère comme à l'exemple 38 à partir de 116 mg du produit obtenu ci-dessus et obtient 91 mg de produit attendu.

Spectre RMN (DMSO)

6,91 - 6,94 ppm $H_5$ du thiazole
5,03 ppm H de l'acétonitrile
8,14 à 8,31 ppm H en β de $N^+$ }
8,61 à 8,77 ppm H en γ de $N^+$ } pyridine
9,13 à 9,2 ppm H en α de $N^+$. }

Exemple 65 : Trifluoroacétate de l'acide 7-/2-(2-amino thiazol-4-yl) 2-méthoxy imino acétamido 3-//4-(2-chloro 2-propényl) 1,4,5,6-tétrahydro 5,6-dioxo 1,2,4-triazin-3-yl/ thio méthyl/ 8-oxo 4-thia 1-azabicyclo /4.2.0/ oct-2-èn 2-carboxylique syn cis, racémique.

Stade A : 7-/2-(2-tritylamino thiazol-4-yl) 2-méthoxy imino acétamido 3-//4-(2-chloro 2-propényl) 1,4,5,6-tétrahydro 5,6-dioxo 1,2,4-triazin-3-yl/ thiométhyl/ 8-oxo 4-thia 1-azabicyclo /4.2.0/ oct-2-èn carboxylate de 1,1-diméthyl éthyle syn cis, racémique.

On mélange 146 mg de 7-/2-(2-tritylamino thiazol-4-yl) 2-méthoxy imino acétamido 3-chlorométhyl 8-oxo 4-thia 1-azabicyclo /4.2.0/ oct-2-èn 2-carboxylate de 1,1-diméthyl éthyle syn cis, racémique (voir exemple 27, stade G), 90 mg de 4-(2-chloro 2-propényl) 1,4,5,6-tétrahydro 1,2,4-triazin-3-mercapto 5,6-dione, 83 mg de carbonate de potassium, 60 mg d'iodure de sodium et 1,5 cm3 de diméthyl formamide. On agite pendant trois heures, ajoute 20 cm3 d'eau, ajuste le pH à 2 avec de l'acide chlorhydrique N et extrait à l'acétate d'éthyle. On lave à l'eau la phase organique, la sèche et concentre à sec sous pression réduite. On chromatographie le résidu sous pression, sur silice en éluant par un mélange chlorure de méthylène - acétate d'éthyle (1-1). On obtient 113 mg de produit attendu.

Stade B : Trifluoroacétate de l'acide 7-/2-(2-amino thiazol-4-yl) 2-méthoxy imino acétamido 3-//4-(2-chloro 2-propényl) 1,4,5,6-tétrahydro 5,6-dioxo 1,2,4-triazin-3-yl/ thio méthyl/ 8-oxo 4-thia 1-azabicyclo /4.2.0/ oct-2-èn 2-carboxylique syn cis, racémique.

On dissout 113 mg du produit obtenu ci-dessus dans 0,5 cm3 d'acide trifluoroacétique, laisse en contact 50 minutes puis ajoute 5 cm3 d'éther. Après filtration, on obtient 70 mg de produit attendu.

Spectre RMN (DMSO)

6,83 ppm $H_5$ du thiazole
5,44 à 5,71 ppm $H_7$ et les H éthyléniques
11,5 ppm H de l'hydroxyle

Exemple 66 : Trifluoroacétate de l'acide 7-/2-(2-amino thiazol-4-yl) 2-méthoxy imino acétamido 3-/(1,2,5,6-tétrahydro 2-méthyl 5,6-dioxo,1,2,4-triazin-3-yl) thio méthyl/ 8-oxo 4-thia 1-azabicyclo /4.2.0/ oct-2-èn 2-carboxylique syn cis, racémique.

On opère comme indiqué à l'exemple 65 en partant de 3-mercapto 2-méthyl 1,2,5,6-tétrahydro 1,2,4-triazin 5,6-dione et obtient 68 mg du produit attendu.

Spectre RMN (DMSO)

6,81 ppm $H_5$ du thiazole
7,3 ppm H mobile
3,99 ppm $H_6$

Exemple 67 : Trifluoroacétate de l'acide 7-/2-(2-amino thiazol-4-yl) 2-méthoxy imino acétamido 8-oxo 4-thia 3-/(4-nitrophényl) thio méthyl/ 1-azabicyclo /4.2.0/ oct-2-èn 2-carboxylique syn cis, racémique.

On opère comme indiqué dans l'exemple 65.

On élue la chromatographie par un mélange chlorure de méthylène-éther (82-18). On obtient 82 mg de produit attendu.

Spectre RMN (DMSO)

6,84 ppm $H_5$ du thiazole
7,54 - 7,64 ppm H en $\beta$ du $-NO_2$
8,21 - 8,11 ppm H en $\alpha$ du $-NO_2$

Exemple 68 : Acide 7-/2-(2-amino thiazol-4-yl) 2-méthoxy imino acétamido/ 3-méthyl thio 8-oxo 4-thia 1-azabicyclo /4.2.0/ oct-2-èn 2-carboxylique (6RS) (7RS) (Z).

On agite 1,3 g d'acide 7-/(1,1-diméthyl éthyl oxy) carbonyl amino/ 3-méthylthio 8-oxo 4-thia 1-azabicyclo /4.2.0/ oct-2-èn 2-carboxylique (6RS) (7RS) et 13 cm3 de nitrométhane, refroidit à 0°C et fait passer un courant d'acide chlorhydrique gazeux pendant 15 minutes. On ajoute 130 cm3 d'éther, essore, rince à l'éther et obtient 0,74 g de chlorhydrate de l'acide 7-amino 3-méthylthio 8-oxo 4-thia 1-azabicyclo /4.2.0/ oct-2-èn 2-carboxylique.

On dissout 1,33 g d'acide 2-(2-tritylamino thiazol-4-yl) 2-méthoxy imino acétique dans 13 cm3 d'acétone et 0,44 cm3 de triéthylamine. Après dissolution totale, on ajoute 0,6 g de chlorure de tosyle puis agite une heure à température ambiante.

On filtre l'insoluble, ajoute au filtrat les 0,74 g de produit obtenu ci-dessus, 7 cm3 de chlorure de méthylène et 1,1 cm3 de triéthylamine. On agite une heure à température ambiante et verse dans 100 cm3 d'acide chlorhydrique 0,1N. On extrait au chlorure de méthylène, sèche la phase organique, concentre à sec sous pression réduite à 35° C et obtient 1,97 g d'acide 7-/2-(2-tritylamino thiazol-4-yl) 2-méthoxy imino acétamido/ 3-méthyl thio 8-oxo 4-thia 1-azabicyclo /4.2.0/ oct-2-èn 2-carboxylique.

On reprend les 1,97 g de ce produit par 10 cm3 d'acide formique à 66 %, agite 15 minutes à 50° C, essore, chasse le solvant du filtrat sous pression réduite à 35° C. On reprend le résidu à l'éthanol puis chasse le solvant, reprend le résidu à l'eau, délite, essore, sèche et obtient 1,445 g de produit brut. On chromatographie sur silice, élue par un mélange chloroforme-acétone (1-1), recristallise le résidu dans l'éthanol, essore, rince, sèche vingt heures sous pression réduite à température ambiante et obtient 0,046 g de produit attendu.

| Analyse : $C_{14}H_{15}N_5O_5S_3$ = 429,50 | | | | |
|---|---|---|---|---|
| Calculé : | C% 39,2 | H% 3,5 | N% 16,3 | S% 22,4 |
| Trouvé : | 42,7 | 3,6 | 15,9 | 22 |

Préparation de l'acide 7-/(1,1-diméthyl éthyl oxy) carbonyl amino/ 3-méthyl thio 8-oxo 4-thia 1-azabicyclo /4.2.0/ oct-2-èn 2-carboxylique (6RS) (7RS) utilisé au départ de l'exemple

Stade A : Tosylate de glycinate de paranitrobenzyle.

On porte au reflux 190 g d'acide paratoluène sulfonique monhydraté dans 2 litres de toluène pendant une heure et demie en éliminant l'eau par entraînement azéotropique. On refroidit et ajoute en une fois 75 g de glycine et 135 g d'alcool paranitrobenzylique. On porte au reflux en distillant l'eau formée, pendant trois heures. Après refroidissement, on filtre, lave avec du toluène, empâte avec du méthanol, sèche sous pression réduite à 40 ° C et obtient 294 g de produit attendu.F = 210 ° C.

Stade B : Glycinate de paranitrobenzyle.

On met sous forte agitation un mélange constitué d'un litre de chlorure de méthylène, 500 cm3 d'eau et 100 g de tosylate de glycinate de paranitrobenzyle et ajoute, goutte à goutte, 26 cm3 d'hydroxyde de sodium 10 N. On agite encore 5 minutes après l'addition, décante et réextrait au chlorure de méthylène. On lave à l'eau les phases organiques, sèche et concentre à sec sous pression réduite à moins de 40 ° C. On obtient 52,3 g de produit attendu. F = 45-50 ° C.

Stade C : 4-/(2-phényl) éthényl 3-phtalimido azétidin-2-one 1-acétate de (4-nitrophényl) méthyle (3RS) (4SR) (E).

On mélange 105 g de glycinate de paranitrobenzyle, 100 g de sulfate de magnésium, 1000 cm3 de chlorure de méthylène et 65 cm3 de cinnamaldéhyde, filtre et refroidit à -70 ° C, ajoute 70 cm3 de triéthylamine puis en vingt minutes et à -70 ° C, une solution de 128 g de chlorure de l'acide phtalimido acétique dans 600 cm3 de chlorure de méthylène. On porte au reflux pendant une demi-heure.Après refroidissement, on verse le mélange réactionnel dans un mélange de 1000 cm3 d'acide 0,1N et 1000 cm3 d'eau glacée et agite pendant un quart d'heure. On décante et réextrait avec du chlorure de méthylène. On filtre les phases organiques, les lave à l'eau, sèche et concentre à sec sous pression réduite. On reprend le résidu dans 250 cm3 d'acétate d'éthyle, délite les résines et après vingt heures au froid, filtre, lave à l'acétate d'éthyle, sèche et obtient 154 g de produit attendu. F = 150 ° C.

Stade D : 4-/2-phényl éthényl/ 3-/(1,1-diméthyl éthyl oxy) carbonyl amino/ azétidin-2-one 1-acétate de nitrophényl méthyle (3RS) (4SR) (E).

On mélange 20 g de produit obtenu au stade C et 200 cm3 de dioxanne, ajoute à 0 ° C, 2,05 cm3 d'hydrate d'hydrazine et agite pendant une heure.

On ajoute 58,5 cm3 d'acide chlorhydrique N et laisse vingt heures à température ambiante. On filtre l'insoluble, évapore le dioxanne sous pression réduite et reprend le résidu par 150 cm3 d'eau, 70 cm3 de chlorure de méthylène et 70 cm3 d'une solution molaire de bicarbonate de sodium. On agite, décante, réextrait au chlorure de méthylène, lave à l'eau les phases organiques, sèche et concentre à sec sous pression réduite. On obtient 14,4 g de 3-amino 4-(2-phényl éthényl) azétidin-2-one 1-acétate de nitrophényl méthyle. On porte au reflux les 14,4 g du produit obtenu dans 150 cm3 de tétrahydrofuranne avec 13 cm3 de dicarbonate de terbutyle pendant une heure. On évapore le solvant, reprend le résidu cristallisé dans l'éther, agite un quart d'heure, filtre, sèche sous pression réduite et obtient 14,1 g de produit attendu. F = 160 ° C.

Stade E : 4-/(hydroxy) (méthyloxy) méthyl/ 3-/(1,1-diméthyl éthyl) oxy carbonyl amino/ azétidin-2-one 1-acétate de 4-nitrophényl méthyle (3RS) (4RS).

On dissout 18,5 g de 4-(2-phényl éthényl) 3-/(1,1-diméthyl éthyl oxy) carbonyl amino/ azétidin-2-one 1-acétate de 4-nitrophényl méthyle (3RS) (4RS) (E) dans 185 cm3 de chlorure de méthylène et 38 cm3 de

méthanol. On refroidit à -70°C et laisse passer un courant d'ozone dans le milieu réactionnel jusqu'à saturation puis après 5 minutes, on chasse l'ozone en excès et enfin, on ajoute à -70°C, 5,57 cm3 de diméthyl sulfure. On laisse revenir à température ambiante. Après une heure, on verse dans 350 cm3 d'acide chlorhydrique 0,1N et extrait au chlorure de méthylène. On lave à l'eau la phase organique, la sèche et concentre à sec sous pression réduite. On reprend le résidu dans l'éther, refroidit à 0-5°C pendant une heure, filtre, sèche et obtient 12,5 g de produit attendu. F = ~125°C.

Stade F : 4-hydroxy méthyl 3-/(1,1-diméthyl éthyl) oxy carbonyl amino/ azétidin-2-one 1-acétate de 4-nitrophényl méthyl (3RS) (4RS).

On refroidit à -20°C sous agitation 18 g de 4-/(hydroxy (méthyl oxy) méthyl/ 3-/(1,1-diméthyl éthyl) oxy carbonyl amino/ azétidin-2-one 1-acétate de 4-nitrophényl méthyle dans 180 cm3 de tétrahydrofuranne et 90 cm3 d'acide acétique. On ajoute d'un seul coup 5,25 g de cyano hydroborure de sodium. On laisse revenir à température ambiante et laisse une heure sous agitation et sous atmosphère inerte. On concentre à sec sous pression réduite. On ajoute 350 cm3 d'eau et extrait au chlorure de méthylène. On sèche la phase organique, concentre à sec sous pression réduite. On reprend le résidu à l'éther et obtient 15,3 g de produit attendu.

Stade G : 4-/(4-méthyl phényl) sulfonyl oxy méthyl/ 3-/(1,1-diméthyl éthyl) oxy carbonyl amino/ 2-oxo azétidin-1-yl 1-acétate de (4-nitrophényl) méthyle (3RS) (4RS).

On refroidit à 0°C, 15,9 g de chlorure de tosyle dans 170 cm3 de pyridine et ajoute 17 g de produit obtenu ci-dessus. On laisse sous agitation à température ambiante pendant vingt-quatre heures. On évapore la pyridine sous vide et ajoute 500 cm3 d'eau. On extrait au chlorure de méthylène, lave la phase organique par un mélange composé de 200 cm3 d'eau et 200 cm3 d'acide chlorhydrique 0,1N, la sèche et concentre à sec sous pression réduite. On reprend le résidu par 70 cm3 d'éthanol. Après une heure à 0-5°C, on filtre, lave, sèche et obtient 16,6 g de produit attendu. F = 132°C.

Stade H : 7-(1,1-diméthyl éthyl) oxy carbonyl amino/ 3-mercapto 8-oxo 4-thia 1-azabicyclo /4.2.0/ oct-2-èn 2-carboxylate de (4-nitrophényl) méthyle (6RS) (7RS).

On dissout 0,112 g de 4-/(4-méthyl phényl) sulfonyl oxy méthyl/ 3-/(1,1-diméthyl éthyl) oxy carbonyl amino/ azétidin-2-one 1-acétate de (4-nitrophényl) méthyle dans 2 cm3 de tétrahydrofuranne, refroidit à -70°C, ajoute goutte à goutte, en maintenant la température inférieure ou égale à -65°C, 0,4 cm3 d'une solution M de bis triméthyl silyl amidure de lithium dans le tétrahydrofuranne, agite 3 minutes et ajoute 0,2 cm3 de disulfure de carbone dans les mêmes conditions. On laisse remonter à température ambiante et verse dans 10 cm3 d'acide chlorhydrique 0,1N puis extrait au chlorure de méthylène. On sèche les phases organiques, concentre à sec sous pression réduite. On reprend le résidu sec par de l'éther, essore et obtient 0,067 g de produit attendu. F≃170°C.

Stade I : 7-/(1,1-diméthyl éthyl) oxy carbonyl amino/ 3-méthyl thio 8-oxo 4-thia 1-azabicyclo /4.2.0/ oct-2-èn 2-carboxylate de (4-nitrophényl) méthyle (6RS) (7RS).

On mélange 1,7 g de 7-/(1,1-diméthyl éthyl) oxy carbonyl amino/ 3-mercapto 8-oxo 4-thia 1-azabicyclo /4.2.0/ oct-2-èn 2-carboxylate de (4-nitrophényl) méthyle (6RS) (7RS), 25 cm3 d'une solution 0,5M de diazométhane dans le chlorure de méthylène. On agite 5 minutes à température ambiante et chasse le solvant sous pression réduite. On chromatographie sur silice, sous pression, le résidu en éluant par un mélange chlorure de méthylène - éther (9-1) et obtient 1,45 g de produit attendu. F = 154°C.

Stade J : Acide 7-/(1,1-diméthyl éthyl) oxy carbonyl amino/ 3-méthyl thio 8-oxo 4-thia 1-azabicyclo /4.2.0/ oct-2-èn 2-carboxylique (6RS) (7RS).

On fait une hydrogénation avec 1,45 g de 7-/(1,1-diméthyl éthyl) oxy carbonyl amino/ 3-méthyl thio 8-oxo 4-thia 1-azabicyclo /4.2.0/ oct-2-èn 2-carboxylate de (4-nitrophényl) méthyle (6RS) (7RS), 30 cm3 de tétradrofuranne, 30 cm3 de méthanol 1,45 g de carbone palladié à 10 % et une pression de 2 atmosphères d'hydrogène en agitant vivement. Après une heure, on amène à pression ambiante, sous atmosphère inerte et ajoute 4 cm3 d'acide chlorhydrique N. On essore et chasse le solvant sous pression réduite à 35°C. On reprend le résidu par 100 cm3 d'eau distillée et extrait au chlorure de méthylène. On sèche la phase

organique, la sèche et chasse le solvant sous pression réduite. On obtient 1,3 g de produit attendu.

Exemple 69 : Iodure de 1-/2-/7-/2-(2-aminothiazol-4-yl) 2-méthoxyimino acétamido/ 2-carboxy 8-oxo 4-thia 1-azabicyclo /4.2.0/ oct-2-èn-3-yl/ 2-thio éthyl/ 1-méthyl pyrrolidinium syn, cis racémique.

Stade A : 7-/2-(2-tritylamino thiazol-4-yl) 2-méthoxyimino acétamido/ 3-/(2-(1-pyrrolidinyl) éthyl) thio/ 8-oxo 4-thia 1-azabicyclo /4.2.0/ oct-2-èn 2-carboxylate de diphénylméthyle syn, cis, racémique.

On mélange 85 mg de chlorhydrate de N-chloroéthyl pyrrolidine, 2 cm3 de chlorure de méthylène et 210 ul de triéthylamine. On ajoute 412 mg de 7-/2-(2-tritylamino thiazol-4-yl) 2-méthoxyimino acétamido/ 3-mercapto 8-oxo 4-thia 1-azabicyclo /4.2.0/ oct-2-èn 2-carboxylate de diphényl méthyle dans 3 cm3 de chlorure de méthylène. On chauffe au reflux pendant 3 heures. Après refroidissement à 20°C, on verse le mélange réactionnel dans 10 cm3 d'eau glacée, agite pendant 5 minutes, décante et réextrait au chlorure de méthylène. On sèche les phases organiques réunies, concentre à sec sous pression réduite et chromatographie sur silice le résidu obtenu. On élue par un mélange chlorure de méthylène-acétone (1-1) et obtient, après reprise à l'éther isopropylique, 157 mg de produit attendu.

Stade B : Iodure de 1-/2-/7-/2-(2-tritylamino thiazol-4-yl) 2-méthoxy-imino acétamido/ 2-diphénylméthyloxy carbonyl 8-oxo 4-thia 1-azabyciclo /4.2.0/ oct-2-èn-3-yl/ thio éthyl/ 1-méthyl pyrrolidinium syn, cis, racémique.

On ajoute 0,1 cm3 d'iodure de méthyle à 122 mg du produit obtenu au stade A dans 2,4 cm3 d'acétonitrile et laisse en contact 30 minutes à 20°C. On concentre à sec sous pression réduite et obtient 143 mg de produit attendu.

Stade C : Iodure de 1-/2-/7-/2-(2-aminothiazol-4-yl) 2-méthoxyimino acétamido/ 2-carboxy 8-oxo 4-thia 1-azabicyclo /4.2.0/ oct-2-èn-3-yl/ 2-thio éthyl/ 1-méthyl pyrrolidinium syn, cis, racémique.

On chauffe 2 heures à 60°C sous agitation, 245 mg de produit obtenu comme au stade B dans 2,5 cm3 d'acide formique à 50% d'eau. On concentre sous pression réduite, cristallise le résidu dans l'éthanol 100°, agite 1 heure à 20°C, essore, rince avec très peu d'éthanol, puis empâte 2 fois avec 1 cm3 d'éther isopropylique. On obtient 110 mg de produit attendu.

Spectre RMN (DMSO)

6,83 ppm : H en 3 du thiazole
~ 2,11 ppm : H en 3 et 4 du pyrrolidinyle
3,05 ppm : H du méthyle en 1 du pyrrolidinyle

Préparation du 7-/2-(2-tritylamino thiazol-4-yl) 2-méthoxyimino acétamido/ 3-mercapto 8-oxo 4-thia 1-azabyciclo /4.2.0/ oct-2-èn -2-carboxylate de diphénylméthyle utilisé au départ de l'exemple.

Stade A : 3-/2-/2-tritylamino thiazol-4-yl/ 2-méthoxyimino acétamido/ 4-/4-méthyl phényl sulfonyloxy méthyl/ 2-oxo azétidin-1-yl 1-acétate de (4-nitrophényl) méthyle syn, cis, racémique.

On refroidit à 0/+5°C, 2,818 g de 4-/(4-méthyl phényl) sulfonyloxy méthyl/ 3-/(1,1-diméthyléthyloxy) carbonylamino/ 2-oxo azétidin-1-yl 1-acétate de (4-nitrophényl) méthyle (3RS) (4RS) dans 20 cm3 de chlorure de méthylène, ajoute 0,968 cm3 de trifluorométhyl sulfonate de triméthyl silyle, agite 5 minutes à 0/+5°C puis laisse remonter la température à 20°C. On ajoute 10 cm3 de méthanol, concentre à sec sous pression réduite à 30°C maximum et obtient le trifluorométhyl sulfonate de 3-amino 4-/(4-méthylphényl) sulfonyl méthyl/ 2-oxo azétidin-1-yl 1-acétate de 4-(nitrophényl) méthyle (A).

On agite pendant 1 heure 2,88 g d'acide 2-/(2-trityl amino thiazol-4-yl)/ 2-méthoxyimino acétique, 28,8 cm3 d'acétone, 0,9 cm3 de triéthylamine et 1,236 g de chlorure de tosyle. On refroidit à +10°C et y introduit la solution de (A) dans 15 cm3 de chlorure de méthylène avec 1,68 cm3 de triéthylamine et laisse à 20°C pendant 40 minutes et verse dans un mélange de 150 cm3 d'eau, 100 cm3 de chlorure de méthylène et 15 cm3 d'une solution aqueuse saturée de bicarbonate de sodium. On décante, réextrait au chlorure de méthylène, sèche lesphases organiques et évapore à sec sous pression réduite. On chromatographie sur silice le résidu, élue par un mélange chlorure de méthylène-acétone (9-1) et isole 3,46 g de

produit attendu.

Stade B : 3-/2-(2-tritylamino thiazol-4-yl) 2-méthoxyimino acétamido/ 4-/4-(méthylphényl) sulfonyloxy méthyl/ 2-oxo azétidin-1-yl 1-acétate de diphénylméthyl syn, cis, racémique.

On hydrogénolyse 7,210 g de produit obtenu au stade A dans 72 cm3 d'un mélange tétrahydrofuranne-méthanol (1-1) et 720 mg de charbon actif à 9,5% de palladium. On filtre, évapore les solvants sous pression réduite, reprend le résidu par 30 cm3 d'eau, 12 cm3 d'acide chlorhydrique N et extrait au chlorure de méthylène. On sèche la phase organique, concentre à sec sous pression réduite et obtient 7,58 g du dérivé N-acétique. On agite 40 minutes 9,813 g de benzophénone hydrazone, 150 cm3 d'éther, 11,27 g de sulfate de sodium sec et 26,3 g d'oxyde mercurique. On filtre, rince l'insoluble à l'éther et verse 45 cm3 du filtrat (contenant du diphényl diazométhane) sur les 7,58 g de produit obtenu ci-dessus dissous dans 70 cm3 de dioxanne. Après 40 minutes sous agitation à 20° C, on ajoute encore 22,5 cm3 de diphényl diazométhane en solution dans l'éther. Après un nouveau contact à 20° C de 40 minutes, on ajoute 1 cm3 d'acide acétique chasse les solvants sous pression réduite à 30° C maximum. On chromatographie le résidu sur silice, élue par un mélange chlorure de méthylène-acétone (9-1) et obtient 7,462 g de produit attendu.

Stade C : 7-/2-(2-tritylamino thiazol-4-yl) 2-méthoxyimino acétamido/ 3-mercapto 8-oxo 4-thia 1-azabicyclo /4.2.0/ oct-2-èn 2-carboxylate de diphénylméthyle.

On refroidit à -70° C, 1,840 g de produit obtenu ci-dessus dans 18,4 cm3 de tétrahydrofuranne, ajoute 4 cm3 d'une solution molaire de bis (triméthylsilyle) amidure de lithium, agite 15 minutes à -70° C sous atmosphère inerte. On ajoute 2 cm3 de sulfure de carbone et laisse pendant 40 minutes à 20° C puis, ajoute 113 ul d'acide acétique. On évapore les solvants sous pression réduite. On reprend le résidu à l'eau, extrait à l'acétate d'éthyle, lave à l'eau les phases organiques, sèche, évapore à sec sous pression réduite. On reprend le résidu dans 10 cm3 d'éther isopropylique, essore et obtient 1,270 g de produit attendu.

Spectre RMN ($CDCl_3$)

6,67 ppm : $H_5$ du thiazole
6,76 ppm : H du méthyl du diphényl
7,28 ppm : H du diphényl et du trityl.

En opérant comme à l'exemple 69, on a préparé les produits suivants :

Exemple 70 : Iodure de 4-/7-/2-(2-amino thiazol-4-yl) 2-méthoxyimino acétamido/ 2-carboxy 8-oxo 4-thia 1-azabicyclo /4.2.0/ oct-2-èn 3-yl/ thiométhyl/ 1-méthyl pyridinium syn, cis, racémique à partir du chlorhydrate de 4-chlorométhyl pyridine.

Spectre RMN (DMSO)

6,85 ppm : H en 5 du thiazole
7,95-8,05 ppm : H en 3 et 5 du pyridyle
8,85-8,95 ppm : H en 2 et 6 du pyridyle
4,28 ppm : H du méthyle en 1 du pyridyle.

Exemple 71 : Iodure de 2-/7-/2-(2-amino thiazol-4-yl) 2-méthoxyimino acétamido/ 2-carboxy 8-oxo 4-thia 1-azabicyclo /4.2.0/ oct-2-èn-3-yl/ thiométhyl 1-méthyl pyridinium syn, cis, racémique à partir du chlorhydrate de 2-chlorométhyl pyridine.

Spectre RMN (DMSO)

6,85 ppm : H en 5 du thiazole
7,73-7,81 ppm : H en 3 du pyridyle

7,96 à 8,1 ppm
8,37 à 8,54 ppm } H en 4 et 5 du pyridyle
9,02 à 9,1 ppm: H en 6 du pyridyle.

Exemple 72 : Trifluoroacétate de l'acide 7-/2-(2-aminothiazol-4-yl) 2-méthoxyimino acétamido 3-/(dihydro (4H) 5-hydroxy 4-oxo pyran-2-yl) méthylthio/ 8-oxo 4-thia 1-azabicyclo /4.2.0/ oct-2-èn-2-carboxyliquesyn, cis, racémique.

Stade A : 7-/2-(2-tritylamino thiazol-4-yl) 2-méthoxyimino acétamido/ 3-/(dihydro (4H) 5-hydroxy 4-oxo pyran-2-yl) méthylthio 8-oxo 4-thia 1-azabicyclo /4.2.0/ oct-2-èn-2-carboxylate de diphénylméthyle syn, cis, racémique.

On opère comme au stade A de l'exemple 69 à partir de 81,5 mg de 2-chlorométhyl dihydro (4H) 5-hydroxy 4-oxo pyran-2-yle. On obtient 225 mg de produit attendu.

Stade B : Trifluoroacétate de l'acide 7-/2-(2-aminothiazol-4-yl) 2-méthoxyimino acétamido 3-/(dihydro (4H) 5-hydroxy 4-oxo pyran-2-yl) méthylthio/ 8-oxo 4-thia 1-azabicyclo /4.2.0/ oct-2-èn-2-carboxylique.

A 0/+5°C, on agite 150 mg de produit obtenu ci-dessus et 2,5 cm3 de chlorure de méthylène à 10% d'acide trifluoroacétique pendant 2 heures 45 minutes. On ajoute 25 cm3 d'éther isopropylique, agite 10 minutes, sépare l'insoluble par centrifugation, sèche et obtient 90 mg de produit attendu.

Spectre RMN (DMSO)

6,88 ppm : H en 5 du thiazole
8,04 ppm : H en 6 du pyrannyle
6,37 ppm : H en 3 du pyrranyle
4,0 ppm : H en $\alpha$ du pyrannyle.

Exemple 73 : Acide 7-/2-(2-aminothiazol-4-yl) 2-méthoxyimino acétamido/ 3-cyanométhylthio 8-oxo 4-thia 1-azabicyclo /4.2.0/ oct-2-èn-2-carboxylique syn, cis, racémique.

On opère comme à l'exemple 72 à partir de bromure d'acétonitrile. On dissout le trifluoroacétate obtenu dans l'éthanol 100° et ajoute une trace de pyridine, amorce la cristallisation. On agite 1 heure à 20°C, essore, rince avec un minimum d'éthanol, empâte 2 fois avec de l'éther isopropylique et obtient le produit attendu.

Spectre RMN (DMSO)

6,85 ppm : H en 5 du thiazole
4,07 ppm : H de l'acétonitrile.

Exemple 74 : Acide 7-/2-(2-aminothiazol-4-yl) 2-méthoxyimino acétamido/ 3-/(5-méthyl 1,2,4-triazolo /1,5-a/ pyrimidin-7-yl) thio/ 8-oxo 4-thia 1-azabicyclo /4.2.0/ oct-2-èn-2-carboxylique syn, cis, racémique.

On opère comme à l'exemple 73 à partir du 7-chloro 5-méthyl 1,2,4-triazolo /1,5-a/ pyrimidine et obtient le produit attendu.

Spectre RMN (DMSO)

6,9 ppm : H en 5 du thiazole
8,67 ppm : H en 3 du triazole
7,03 ppm : H en 5 de la pyrimidine
2,65 ppm : H du $CH_3$.

Exemple 75 : Trifluoroacétate de l'acide 7-/2-(2-aminothiazol-4-yl) 2-méthoxyimino acétamido/ 3-chlorométhylthio 8-oxo 4-thia 1-azabicyclo /4.2.0/ oct-2-èn-2-carboxylique syn, cis, racémique.

Stade A : 7-/2-(2-tritylamino thiazol-4-yl) 2-méthoxyimino acétamido/ 3-chlorométhylthio 8-oxo 4-thia 1-azabicyclo /4.2.0/ oct-2-èn-2-carboxylate de diphénylméthyle.

A 412 mg de 7-/2-(2-tritylamino thiazol-4-yl) 2-méthoxyimino acétamido/ 3-mercapto 8-oxo 4-thia 1-azabicyclo /4.2.0/ oct-2-èn-2-carboxylate de diphénylméthyle dans 3 cm3 de diméthylformamide, on ajoute successivement 97 mg de iodochlorométhane et 75,9 mg de triéthylamine. Après une demi-heure de contact, on verse le mélange réactionnel sur 80 cm3 d'eau et 0,7 cm3 d'acide chlorhydrique N. On extrait au chlorure de méthylène, lave les phases organiques à l'eau, sèche et concentre à sec sous pression réduite. On chromatographie sur silice le résidu, élue avec un mélange chlorure de méthylène-acétate d'éthyle (95-5) et isole 199 mg de produit attendu.

Stade B : Trifluoroacétate de l'acide 7-/2-(2-amino thiazol-4-yl) 2-méthoxyimino acétamido/ 3-chlorométhylthio 8-oxo 4-thia 1-azabicyclo /4.2.0/ oct-2-èn-2-carboxylique syn, cis, racémique.

On agite 162 mg de produit obtenu ci-dessus dans 1,5 cm3 d'acide trifluoroacétique à 0 ° C et sous atmosphère inerte pendant 5 minutes. On verse la solution obtenue dans 25 cm3 d'éther isopropylique refroidi à 0 ° C, agite quelques minutes, centrifuge, remet le résidu en suspension dans 25 cm3 d'éther, centrifuge à nouveau et renouvelle cette opération. On sèche le trifluoroacétate sous pression réduite et obtient 88 mg de produit attendu.

Spectre RMN (DMSO)

6,91 ppm : H en 5 du thiazole
5,21 ppm : H du chlorométhyle.

Exemple 76 : Acide 7-/2-/2-amino thiazol-4-yl/ 2-méthoxyimino acétamido 3-/(3-éthoxy 3-oxo 1-((E) propényl) thio/ 8-oxo 4-thia 1-azabicyclo /4.2.0/ oct-2-èn 2-carboxylique syn cis, racémique.

Stade A : 7-/2-/2-tritylamino thiazol-4-yl) 2-méthoxy imino acétamido/ 3-/(3-éthoxy 3-oxo 1-(E) propényl) thio/ 8-oxo 4-thia 1-azabicyclo /4.2.0/ oct-2-èn 2-carboxylate de diphényl méthyle syn cis, racémique.

On opère comme au stade A de l'exemple 72 à partir de -bromoacrylate d'éthyle et obtient le produit attendu.

Stade B : Acide 7-/2-/2-amino thiazol-4-yl/ 2-méthoxy imino acétamido 3-/(3-éthoxy 3-oxo 1-(E) propényl) thio/ 8-oxo 4-thia 1-azabicyclo /4.2.0/ oct-2-èn 2-carboxylique syn cis, racémique.

On opère comme au stade B de l'exemple 69 à partir du produit obtenu en A avec de l'acide formique à 33 % d'eau et obtient le produit attendu.

Spectre RMN ( DMSO)

6,84 ppm H en 5 du thiazole
5,83 - 6 ppm H du propényle
1,14 - 1,22 - 1,3 ppm }
4,0 - 4,05 - 4,17 - 4,25 ppm } H de l'éthyle

En opérant comme à l'exemple 76 on a préparé les produits suivants.

Exemple 77 : Acide 7-/2-(2-amino thiazol-4-yl) 2-méthoxy imino acétamido/ 3-/(3-cyano 2-(E) propényl) thio/ 8-oxo 4-thia 1-azabicyclo /4.2.0/ oct-2-èn 2-carboxylique syn cis, racémique à partir du 4-bromocrotonitrile.

Spectre RMN (DMSO)

6,83 ppm H en 5 du thiazole
3,78 ppm H en 1 du propényle
6,13 ppm H éthylénique du propényle.

Exemple 78 : Acide 7-/2-(2-amino thiazol-4-yl) 2-méthoxy imino acétamido/ 3-thiocyanato 8-oxo 4-thia 1-azabicyclo /4.2.0/ oct-2-èn 2-carboxylique syn cis, racémique à partir de cyanure de tosyle.

Spectre RMN (DMSO)

6,82 ppm H en 5 du thiazole
5,55 ppm H en 7 de l'isocéphéme
3 ppm H en 8 de l'isocéphème
3,8 ppm H du méthoxyle.

Exemple 79 : Iodure de /7-/2-(2-amino thiazol-4-yl) 2-méthoxy imino acétamido/ 2-carboxy 8-oxo 4-thia 1-azabicyclo /4.2.0/ oct-2-èn 3-yl/ thio méthyl triméthylammonium syn cis, racémique.

Stade A : 7-/2-(2-tritylamino thiazol-4-yl) 2-méthoxy imino acétamido/ 3-iodométhyl thio 8-oxo 4-thia 1-azabicyclo /4.2.0/ oct-2-èn 2-carboxylate de diphényl méthyle syn cis, racémique.

On ajoute 225 mg d'iodure de sodium solide à une solution de 873 mg de dérivé chloré obtenu à l'exemple 75 dissous dans 6 cm3 d'acétonitrile. On agite quelques minutes et porte la solution au reflux pendant un quart d'heure. On verse le mélange réactionnel dans 150 cm3 d'eau, extrait au chlorure de méthylène, lave à l'eau les phases organiques, les sèche et concentre à sec sous pression réduite. On chromatographie sur silice le résidu, élue avec un mélange chlorure de méthylène-acétate d'éthyle (95-5) et isole 385 mg de produit attendu.

Stade B : Iodure de /7-/2-(2-tritylamino thiazol-4-yl) 2-méthoxy imino acétamido/ 2-diphényl méthyloxy carbonyl 8-oxo 4-thia 1-azabicyclo /4.2.0/ oct-2-èn 3-yl/ thio méthyl triméthyl ammonium syn cis, racémique

On dissout 144,6 mg de dérivé 2-iodo obtenu ci-dessus dans 1 cm3 de tétrahydrofuranne, ajoute 0,6 cm3 d'une solution 2,5M de triméthylamine dans l'éther. Après 16 heures de contact, on verse goutte à goutte le mélange réactionnel dans 10 cm3 d'éther sous agitation. On agite encore un quart d'heure après addition, essore, sèche et obtient 131 mg de produit attendu.

Stade C : Iodure de /7-/2-(2-amino thiazol-4-yl) 2-méthoxy imino acétamido/ 2-carboxy 8-oxo 4-thia 1-azabicyclo /4.2.0/ ot-2-èn 3-yl/ thio méthyl triméthyl ammonium syn cis, racémique.

On met en suspension et agite sous atmosphère inerte, 109 mg de produit obtenu au stade B dans 2,2 cm3 d'acide formique à 66 % jusqu'à la fin de réaction. On concentre à 40°C sous pression réduite (1mmHg), reprend le résidu à l'eau, puis 2 fois à l'èthanol 100° en évaporant à chaque fois dans les mêmes conditions. On triture le résidu dans 15 cm3 d'éther, essore, lave à l'éther, sèche et obtient 52 mg de produit attendu.

Spectre RMN (DMSO)

6,84 ppm H en 5 du thiazole
4,71 - 4,86 ppm }
5,04 - 5,2 ppm } H du thiométhyle.

Exemple 80 : Iodure de /7-/2-(2-amino thiazol-4-yl) 2-méthoxy imino acétamido/ 2-carboxy 8-oxo 4-thia 1-azabicyclo /4.2.0/ oct-2-èn 3-yl/ thio méthyl pyridinium syn cis, racémique.

Stade A : Iodure de /7-/2-(2-tritylamino thiazol-4-yl) 2-méthoxy imino acétamido/ 2-diphényl méthyl oxy carbonyl 8-oxo 4-thia 1-azabicyclo /4.2.0/ oct-2-èn 3-yl/ thio méthyl pyridinium syn cis, racémique.

On mélange 144,6 mg de dérivé 2-iodo obtenu au stade A de l'exemple 79 et 1,5 cm3 de pyridine. Après 16 heures de contact à température ambiante, on évapore la pyridine sous atmosphère inerte, reprend le résidu par 10 cm3 d'éther, triture, essore 135 mg de produit. On dissout ce dernier dans 1 cm3 de tétrahydrofuranne et ajoute goutte à goutte 10 cm3 d'éther sous agitation.
On essore le précipité formé, le sèche et obtient 106,5 mg de produit attendu.

Stade B : Iodure de /7-/2-(2-amino thiazol-4-yl) 2-méthoxy imino acétamido/ 2-carboxy 8-oxo 4-thia 1-azabicyclo /4.2.0/ oct-2-èn 3-yl/ thio méthyl pyridinium syn cis, racémique.

On opère comme au stade B de l'exemple 79 à partir de 90 mg de produit obtenu ci-dessus et obtient 48,5 mg de produit attendu.

Spectre RMN (DMSO)

6,85 ppm H en 5 du thiazole
6,01 ppm H du thio méthyle
8,94 - 8,97 ppm H en 2 et 6 du pyridyle
8,16 ppm H en 3 et 5 du pyridyle
8,7 ppm H en 4 du pyridyle

Exemple 81 : Acide 7-/2-(2-amino thiazol-4-yl) 2-méthoxy imino acétamido/ 3-//(1-méthyl 1H-tétrazol-5-yl) amino carbonyl méthyl/ thio/ 8-oxo 4-thia 1-azabicyclo /4.2.0/ oct-2-èn 2-carboxylique syn cis, racémique.

On opère comme à l'exemple 76 à partir de chloroacétyl amino 1-méthyl 1H-tétrazol-5-yle et obtient le produit attendu.

Spectre RMN ($CDCl_3$)

6,82 ppm $H_5$ du thiazole
3,86 et 3,88 ppm H du 1-méthyl tétrazole.

Exemple 82 : Acide 7-/2-(2-amino thiazol-4-yl) 2-méthoxyimino acétamido/ 3-//(2-méthyl 2H-tétrazol-5-yl) aminocarbonyl méthyl/ thio/ 8-oxo 4-thia 1-azabicyclo /4.2.0/ oct-2-èn-2-carboxylique syn, cis, racémique.

En opérant comme à l'exemple 74 à partir de chloroacétylamino 2-méthyl 2H-tétrazol-5-yle, on obtient le produit attendu.

Spectre RMN (DMSO)

6,84 ppm : $H_5$ du thiazole
4,3 ppm : H du 2-méthyl tétrazole.

Exemple 83 : Acide 7-/2-(2-amino thiazol-4-yl) 2-méthoxyimino acétamido/ 3-/(2-cyano (Z) éthén-1-yl) thio/ 8-oxo 4-thia 1-azabicyclo /4.2.0/ oct-2-èn-2-carboxylique syn, cis, racémique.

En opérant comme à l'exemple 76 à partir de cyanoéthynyle, on obtient le produit attendu.

Spectre RMN (DMSO)

6,83 ppm : $H_5$ du thiazole
7,6 - 7,72 ppm : H en 1 de l'éthényle
5,88 - 6 ppm : H en 2 de l'éthényle.

Exemple 84 : Trifluoroacétate de l'acide 7-/2-(2-amino thiazol-4-yl) 2-méthoxyimino acétamido/ 3-/(7-nitro 4-benzofurazanyl) thio/ 8-oxo 4-thia 1-azabicyclo /4.2.0/ oct-2-èn-2-carboxylique syn, cis, racémique.

En opérant comme à l'exemple 72 à partir de 4-chloro 7-nitrofurazane, on obtient le produit attendu.

Spectre RMN (DMSO)

6,9 ppm : $H_5$ du thiazole
7,47 - 7,55 ppm : H en 5 du benzofurazanyle
8,63 - 8,72 ppm : H en 6 du benzofurazanyle.

Exemple 85 : Acide 7-/2-(2-amino thiazol-4-yl) 2-méthoxyimino acétamido/ 8-oxo 4-thia 1-azabicyclo /4.2.0/ oct-2-èn-2,3-dicarboxylique syn, cis, racémique.

Stade A : 7-/2-(2-tritylamino thiazol-4-yl) 2-méthoxyimino acétamido/ 3-hydroxyiminométhyl 8-oxo 4-thia 1-azabicyclo /4.2.0/ oct-2-èn-2-carboxylate de (1,1-diméthyl) éthyle.

On dissout sous atmosphère inerte, 142 mg de 7-/2-(2-tritylamino thiazol-4-yl) 2-méthoxyimino acétamido/ 3-formyl 8-oxo 4-thia 1-azabicyclo /4.2.0/ oct-2-èn-2-carboxylate de (1,1-diméthyl) éthyle (préparé au stade A, exemple 52), 42 mg de chlorhydrate d'hydroxylamine dans 0,4 cm3 de pyridine. On agite 50 minutes, dilue à l'éther et à l'acétate d'éthyle, lave avec 6 cm3 d'acide chlorhydrique N. Après extraction de la phase aqueuse par l'acétate d'éthyle, on sèche les phases organiques et concentre à sec. On chromatographie sous pression sur silice en éluant avec un mélange acétate d'éthyle-chlorure de méthylène (25-75). On isole 91 mg de produit attendu.

Stade B : 7-/2-(2-tritylamino thiazol-4-yl) 2-méthoxyimino acétamido/ 3-cyano 8-oxo 4-thia 1-azabicyclo /4.2.0/ oct-2-èn-2-carboxylate de (1 ,1-diméthyl) éthyle.

On dissout sous atmosphère inerte, 151 mg du produit obtenu ci-dessus, 80 mg de chlorure de tosyle dans 6 cm3 de chlorure de méthylène et ajoute goutte à goutte 120 ul de triéthylamine. On agite 25 minutes, lave à l'eau légèrement acidulée, décante et réextrait au chlorure de méthylène. On sèche les phases organiques, et distille le solvant. On chromatographie sous pression sur silice le résidu et élue par un mélange chlorure de méthylène-acétone (93-7). On obtient 97 mg de produit attendu.

Stade C : Acide 7-/2-(2-amino thiazol-4-yl) 2-méthoxyimino acétamido/ 8-oxo 4-thia 1-azabicyclo /4.2.0/ oct-2-èn- 2,3-dicarboxylique syn, cis, racémique.

On chauffe à 65 °C extérieur pendant 1 heure 30 minutes, une suspension comprenant 83 mg de produit obtenu ci-dessus et 0,9 cm3 d'acide formique à 66%, distille le solvant, reprend le résidu à l'éthanol et obtient 27 mg de produit attendu.

Spectre RMN (DMSO)

6,85 ppm : $H_5$ du thiazole
5,59 - 5,73 ppm : $H_7$

Exemple 86 : Trifluoroacétate de l'acide 7-/2-(2-amino thiazol-4-yl) 2-méthoxyimino acétamido/ 3-/-(aminocarbonyl) hydrazonométhyl/ 8-oxo 4-thia 1-azabicyclo /4.2.0/ oct-2-èn-2-carboxylique syn, cis, racémique.

Stade A : 7-/2-(2-tritylamino thiazol-4-yl) 2-méthoxyimino acétamido/ 3-/(aminocarbonyl) hydrazonométhyl/ 8-oxo 4-thia 1-azabicyclo /4.2.0/ oct-2-èn-2-carboxylique de 1,1-diméthyléthyle.

On mélange 142 mg de 7-/2-(2-tritylamino thiazol-4-yl) 2-méthoxyimino acétamido/ 3-formyl 8-oxo 4-thia 1-azabicyclo /4.2.0/ oct-2-èn-2-carboxylate de 1,1-diméthyléthyle, 60 mg de chlorhydrate de semi carbazide et 0,8 cm3 de diméthylformamide. Après 1 heure 30 minutes, on ajoute 10 cm3 d'eau et extrait à l'acétate d'éthyle. On sèche les phases organiques, concentre à sec et dissout dans l'éthanol. On laisse cristalliser pendant 45 minutes, essore et obtient 113 mg de produit attendu.

Stade B : Trifluoroacétate de l'acide 7-/2-(2-amino thiazol-4-yl) 2-méthoxyimino acétamido/ 3-/-(aminocarbonyl) hydrazonométhyl/ 8-oxo 4-thia 1-azabicyclo /4.2.0/ oct-2-èn-2-carboxylique syn, cis, racémique.

On opère comme à l'exemple 84 à partir du produit obtenu ci-dessus et obtient le produit attendu.

Spectre RMN (DMSO)

6,9 ppm : $H_5$ du thiazole
5,62 à 5,77 ppm : $H_7$.

Exemple 87 : Acide 7-/2-(2-amino thiazol-4-yl) 2-méthoxyimino acétamido/ 3-/(4-nitrophényl) hydrazonométhyl/ 8-oxo 4-thia 1-azabicyclo /4.2.0/ oct-2-èn-2-carboxylique syn, cis, racémique.

Stade A : 7-/2-(2-tritylamino thiazol-4-yl) 2-méthoxyimino acétamido/ 3-/(4-nitrophényl) hydrazonométhyl/ 8-oxo 4-thia 1-azabicyclo /4.2.0/ oct-2-èn-2-carboxylate de (1,1-diméthyl) éthyle.

On opère comme au stade A de l'exemple 86 à partir de paranitro phényl hydrazine et obtient le produit attendu qui a cristallisé dans l'acétate d'éthyle.

Stade B : Acide 7-/2-(2-amino thiazol-4-yl) 2-méthoxyimino acétamido/ 3-/(4-nitrophényl) hydrazonométhyl/ 8-oxo 4-thia 1-azabicyclo /4.2.0/ oct-2-èn-2-carboxylique syn, cis, racémique.

On opère comme à l'exemple 85, stade C, à partir du produit obtenu ci-dessus et distille le solvant par entraînement azéotropique avec de l'éthanol. On empâte le résidu au méthanol, filtre, sèche et obtient le produit attendu.

Spectre RMN (DMSO)

6,85 : $H_5$ du thiazole
8,13 - 8,23 ppm : H en ortho du $NO_2$ aromatique
7,07 - 7,17 ppm : H en ortho du NH aromatique.

Exemple 88 : Trifluoroacétate de l'acide 7-/2-(2-amino thiazol-4-yl) 2-méthoxyimino acétamido/ 3-/-(éthoxycarbonyl) éthèn-1-yl/ 8-oxo 4-thia 1-azabicyclo /4.2.0/ oct-2-èn-2-carboxylique syn, cis, racémique.

Stade A : 7-/2-(2-tritylamino thiazol-4-yl) 2-méthoxyimino acétamido/ 3-/(éthoxycarbonyl) éthèn-1-yl/ 8-oxo 4-thia 1-azabicyclo /4.2.0/ oct-2-èn-2-carboxylate de (1,1-diméthyl) éthyle.

On dissout 142 mg de 7-/2-(2-tritylamino thiazol-4-yl) 2-méthoxyimino acétamido/ 3-formyl 8-oxo 4-thia 1-azabicyclo /4.2.0/ oct-2-èn-2-carboxylate de (1,1-diméthyl) éthyle et 139 mg de carbéthoxy méthylène triphényl phosphorane dans 4 cm3 de chlorure de méthylène. On agite pendant 48 heures, chasse le solvant et chromatographie le résidu sur silice en éluant par un mélange chlorure de méthylène-acétate d'éthyle (9-1). On isole 86 mg de produit attendu.

Stade B : Trifluoroacétate de l'acide 7-/2-(2-amino thiazol-4-yl) 2-méthoxyimino acétamido/ 3-/-(éthoxycarbonyl) éthèn-1-yl/ 8-oxo 4-thia 1-azabicyclo /4.2.0/ oct-2-èn-2-carboxylique syn, cis, racémique.

On opère comme au stade B de l'exemple 84 à partir du produit obtenu ci-dessus et obtient le produit attendu.

Spectre RMN (DMSO)

6,88 ppm : $H_5$ du thiazole
6,07 - 6,24 ppm : H éthylénique en $\alpha$ du $CO_2^-$
7,74 - 7,92 ppm : H éthylénique en $\beta$ du $CO_2$
1,67 - 1,24 - 1,32 ppm
4,04 - 4,12 - 4,21 - 4,28 ppm } H de l'éthoxy.

Exemple 89 : Acide 7-/2-(2-amino thiazol-4-yl) 2-méthoxyimino acétamido/ 3-(3-oxo 1-butèn-1-yl) 8-oxo 4-thia 1-azabicyclo /4.2.0/ oct-2-èn-2-carboxylique syn, cis, racémique.

On opère comme à l'exemple 88 à partir de triphényl phosphoranylidène 2-propanone, obtient le trifluoroacétate que l'on dissout dans de l'eau bicarbonatée en excès, filtre, acidifie le filtrat par 2 gouttes d'acide formique. Après 15 minutes, on filtre les cristaux formés, rince à l'eau puis à l'éther et obtient le produit attendu.

Spectre RMN (DMSO)

6,83 ppm : $H_5$ du thiazole
6,32 - 6,48 ppm : H éthylénique en $\alpha$ du C=0
7,63 - 7,80 ppm : H éthylénique en $\beta$ du C=0.

Exemple 90 : Trifluoroacétate d'acide 7-/2-(2-amino thiazol-4-yl) 2-méthoxyimino acétamido/ 3-(3-cyano éthèn-1-yl) 8-oxo 4-thia 1-azabicyclo /4.2.0/ oct-2-èn-2-carboxylique syn, cis, racémique.

On opère comme à l'exemple 88 à partir de cyanométhylène triphényl phosphorane et obtient le produit attendu.

Spectre RMN (DMSO)

6,88 ppm : $H_5$ du thiazole
5,81 - 5,94 ppm : H éthylénique en du CN
7,24 - 7,38 ppm : H éthylénique en du CN.

Exemple 91 : Acide 7-/2-(2-amino thiazol-4-yl) 2-méthoxyimino acétamido/ 3-/(4-nitrophényl) méthylthio/ 8-oxo 4-thia 1-azabicyclo /4.2.0/ oct-2-èn-2-carboxylique syn, cis, racémique.

Stade A : Disulfure de paranitrobenzyle.

On dissout 1,0 g de paranitrobenzylmercaptan dans 30 cm3 de chlorure de méthylène, ajoute 0,83 cm3 de triéthylamine, 750 mg d'iode en maintenant la température à 25°C et agite pendant 10 minutes. On lave avec de l'acide chlorhydrique N, sèche la phase organique et évapore le solvant. On ajoute 20 cm3 d'éther au résidu, agite 10 minutes, essore, sèche et obtient 839 mg de produit attendu. F = 126°C.

Stade B : 7-/2-(2-tritylamino thiazol-4-yl) 2-méthoxyimino acétamido/ 2-hydroxy 3-/(4-nitrophényl) méthylthio/ 8-oxo 4-thia 1-azablcyclo /4.2.0/ octane 2-carboxylate de 1,1-diméthyl éthyle.

On met en suspension 925 mg de disulfure obtenu ci-dessus dans 10 cm3 de chlorure de méthylène et ajoute, goutte à goutte, sous forte agitation 1,75 mmole de chlore dans 2,5 cm3 de chlorure de méthylène.

Après 10 minutes d'agitation, on verse goutte à goutte le mélange réactionnel dans une solution de 1,12 g de diazopyruvate de terbutyle dissous dans 5 cm3 de chlorure de méthylène. Cette solution est ajoutée parallèlement à une solution de 0,77 cm3 de triéthylamine diluée dans 4 cm3 de chlorure de méthylène dans une suspension de 1,23 g de 4-mercaptométhyl 3-/2-(2-tritylamino thiazol-4-yl) 2-méthoxyimino acétamido/ 2-oxo 1-azétidine dans 10 cm3 de chlorure de méthylène. On agite 30 minutes, lave avec de l'acide chlorhydrique 0,1 N puis avec une solution de bicarbonate de sodium, sèche et concentre à sec. On chromatographie le résidu sur silice, élue avec un mélange chlorure de méthylène-acétone (9-1) et isole 960 mg de produit attendu (mélange de diastéréoisomères).

Stade C : 7-/2-(2-tritylamino thiazol-4-yl) 2-méthoxyimino acétamido/ 2-hydroxy 3-/(4-nitrophényl) méthylthio/ 8-oxo 4-thia 1-azabicyclo /4.2.0/ oct-2-èn-2-carboxylate de 1,1-diméthyl éthyle.

On opère comme à l'exemple 1, stade D, à partir du produit ci-dessus et obtient le produit attendu.

Stade D : Acide 7-/2-(2-amino thiazol-4-yl) 2-méthoxyimino acétamido/ 3-/(4-nitrophényl) méthylthio/ 8-oxo 4-thia 1-azabicyclo /4.2.0/ oct-2-èn-2-carboxyliquesyn, cis racémique.

On chauffe à 50 °C, 200 mg de produit obtenu ci-dessus dans 4 cm3 d'acide formique à 66% pendant 3 heures. On refroidit, filtre, lave l'insoluble avec de l'acide formique à 66%, évapore le filtrat sous pression réduite à 40 °C. On reprend le résidu avec 10 cm3 d'acétonitrile et 5 cm3 de méthanol. Après évaporation des solvants, on ajoute 5 cm3 de chlorure de méthylène et 1 cm3 de méthanol et agite 16 heures. On filtre, sèche et obtient 103 mg de produit attendu. F = 185-190 °C (décomposition).

Spectre RMN (DMSO)

6,82 ppm : $H_5$ du thiazole
8,15 - 8,24 ppm : H en ortho du $NO_2$
7,54 - 7,63 ppm : H en méta du $NO_2$.

Exemple 92 / Acide 7-/2-(2-amino thiazol-4-yl) 2-méthoxy imino acétamido/ 3-/(5-méthyl 1,3,4-thiadiazol-2-yl) thio/ 8-oxo 4-thia 1-azabicyclo /4.2.0/ oct-2-èn 2-carboxylique syn cis, racémique.

Stade A : 3-chloro 2-hydroxy 3-(5-méthyl 1,3,4-thiadiazol 2-yl) thio propénoate de 1,1-diméthyl éthyle et 3-chloro 3-(5-méthyl 1,3,4-thiadiazol-2-yl) thio 2-oxo propanoate de 1,1-diméthyl éthyle.

On refroidit à 10 °C, 1,34 g de N-chlorosuccinimide dans 25 cm3 de benzène et ajoute 1,32 g de 5-mèthyl 4-mercapto 1,3,4-thiadiazole en 10 minutes. On agite à 7 °C pendant 15 minutes puis ajoute en 10 minutes 1,72 g de diazopiruvate de terbutyle dissous dans 5 cm3 de chlorure de méthylène à 7/8 °C. On laisse remonter la température pendant une heure, filtre l'insoluble et évapore le filtrat.

On reprend le résidu dans l'éther, filtre, évapore le filtrat et chromatographie le résidu sur silice en éluant par un mélange chlorure de méthylène - acétone (8,7-1,3). On obtient 820 mg de produit attendu sous forme d'un mélange des 2 tautomères.

Stade B : 7-/2-(2-tritylamino thiazol-4-yl) 2-méthoxy imino acétamido/ 2-hydroxy 3-/(5-méthyl 1,3,4-thiadiazol-2-yl) thio/ 8-oxo 4-thia 1-azabicyclo /4.2.0/ octan 2-carboxylate de 1,1-diméthyléthyle syn cis, racémique.

On agite à température ambiante pendant deux heures 493 mg de produit obtenu ci-dessus, 685 mg de 4-mercaptométhyl 3-/2-(2-tritylamino thiazol-4-yl) 2-méthoxy imino acétamido/ 2-oxo 1-azétidine syn cis, racémique et 0,26 cm3 de triéthylamine dans 10 cm3 de chlorure de méthylène. On filtre, lave la solution avec 4 cm3 d'acide chlorhydrique N puis 4 cm3 d'une solution de bicarbonate de sodium, sèche et concentre à sec sous pression réduite.

On chromatographie le résidu sur silice en éluant par un mélange chlorure de méthylène - acétone (8,5-1,5) et obtient 664 mg de produit attendu sous forme de mélange de stéréoisomères.

Stade C : 7-/2-(2-tritylamino thiazol-4-yl) 2-méthoxy imino acétamido/ 3-/(5-méthyl 1,3,4-thiadiazol-2-yl) thio/ 8-oxo 4-thia 1-azabicyclo /4.2.0/ oct-2-èn 2-carboxylate de 1,1-diméthyl éthyle.

On opère comme à l'exemple 1 stade D à partir de 895 mg de produit obtenu au stade C ci-dessus et obtient 234 mg de produit attendu.

Stade D : Acide 7-/2-(2-amino thiazol-4-yl) 2-méthoxy imino acétamido/ 3-/(5-méthyl 1,3,4-thiadiazol-2-yl) thio/ 8-oxo 4-thia 1-azabicyclo /4.2.0/ oct-2-èn 2-carboxylique syn cis, racémique.

On dissout 192 mg de produit obtenu au stade C dans 4 cm3 d'acide formique aqueux à 66%, agite la solution pendant 3 heures 50 minutes à 50 ° C. Après refroidissement, on filtre et concentre à sec le filtrat sous pression réduite, sèche le résidu à 50 ° C pendant 30 minutes puis ajoute 5 cm3 de méthanol et agite pendant 16 heures à température ambiante. On essore, rince avec du méthanol, sèche sous pression réduite à 50 ° C et obtient 94 mg de produit attendu.

Spectre RMN (DMSO)

6,81 ppm $H_5$ du thiazole
2,72 ppm H du 5-méthyl thiadiazole

Exemple 93 : Trifluoroacétate de trifluorométhane sulfonate de 1-//7-/2-(2-amino thiazol-4-yl) 2-méthoxy imino acétamido/ 2-carboxy 8-oxo 4-thia 1-azabicyclo /4.2.0/ oct-2-èn 3-yl/ méthyl (2-méthylthio)/ pyridinium syn cis, racémique.

On opère comme à l'exemple 38 à partir de 2-méthyl thio pyridine et obtient le produit attendu.

Spectre RMN (DMSO)

6,83 ppm $H_5$ du thiazole
8,0 - 8,14 ppm $H_3$ du pyridinium
8,97 - 9,06 ppm H6 du pyridinium

7,8 à 7,96 ppm }
8,4 à 8,56 ppm } $H_4$ et $H_5$ du pyridinium.

Exemple 94 : Trifluoroacétate de trifluorométhane sulfonate de 1-//7-/2-(2-amino thiazol-4-yl) 2-méthoxy imino acétamido/ 2-carboxy 8-oxo 4-thia 1-azabicyclo /4.2.0/ oct-2-èn 3-yl/ méthyl 4-(méthyl) pyridinium syn cis racémique.

On opère comme à l'exemple 38 à partir de 4-picoline et obtient le produit attendu.

Spectre RMN (DMSO)

6,83 ppm $H_5$ du thiazole
8,93 - 9 ppm $H_2$ et $H_6$ du pyridinium
8,01 - 8,08 ppm $H_3$ et $H_5$ du pyridinium.

EXEMPLE 95

On a réalisé des préparations pour injections de formule :

| | |
|---|---|
| A)Acide 7-/2-(2-aminothiazol-4-yl) 2-méthoxyimino acétamido/ 8-oxo 3-/(1-méthyl (1H) tétrazol-5-yl) thio/ 4-thia 1-azabicyclo /4-2-0/ oct-2-ène 2-carboxylique isomère syn | 500 cm3 |
| - excipient aqueux stérileq.s.p | 5 cm3 |
| B) Acide 7-/2-(2-aminothiazol-4-yl) 2-méthoxyimino acétamido/ 8-oxo 3-(4-nitrophénylthio) 4-thia 1-azabicyclo /4-2-0/ oct-2-ène 2-carboxylique isomère syn | 500 mg |
| - excipient aqueux stérile q.s.p | 5 cm3 |
| C) Trifluorométhane sulfonate de 1//7-//2-amino 4-thiazolyl) (méthoxyimino) acétyl/ amino/ 2-carboxy 8-oxo 4-thia 1-azabicyclo /4-2-0/ oct-2-ène 3-yl/ méthyl/ pyridinium isomère syn | 500 mg |
| - excipient aqueux stérile q.s.p | 5 cm3 |

**ETUDE PHAMACOLOGIOUE DES PRODUITS DE L'INVENTION**

Activité in vitro, méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre ou quarant-huit heures à l'étuve à 37 ° C, l'inhibition de la croissance est appréciée par transillumination ce qui permet de déterminer les concentrations minimales inhibitrices (C.M.I.) exprimées en µg/cm3.

Les résultats suivants sont obtenus :

| SOUCHES | Prod.ex. 19 | | Prod.ex. 23 | | Prod.ex. 28 | | Prod.ex. 29 | | Prod.ex.33 | | Prod.ex. 34 | | Prod.ex. 44 | |
|---|---|---|---|---|---|---|---|---|---|---|---|---|---|---|
| | 24 H | 48 H | 24 H | 48 H | 24 H | 48 H | 24 H | 48 H | 24 H | 48 H | 24 H | 48 H | 24 H | 48 H |
| Staphylococcus aureus SG 511 | 1,2 | 2,5 | 1,2 | 1,2 | 1,2 | 2,5 | 0,3 | 0,6 | 0,6 | 1,2 | 5 | 5 | 1,2 | 2,5 |
| Staphylococcus aureus 285 | 0,6 | 5 | 1,2 | 1,2 | 2,5 | 5 | 1,2 | 5 | 2,5 | 2,5 | 10 | 20 | 2,5 | 5 |
| Staphylococcus aureus 54146 | 1,2 | 2,5 | 5 | 5 | 5 | 10 | 2,5 | 5 | 2,5 | 5 | 20 | 40 | 5 | 5 |
| Streptococcus pyogenes A 561 | ≤0,04 | ≤0,04 | ≤0,04 | ≤0,04 | ≤0,04 | ≤0,04 | ≤0,04 | ≤0,04 | ≤0,04 | ≤0,04 | ≤0,04 | ≤0,04 | ≤0,04 | ≤0,04 |
| Streptococcus pyogenes 77 A | ≤0,04 | ≤0,04 | ≤0,04 | ≤0,04 | ≤0,04 | ≤0,04 | ≤0,04 | ≤0,04 | ≤0,04 | ≤0,04 | ≤0,04 | ≤0,04 | ≤0,04 | ≤0,04 |
| Escherichia Coli 1894 | - | - | 0,3 | 0,3 | ≤0,04 | ≤0,04 | ≤0,04 | ≤0,04 | ≤0,04 | ≤0,04 | ≤0,04 | ≤0,04 | ≤0,04 | ≤0,04 |
| " " 078 | - | - | 0,6 | 0,6 | 0,08 | 0,08 | 0,15 | 0,15 | ≤0,04 | ≤0,04 | 0,08 | 0,08 | 0,08 | 0,08 |
| " " TEM | - | - | 2,5 | 2,5 | 0,3 | 0,3 | 0,6 | 0,6 | 0,08 | 0,08 | 0,15 | 0,15 | 0,15 | 0,15 |
| " " 1507 E | - | - | 0,6 | 0,6 | 0,08 | 0,08 | 0,15 | 0,15 | ≤0,04 | ≤0,04 | 0,15 | 0,15 | 0,08 | 0,08 |
| " " DCO | - | - | 2,5 | 2,5 | 0,15 | 0,15 | 0,6 | 0,6 | 0,08 | 0,15 | 0,15 | 0,15 | 0,3 | 0,3 |
| " " DC2 | - | - | 0,3 | 0,3 | 0,08 | 0,08 | 0,08 | 0,08 | ≤0,04 | ≤0,04 | 0,15 | 0,15 | 0,08 | 0,08 |
| Salmonella typhimurium MZ 11 | - | - | 1,2 | 1,2 | 0,08 | 0,08 | 0,15 | 0,3 | 0,08 | 0,08 | 0,08 | 0,15 | 0,15 | 0,15 |
| Klebsiella pneumoniae 52145 | - | - | 2,5 | 2,5 | 0,15 | 0,15 | 0,6 | 0,6 | 0,3 | 0,3 | 0,3 | 0,3 | 0,6 | 0,6 |
| " aerogenes 1522 E | - | - | 1,2 | 1,2 | 0,08 | 0,08 | 0,3 | 0,3 | 0,08 | 0,15 | 0,15 | 0,15 | 0,08 | 0,08 |
| Enterobacter cloacae 1321 E | - | - | 0,6 | 1,2 | 0,08 | 0,08 | 0,15 | 0,3 | ≤0,04 | ≤0,04 | 0,08 | 0,08 | 0,08 | 0,08 |
| Proteus mirabilis A 235 | - | - | 0,6 | 0,6 | 0,15 | 0,15 | 0,6 | 0,6 | 0,15 | 0,15 | 0,3 | 0,3 | 0,3 | 0,3 |
| Proteus vulgaris A 232 | - | - | 2,5 | 5 | 0,15 | 0,3 | 0,3 | 0,6 | 0,6 | 0,6 | 0,15 | 0,15 | 0,3 | 0,3 |

| SOUCHES | Prod.ex. 46 | | Prod.ex. 50 | | Prod.ex. 53 | | Prod.ex. 62 | | Prod.ex. 93 | |
|---|---|---|---|---|---|---|---|---|---|---|
| | 24 H | 48 H | 24 H | 48 H | 24 H | 48 H | 24 H | 48 H | 24 H | 48 H |
| Staphylococcus aureus SG 511 | 1,2 | 1,2 | 1,2 | 1,2 | 0,3 | 1,2 | 2,5 | 2,5 | 1,2 | 2,5 |
| Staphylococcus aureus 285 | 1,2 | 5 | 1,2 | 2,5 | 1,2 | 1,2 | 5 | 5 | 2,5 | 5 |
| Staphylococcus aureus 54146 | 5 | 10 | 5 | 10 | 1,2 | 2,5 | 5 | 5 | 5 | 10 |
| Streptococcus pyogenes A 561 | ≤ 0,04 | ≤ 0,04 | ≤ 0,04 | ≤ 0,04 | ≤ 0,02 | ≤ 0,02 | ≤ 0,04 | ≤ 0,04 | ≤ 0,04 | ≤ 0,04 |
| Streptococcus pyogenes 77 A | ≤ 0,04 | ≤ 0,04 | ≤ 0,04 | ≤ 0,04 | ≤ 0,02 | ≤ 0,02 | ≤ 0,04 | ≤ 0,04 | ≤ 0,04 | ≤ 0,04 |
| Escherichia Coli 1894 | ≤ 0,04 | ≤ 0,04 | ≤ 0,04 | ≤ 0,04 | ≤ 0,02 | ≤ 0,02 | ≤ 0,04 | ≤ 0,04 | ≤ 0,04 | ≤ 0,04 |
| " " 078 | 0,15 | 0,15 | 0,15 | 0,15 | ≤ 0,02 | ≤ 0,02 | 0,08 | 0,08 | 0,08 | 0,08 |
| " " TEM | 0,15 | 0,3 | 0,6 | 1,2 | 0,08 | 0,08 | 0,3 | 0,3 | 0,3 | 0,3 |
| " " 1507 E | ≤ 0,04 | 0,08 | 0,08 | 0,08 | ≤ 0,02 | ≤ 0,02 | ≤ 0,04 | ≤ 0,04 | 0,08 | 0,08 |
| " " DCO | 0,3 | 0,3 | 0,3 | 0,3 | 0,08 | 0,08 | 0,3 | 0,3 | 0,3 | 0,3 |
| " " DC2 | ≤ 0,04 | 0,04 | 0,04 | 0,08 | ≤ 0,02 | ≤ 0,02 | 0,08 | 0,08 | 0,08 | 0,08 |
| Salmonella typhimurium MZ 11 | 0,08 | 0,15 | 0,15 | 0,15 | 0,08 | 0,08 | 0,6 | 0,6 | 0,3 | 0,3 |
| Klebsiella pneumoniae 52145 | 0,6 | 0,6 | 0,6 | 0,6 | 0,15 | 0,3 | 0,6 | 1,2 | 0,6 | 0,6 |
| " aerogenes 1522 E | 0,3 | 0,3 | 0,6 | 0,6 | 0,08 | 0,08 | 0,6 | 1,2 | 0,3 | 0,3 |
| Enterobacter cloacae 1321 E | 0,08 | 0,08 | 0,15 | 0,15 | 0,04 | 0,04 | 0,15 | 0,3 | 0,15 | 0,15 |
| Proteus mirabilis A 235 | 0,3 | 0,6 | 0,3 | 0,3 | 0,15 | 0,15 | 0,3 | 0,3 | 0,3 | 0,3 |
| Proteus vulgaris A 232 | 1,2 | 2,5 | 0,6 | 2,5 | 0,15 | 0,3 | 0,3 | 0,3 | 0,6 | 0,6 |

## Revendications

**1.** Les produits de formule générale (I') :

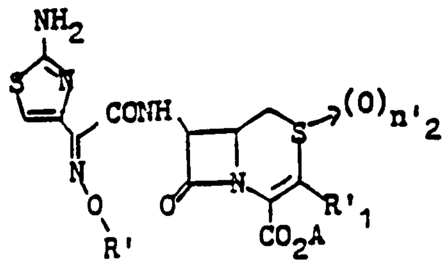

(I')

isomère syn

dans laquelle R' représente un atome d'hydrogène ou un radical alkyle ou alkényle ayant au plus 4 atomes de carbone,

- un radical carboxyméthyle ou 1-carboxyéthyle, ces radicaux étant éventuellement estérifiés ou salifiés,
- un radical phényle, $R'_1$ représente :

  a) un radical $Z'-R'_2$ dans lequel $R'_2$ représente un radical alkyle ou alkényle ayant au plus 4 atomes de carbone, éventuellement interrompu par un hétéroatome et éventuellement substitué par un radical halogène, amino, cyano, carboxy libre estérifié ou salifié, par un radical carbamoyle, Z' représente un atome de soufre ou d'oxygène ;

  soit $Z'-R'_2$ est choisi parmi les radicaux

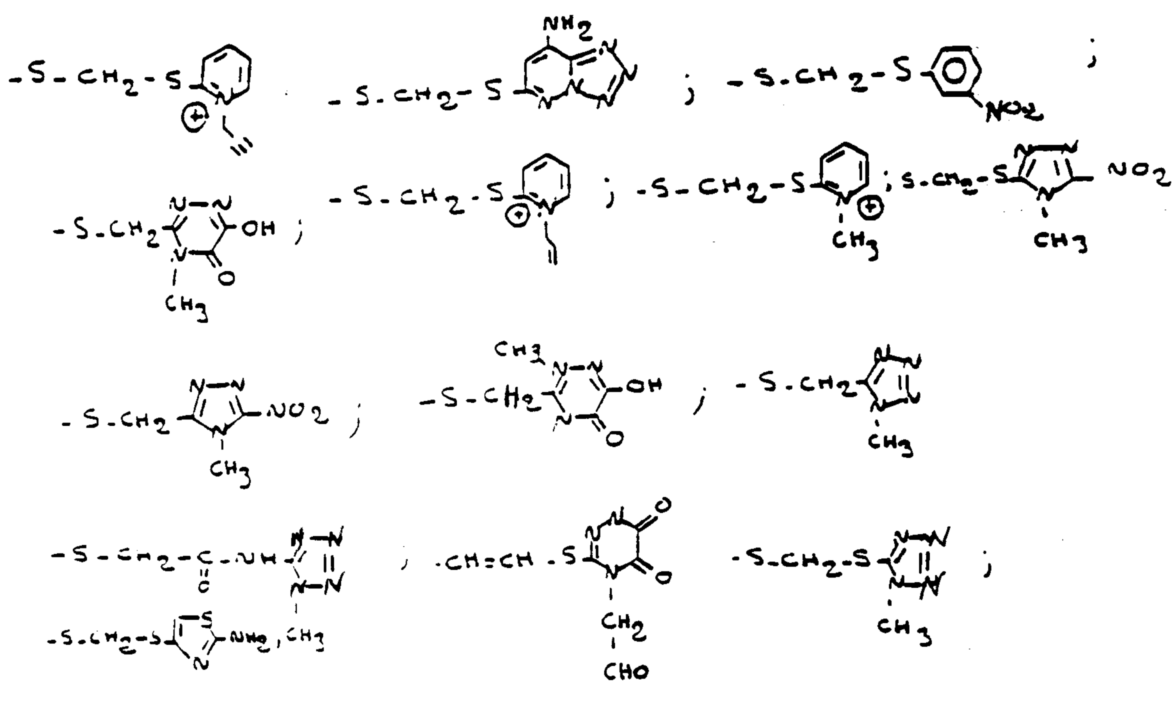

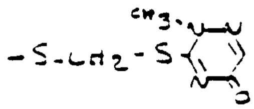 , 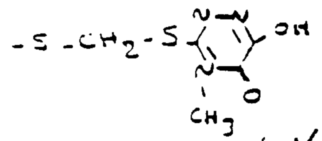 ; 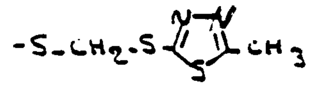

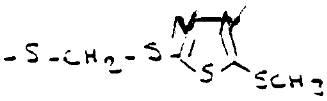 ; 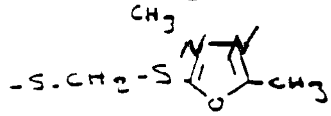 ; 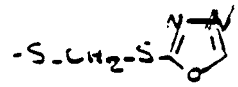

$-OCH_3$ ; 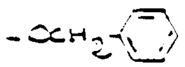 ;

b) soit un radical Z'$_a$-R'$_3$ dans lequel R'$_3$ représente un radical phényle et Z'a représente un radical méthylène, -CH$_2$-S- ou un atome de soufre, d'oxygène, de sélénium ou une simple liaison ;
soit Z'$_a$ -R'$_a$ est choisi parmi les radicaux

; 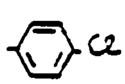 ; 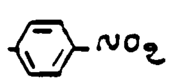 ; 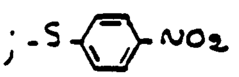 ; 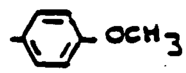 ;

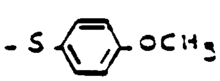 ; ; 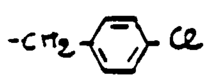 ; 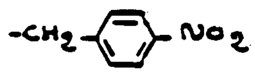 ;

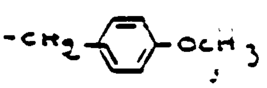 ; ; 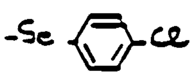 ; 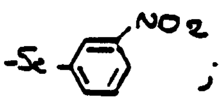 ; 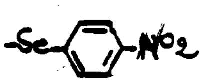 ;

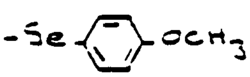 ; 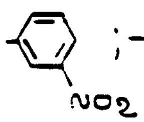 ; 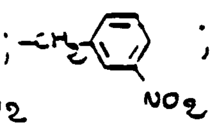 ; 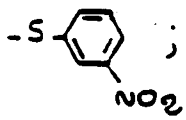 ; 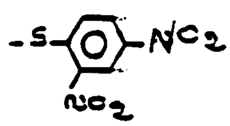 ;

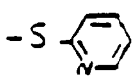 ; 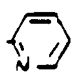 ; 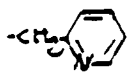 ; 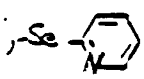 ; ; 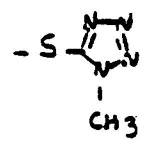 ;

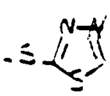 ; 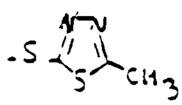 ; 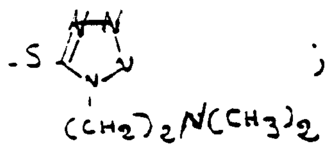 ; 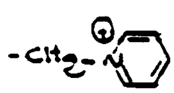 ; 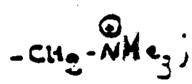 ;

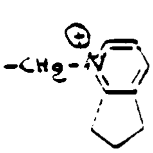 ; 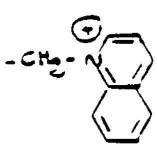 ; 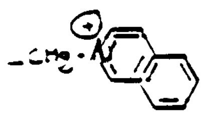 ;

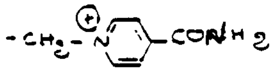 ; 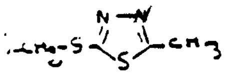 ; 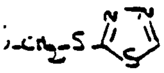 ; 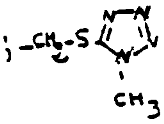

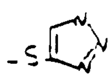 ; 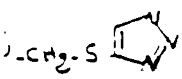 ; 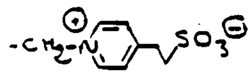 ; 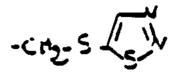 ;

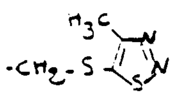 ; 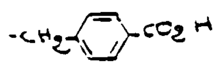 ; 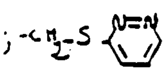 ; 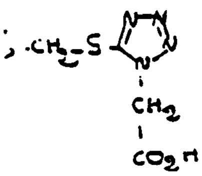 ;

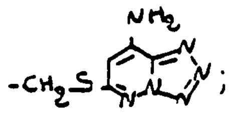 ; 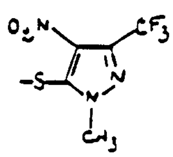 ; 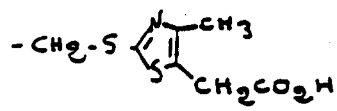 ;

;

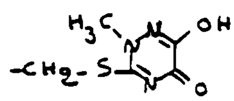 ; 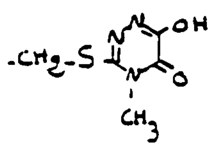 ; 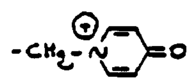 ; 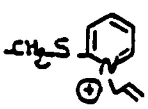 ;

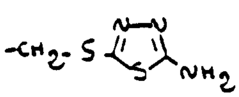 ; 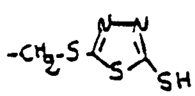 ; 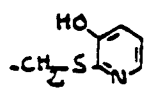 ; 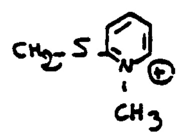 ;

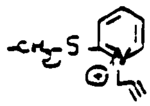 ; 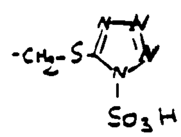 ; 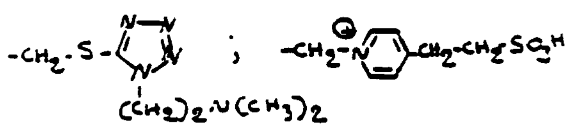 ;

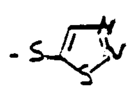 ; 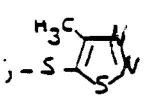 ; 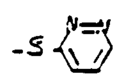 ; 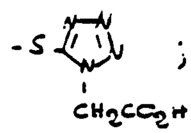 ; 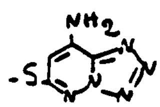 ;

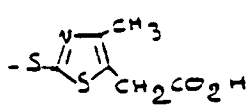 ; 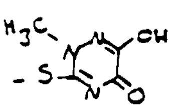 ; 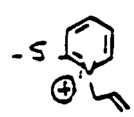 ; 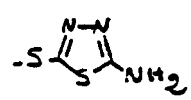 ;

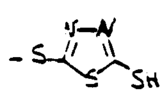 ; 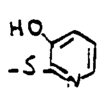 ; 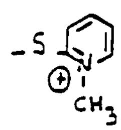 ; 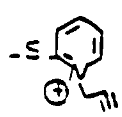 ; 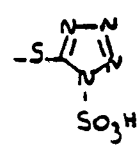 ;

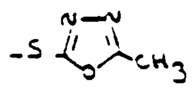 ; 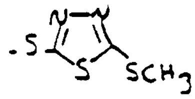 ; 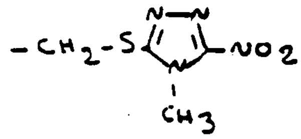 ; 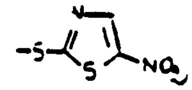

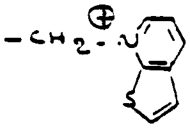 ; 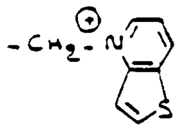 ; 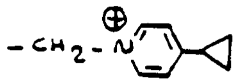 ; 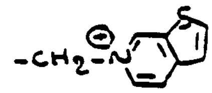

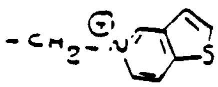 ; 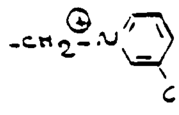 ; 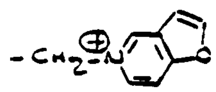 ; 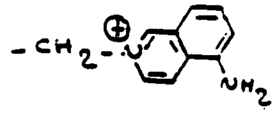

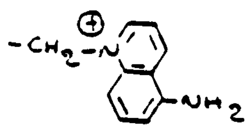 ;

n'$_2$ représente les nombres 0 ou 1 et A représente un atome d'hydrogène, un équivalent de métal alcalin, alcalino-terreux, de magnésium, d'ammonium ou d'une base organique aminée ou A représente un groupement ester ou $CO_2A$ représente un groupement $CO_2^-$ , ainsi que les sels, des produits de formule (I') avec les acides minéraux ou organiques.

**2.** Les produits de formule générale (I') telle que définie à la revendication 1, dans laquelle R' représente un atome d'hydrogène ou un radical méthyle ou allyle et R'$_1$ représente un radical choisi dans le groupe formé par les radicaux $-S-CH_2CO_2H$ ; $-S-CH_2CO_2Et$ ; $-S-CH_2-CO_2tBu$ ;

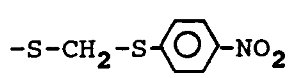 ;

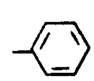 ; 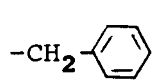 ; 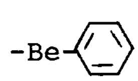 ; 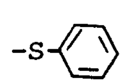 .

**3.** Les produits de formule générale (I') telle que définie à la revendication 1, sous forme racémique ou optiquement active, répondant aux formules suivantes :

- l'acide 7-/2-(2-aminothiazol-4-yl) 2-méthoxy imino acétamido/ 3-(3-nitrophénylthio) 8-oxo 4-thia-1-azabicyclo /4,2,0/ oct-2-ène 2-carboxylique isomère syn ;
- l'acide 7-/2-(2-aminothiazol-4-yl) 2-méthoxyimino acétamido/ 3-(4-nitrophényl) 8-oxo 4-thia 1-azabicyclo /4,2,0/ oct-2-ène 2-carboxylique isomère syn ;
- l'acide 7-/2-(2-aminothiazol-4-yl) 2-méthoxyimino acétamido/ 3-/(1-méthyl (1-H) tétrazol-5-yl) thio/ 8-oxo 4-thia 1-azabicyclo /4,2,0/ oct-2-ène 2-carboxylique isomère syn ;
- l'acide 7-/2-(2-aminothiazol-4-yl) 2-méthoxyimino acétamido/ 8-oxo 3-/(1-méthyl 1H-tétrazol-5-yl) thiométhyl/ 4-thia 1-azabicyclo /4,2,0/ oct-2-ène 2-carboxylique isomère syn ;
- le 1-//7-(2-amino thiazol-4-yl) (méthoxyimino) acétamido/ 2-carboxy 8-oxo 4-thia 1-azabicyclo /4,2,0/ oct 2-ène 3-yl/ méthyl/ pyridinium isomère syn ;
- le 1-//7-(2-aminothiazol-4-yl (méthoxyimino) acétamido/ 2-carboxy 8-oxo 4-thia 1-azabicyclo /4,2,0/ oct-2-ène 3-yl/ méthyl/ (6,7-dihydro) 5-H/1/ pyrindinium isomère syn ;
- le 6-/7-/2-(2-amino thiazol-4-yl) 2-méthoxyimino acétamido/ 2-carboxy 8-oxo 4-thia 1-azabicyclo /4.2.0/ oct-2-èn-3-yl) méthyl thiéno /2,3-c/ pyridinium isomère syn ;
- le 7-/2-(2-amino thiazol-4-yl) 2-méthoxyimino acétamido/ 8-oxo 2-carboxy 4-thia 1-azabicyclo /4.2.0/ oct-2-èn-3-yl/ méthyl/ triméthyl ammonium isomère syn ;

- le //7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ 4-cyclopropyl/ pyridinium isomère syn ;
- le 6-//7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ 7-méthyl thiéno /2,3-c/ pyridinium isomère syn ;
- le //7-/2-(2-amino thiazol-4-yl) 2-méthoxyimino acétamido/ 8-oxo 4-thia 2-carboxy 1-azabicyclo /4.2.0/ oct-2-èn-3-yl/ méthyl/ thiéno /2,3-b/ pyridinium isomère syn ;
- le /7-/2-(2-amino thiazol-4-yl) 2-(2-propényloxy) imino acétamido/ 2-carboxy 8-oxo 4-thia 1-azabicyclo /4.2.0/ oct-2-èn-3-yl/ méthyl pyridinium isomère syn ;
- le 1-//7-/2-(2-amino thiazol-4-yl) 2-méthoxyimino acétamido/ 2-carboxy 8-oxo 4-thia 1-azabicyclo /4.2.0/ oct-2-èn-3-yl) méthyl (2-méthylthio)/ pyridinium isomère syn.

**4.** Procédé de préparation des produits de formule générale (I') :

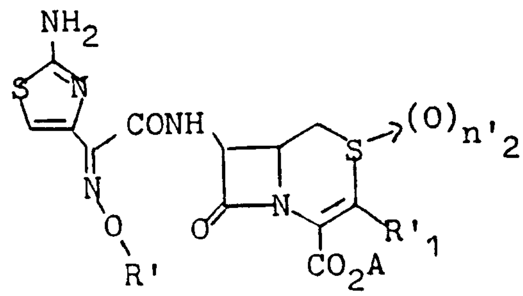

(I')

isomère syn
dans laquelle R' , $R'_1$, A et $n'_2$ ont la signification indiquée à la revendication 1 caractérisé en ce que l'on traite un produit de formule (II') :

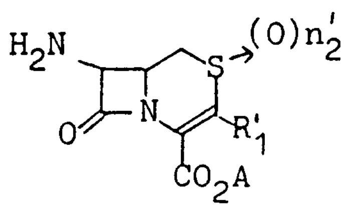

(II')

dans laquelle $R'_1$, A et $n'_2$ ont la signification précédente par un acide de formule (IV') :

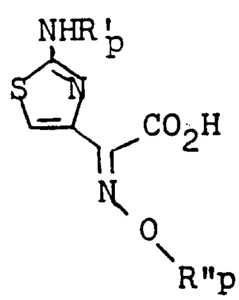

(IV')

ou un dérivé fonctionnel de cet acide, formule (IV') dans laquelle R'p représente un atome d'hydrogène ou un groupement protecteur du radical amino et R"p représente un groupement protecteur du radical hydroxyle ou R"p représente R' pour obtenir un produit de formule (V') :

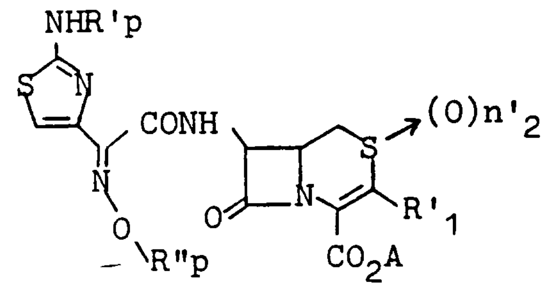

(V')

produit que l'on soumet, si nécessaire ou si désiré, à l'une ou plusieurs des réactions suivantes, dans un ordre quelconque :

a) coupure par hydrolyse, hydrogénolyse ou par action de la thiourée de tout ou partie du ou des groupements protecteurs ;

b) estérification ou salification par une base du ou des groupements carboxy ;

c) salification par un acide du ou des groupements amino ;

d) dédoublement de la molécule pour obtenir un produit optiquement actif ;

e) oxydation de l'atome de soufre en position 2 du cycle isocéphème.

**5.** Procédé de préparation des produits de formule (A') :

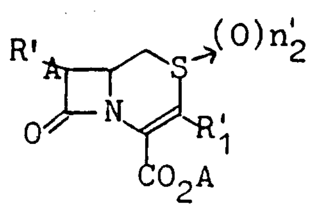

(A')

dans laquelle A, $R'_1$ et $n'_2$ ont la signification indiquée à la revendication 1 et $R'_A$ représente un radical amino libre ou protégé par un groupement protecteur mono ou divalent, ou $R'_A$ représente un radical :

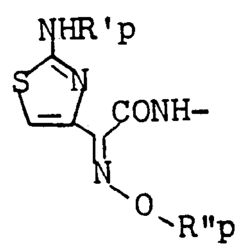

dans laquelle R'p et R''p ont la signification indiquée à la revendication 4,caractérisé en ce que l'on traite un produit de formule ($II'_A$) :

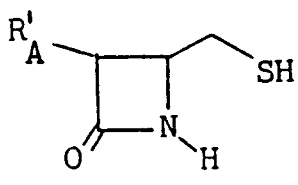

($II'_A$)

par un produit de formule ($III'_A$) :

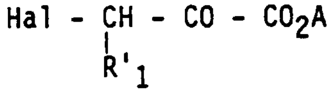 $(III'_A)$

dans laquelle Hal représente un atome d'halogène, $R'_1$ et A ont la signification précédente, pour obtenir un produit de formule (A'), produit que l'on soumet, si nécessaire ou si désiré, à l'une quelconque ou à plusieurs des réactions suivantes, dans un ordre quelconque :

a) coupure par hydrolyse, hydrogénolyse ou par action de la thiourée de tout ou partie du ou des groupements protecteurs ;
b) estérification ou salification par une base du ou des groupements carboxy ;
c) salification par un acide du ou des groupements amino ;
d) dédoublement de la molécule pour obtenir un produit optiquement actif ;
e) oxydation de l'atome de soufre en position 2 du cycle isocéphème.

**6.** Procédé selon la revendication 5 de préparation des produits de formule $(III'_{Ai})$ :

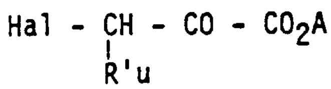 $(III'_{A1})$

dans laquelle Hal représente un atome d'halogène, R'u représente les valeurs de $R'_1$ liées par un atome de carbone, $R'_1$ et A ayant la signification indiquée à la revendication 1 caractérisé en ce que l'on traite un produit de formule R'uCHO par un produit de formule $(Hal_1)_2$ $CHCO_2A$ en présence d'une base forte pour obtenir un produit de formule :

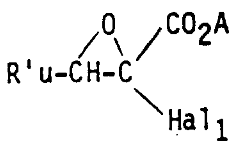 $(III'_{Ai})$

formules dans lesquelles $Hal_1$ représente un atome d'halogène, produit que l'on traite par un halogénure pour obtenir le produit de formule $(III'_{A1})$ attendu.

**7.** Variante du procédé tel que décrit à la revendication 5 de préparation des produits de formule (A') :

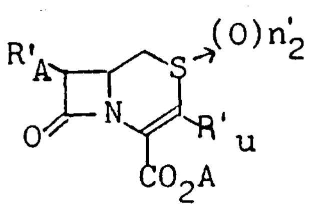 (A')

dans laquelle, $R'_A$, $n_2$'et A ont la signification indiquée à la revendication 5 et R'u a la signification indiquée à la revendication 6 , caractérisé en ce que l'on traite un produit de formule $(II'_A)$ tel que décrit à la revendication 5 par un produit de formule $(III'_{Ai})$ telle que décrit à la revendication 6.

**8.** Procédé selon la revendication 5 de préparation des produits de formule $(III''_{A1})$ :

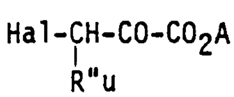 $(III''_{A1})$

dans laquelle Hal représente un atome d'halogène, R''u représente les valeurs de R'$_1$ liées par un hétéroatome, R'$_1$ et A ayant la signification indiquée à la revendication 1, caractérisé en ce que l'on fait réagir un produit de formule :

$N_2 = CH\text{-}COCO_2A$ (III''$_{A1}$)

avec un produit de formule R''u Hal pour obtenir le produit de formule (III''$_{A1}$) attendu.

**9.** Procédé selon la revendication 4 de préparation des produits de formule (IX) :

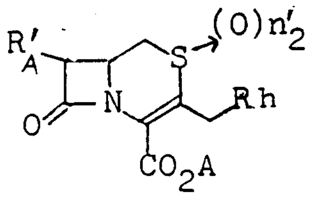

(IX)

dans laquelle R'$_A$, A et n'$_2$ ont la signification indiquée aux revendications 1 et 5 et Rh représente un ammonium quaternaire éventuellement substitué ou un radical S-R'h dans lequel R'h représente un radical aryle carbocyxlique ou hétérocyclique éventuellement substitué, tels que définis à la revendication 1 correspondant à des produits de formule (I') lorsque R'$_A$ représente le radical

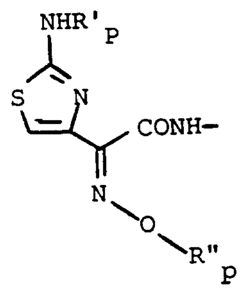

caractérisé en ce que, soit l'on fait agir un produit de formule R'h Sh sur un produit de formule (VI) :

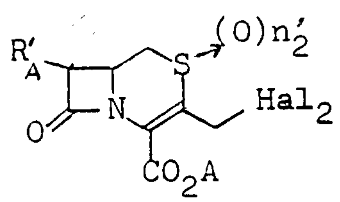

(VI)

formule dans laquelle R$_A$, R$_4$, A et n$_2$ ont la signification précédente et Hal$_2$ représente un atome d'halogène, soit l'on fait agir une amine ou une imine sur un produit de formule (VII) :

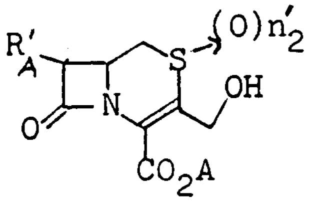

(VII)

pour obtenir un produit de formule (IX) que l'on soumet, si nécessaire ou si désiré, à l'une quelconque

ou à plusieurs des réactions suivantes, dans un ordre quelconque :

a) coupure par hydrolyse, hydrogénolyse ou par action de la thiourée de tout ou partie du ou des groupements protecteurs ;

b) estérification ou salification par une base du ou des groupements carboxy ;

c) salification par un acide du ou des groupements amino ;

d) dédoublement de la molécule pour obtenir un produit optiquement actif ;

e) oxydation de l'atome de soufre en position 2 du cycle isocéphème ;

f) déblocage de la fonction amine lorsque $R_A$ représente un radical amino protégé ;

g) traitement par un produit de formule $(IV_a)$, $(IV_b)$ ou $(IV_c)$, dans les conditions décrites à la revendication 6.

**10.** Procédé selon la revendication 9 de préparation des produits de formule (VI) telle que définie à la revendication 9 , caractérisé en ce que l'on traite un produit de formule $(II_B)$ :

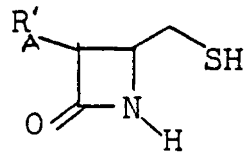

$(II'_B)$

dans laquelle $R'_A$ a la signification indiquée à la revendication 5, par un produit de formule $(III_B)$ :

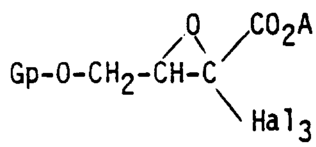

$(III'_B)$

dans laquelle A a la signification indiquée à la revendication 1, $Hal_3$ représente un atome d'halogène et Gp un groupement protecteur du radical hydroxyle pour obtenir un produit de formule (VIII) :

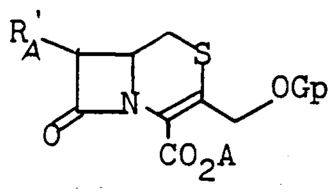

(VIII)

produit que l'on soumet à une réaction de déprotection pour obtenir le produit de formule (VII') :

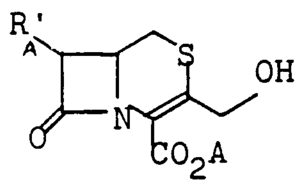

(VII')

produit que l'on soumet éventuellement d'abord à un réactif d'oxydation ou que l'on soumet à une réaction d'halogénation pour obtenir le produit de formule (VI ) :

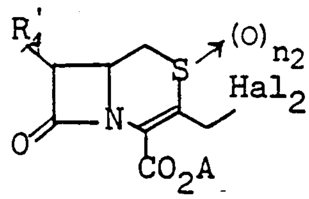

(VI )

produit que l'on soumet éventuellement à une réaction d'oxydation.

**11.** Procédé de préparation des produits de formule générale (XI) :

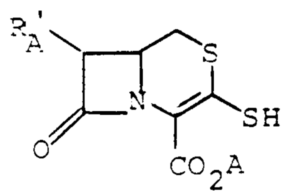

(XI)

dans laquelle $R'_A$ et $CO_2A$ ont la valeur indiquée à la revendication 5, caractérisé en ce que l'on transforme un produit de formule (XII) :

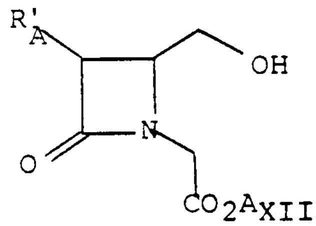

(XII)

dans laquelle $R'_A$ est défini comme précédemment et $A_{XII}$ représente un atome d'hydrogène ou un groupement ester facilement clivable, en un dérivé comportant un groupement réactif du radical hydroxy de formule (XIII) :

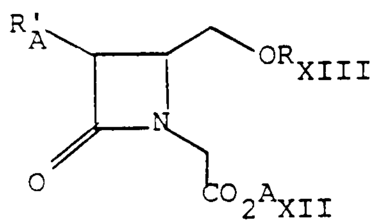

(XIII)

dans laquelle $R'_A$ et $A_{XII}$ sont définis comme précédemment et $R_{XIII}$ représente le reste d'un groupement réactif, que l'on traite par le sulfure de carbone en présence d'une base, pour obtenir le produit de formule (XI).

**12.** A titre de médicaments, les produits de formule (I') telle que définie à la revendication 1, ainsi que leurs sels d'acides pharmaceutiquement acceptables.

**13.** A titre de médicaments, les produits de formule (I') telle que définie à la revendication 2, ainsi que leurs sels d'acides pharmaceutiquement acceptables.

**14.** A titre de médicaments, les produits tels que définis à la revendication 3, ainsi que leurs sels d'acides pharmaceutiquement acceptables.

**15.** A titre de produits industriels nouveaux, les produits de formules ($III_A$) :

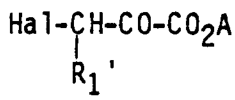

($III'_A$)

et ($III'_{Ai}$) :

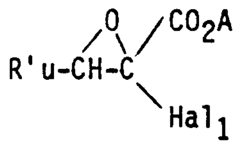

($III'_{Ai}$)

formules dans lesquelles Hal, $R_1$' et A ont la signification indiquée à la revendication 5 et R'u et $Hal_1$ ont la signification indiquée à la revendication 6.

**16.** A titre de produits industriels nouveaux, les produits de formule :

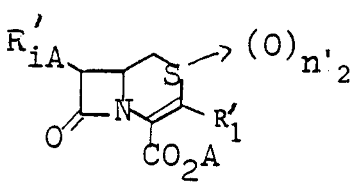

dans laquelle $R'_1$, A et $n'_2$ ont la signification indiquée à la revendication 1 et $R_{iA}$ représente un radical amino libre ou protégé par un groupement protecteur mono ou divalent.

**17.** A titre de produits industriels nouveaux, les produits de formule (XI) :

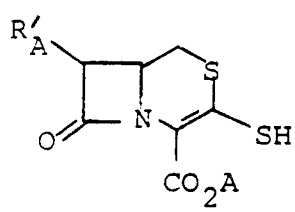

(XI)

dans laquelle $R'_A$ et $CO_2A$ ont la valeur indiquée à la revendication 5.

## Claims

**1.** The products of general formula (I'):

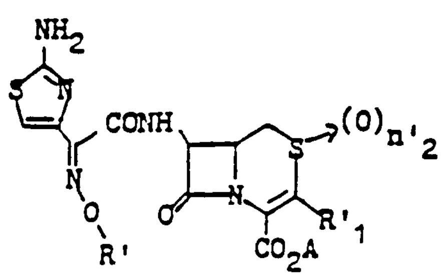

(I')

syn isomer

in which R' represents a hydrogen atom or an alkyl or alkenyl radical having at most 4 carbon atoms,

- a carboxymethyl or 1-carboxyethyl radical, these radicals being optionally esterified or salified,
- a phenyl radical, $R'_1$ represents:

a) a $Z'-R'_2$ radical in which $R'_2$ represents an alkyl or alkenyl radical having at most 4 carbon atoms, optionally interrupted by a heteroatom and optionally substituted by a halogen, amino, cyano radical, a free, esterified or salified carboxy radical, by a carbamoyl radical, Z' represents a sulphur or oxygen atom;

or $Z'-R'_2$ is chosen from the radicals

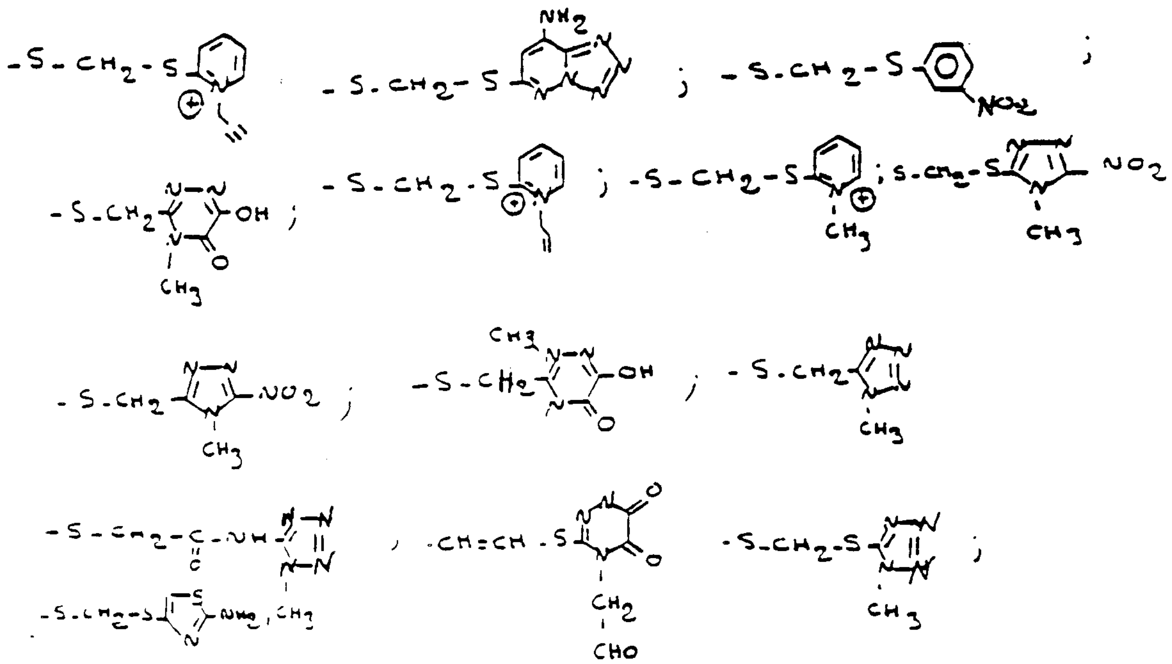

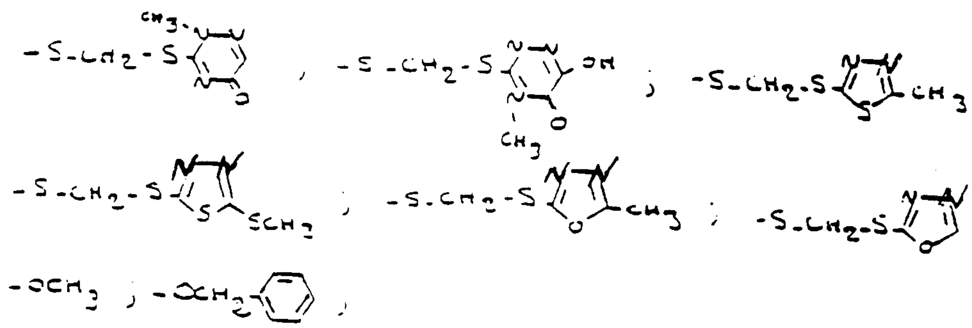

b) or a $Z'_a$-R'3 radical in which $R'_3$ represents a phenyl radical and $Z'_a$ represents a methylene radical, $-CH_2-S-$ or a sulphur, oxygen or selenium atom or a single bond;

or $Z'_a-R'_a$ is chosen from the radicals

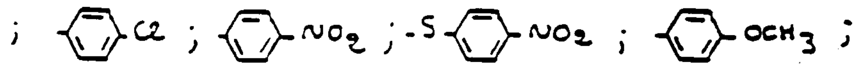

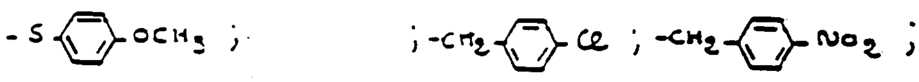

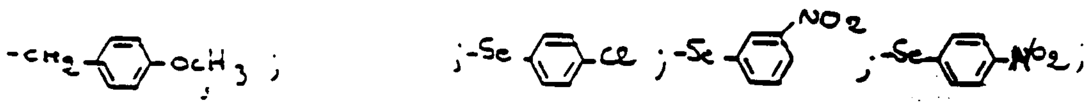

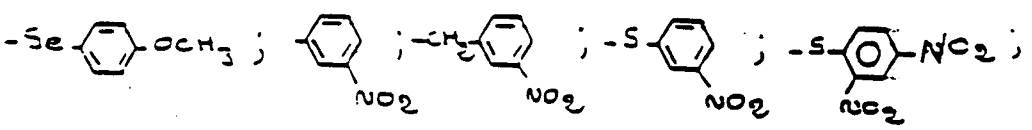

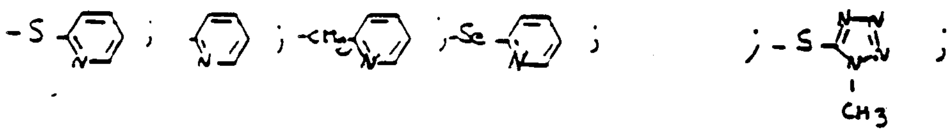

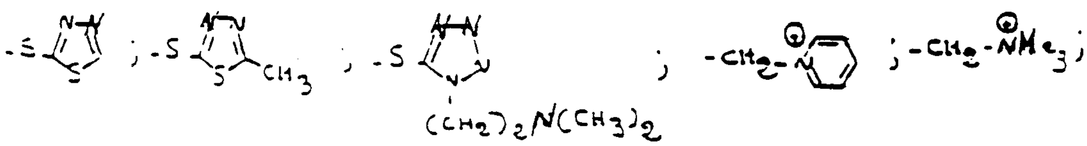

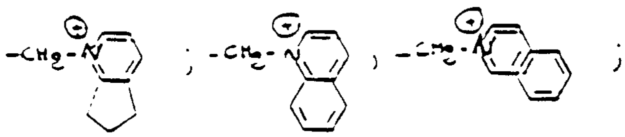

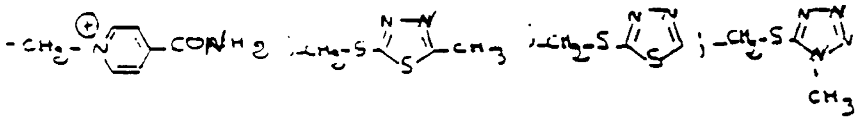

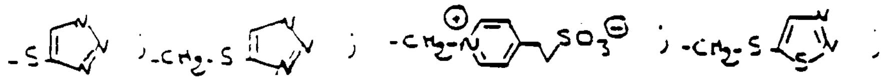

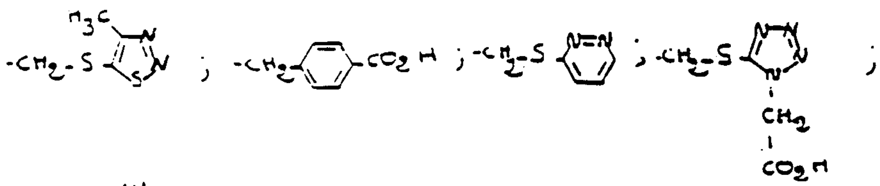

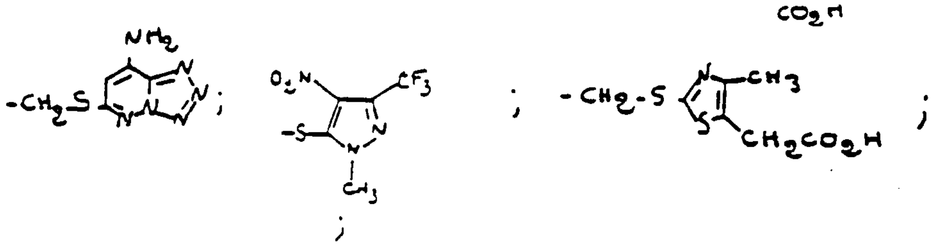

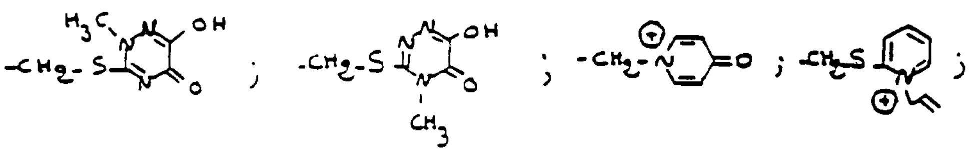

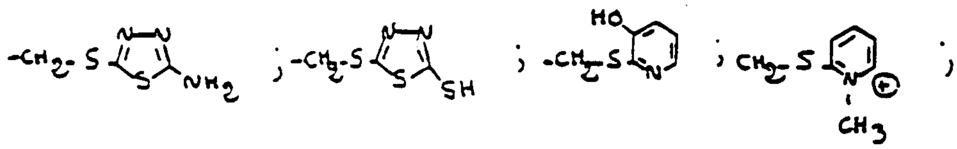

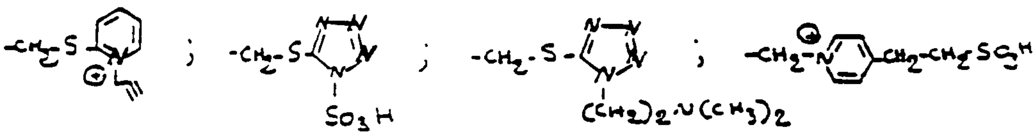

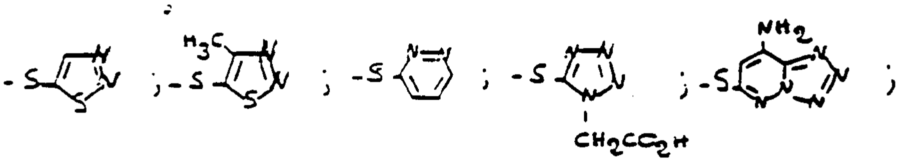

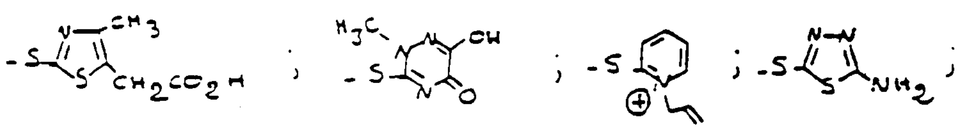

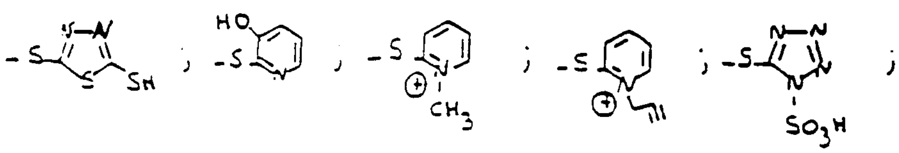

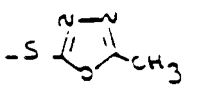 ; 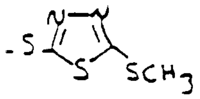 ; 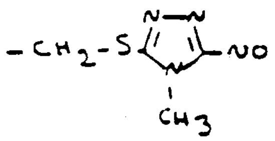 ; 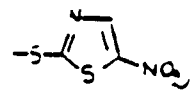

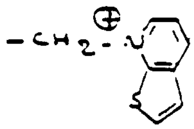 , 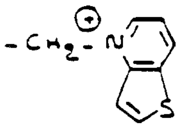 ; 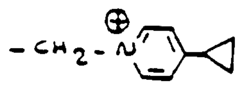 ; 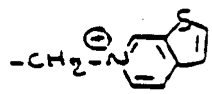

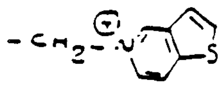 ; 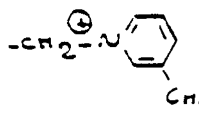 ; 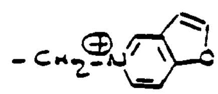 ; 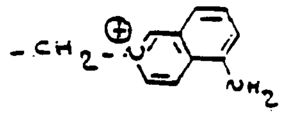

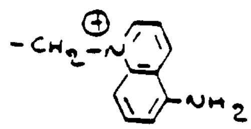 ;

n'$_2$ represents the numbers 0 and 1 and A represents a hydrogen atom, an equivalent of an alkaline or alkaline-earth metal, of magnesium, of ammonium or of an amino organic base or A represents an ester group or $CO_2A$ represents a $CO_2^-$ group, as well as the salts of the products of formula (I') with mineral or organic acids.

**2.** The products of general formula (I') as defined in claim 1, in which R' represents a hydrogen atom or a methyl or allyl radical and R'$_1$ represents a radical chosen from the group formed by the following radicals: -S-$CH_2CO_2H$; -S-$CH_2CO_2Et$; -S-$CH_2$-$CO_2tBu$;

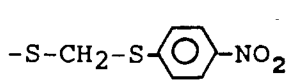 ;

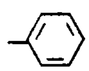 ; 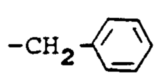 ; 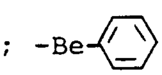 ; 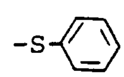 .

**3.** The products of general formula (I') as defined in claim 1, in racemic or optically active form, corresponding to the following formulae:
- syn isomer 7-/2-(2-aminothiazol-4-yl) 2-methoxy imino acetamido/ 3-(3-nitrophenylthio) 8-oxo 4-thia-1-azabicyclo /4,2,0/ oct-2-ene 2-carboxylic acid;
- syn isomer 7-/2-(2-aminothiazol-4-yl) 2-methoxyimino acetamido/ 3-(4-nitrophenyl) 8-oxo 4-thia 1-azabicyclo /4,2,0/ oct-2-ene 2-carboxylic acid;
- syn isomer 7-/2-(2-aminothiazol-4-yl) 2-methoxyimino acetamido/ 3-/(1-methyl (1-H) tetrazol-5-yl) thio/ 8-oxo 4-thia 1-azabicyclo /4,2,0/ oct-2-ene 2-carboxylic acid;
- syn isomer 7-/2-(2-aminothiazol-4-yl) 2-methoxyimino acetamido/ 8-oxo 3-/(1-methyl 1H-tetrazol-5-yl) thiomethyl/ 4-thia 1-azabicyclo /4,2,0/ oct-2-ene 2-carboxylic acid;
- syn isomer 1-//7-(2-amino thiazol-4-yl) (methoxyimino) acetamido/ 2-carboxy 8-oxo 4-thia 1-azabicyclo /4,2,0/ oct 2-ene 3-yl/ methyl/ pyridinium;
- syn isomer 1-//7-(2-aminothiazol-4-yl) (methoxyimino) acetamido/ 2-carboxy 8-oxo 4-thia 1-azabicyclo /4,2,0/ oct-2-ene 3-yl/ methyl/ (6,7-dihydro) 5-H/1/ pyrindinium;
- syn isomer 6-/7-/2-(2-amino thiazol-4-yl) 2-methoxyimino acetamido/ 2-carboxy 8-oxo 4-thia 1-azabicyclo /4,2,0/ oct-2-en-3-yl/ methyl thieno /2,3-c/ pyridinium;
- syn isomer 7-/2-(2-amino thiazol-4-yl) 2-methoxyimino acetamido/ 8-oxo 2-carboxy 4-thia 1-azabicyclo /4,2,0/ oct-2-en-3-yl/ methyl/ trimethyl ammonium;
- syn isomer //7-/2-(2-amino thiazol-4-yl) 2-methoxyimino acetamido/ 8-oxo 4-thia 2-carboxy 1-azabicyclo /4,2,0/ oct-2-en-3-yl/ methyl/ 4-cyclopropyl/ pyridinium;
- syn isomer 6-//7-/2-(2-amino thiazol-4-yl) 2-methoxyimino acetamido/ 8-oxo 4-thia 2-carboxy 1-azabicyclo /4,2,0/ oct-2-en-3-yl/ methyl/ 7-methyl thieno /2,3-c/ pyridinium;

- syn isomer //7-/2-(2-amino thiazol-4-yl) 2-methoxyimino acetamido/ 8-oxo 4-thia 2-carboxy 1-azabicyclo /4,2,0/ oct-2-en-3-yl/ methyl/ thieno /2,3-b/ pyridinium;
- syn isomer /7-/2-(2-amino thiazol-4-yl) 2-(2-propenyloxy) imino acetamido/ 2-carboxy 8-oxo 4-thia 1-azabicyclo /4,2,0/ oct-2-en-3-yl/ methyl pyridinium;
- syn isomer 1-//7-/2-(2-amino thiazol-4-yl) 2-methoxyimino acetamido/ 2-carboxy 8-oxo 4-tha 1-azabicyclo /4,2,0/ oct-2-en-3-yl/ methyl (2-methylthio)/ pyridinium.

**4.** Preparation process for the products of general formula (I'):

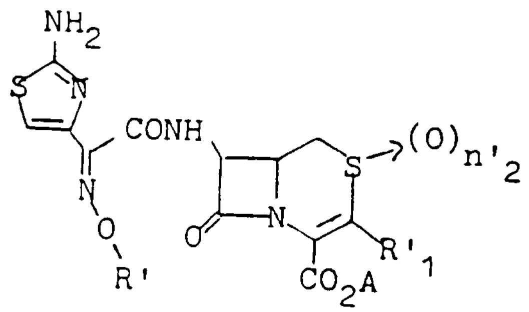

(I')

syn isomer
in which R', $R'_1$, A and $n'_2$ have the meaning indicated in claim 1, characterized in that a product of formula (II'):

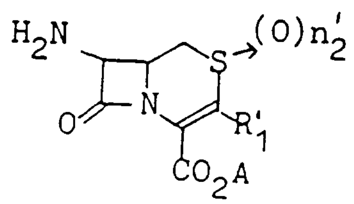

(II')

in which $R'_1$, A and $n'_2$ have the previous meaning, is treated with an acid of formula (IV'):

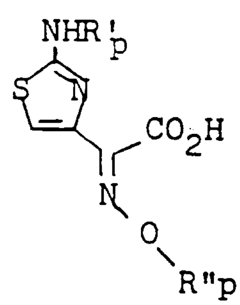

(IV')

or a functional derivative of this acid, in which formula (IV') R' p represents a hydrogen atom or a protective group of the amino radical and R"p represents a protective group of the hydroxyl radical or R"p represents R' in order to obtain a product of formula (V'):

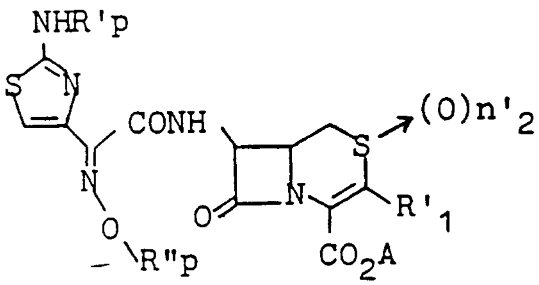

(V')

which product is subjected, if necessary or if desired, to one or more of the following reactions, in any order:

a) cutting of all or part of the protective group or groups by hydrolysis, hydrogenolysis or by the action of thiourea;
b) esterification or salification of the carboxy group or groups by a base;
c) salification of the amino group or groups by an acid;
d) resolution of the molecule in order to obtain an optically active product;
e) oxidation of the sulphur atom in position 2 of the isocepheme ring.

**5.** Preparation process for the products of formula (A'):

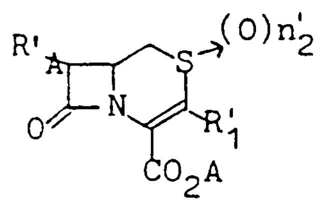

(A')

in which A, $R'_1$ and $n'_2$ have the meaning indicated in claim 1 and $R'_A$ represents a free amino radical or protected by a mono- or divalent protective group, or $R'_A$ represents a radical:

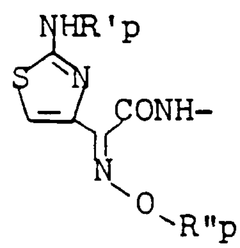

in which R'p and R"p have the meaning indicated in claim 4, characterized in that a product of formula ($II'_A$):

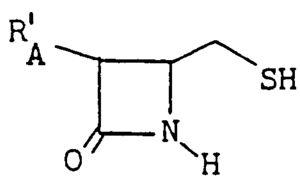

($II'_A$)

is treated with a product of formula ($III'_A$):

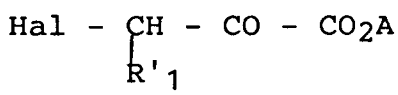

(III'$_A$)

in which Hal represents a halogen atom, $R'_1$ and A have the previous meaning, in order to obtain a product of formula (A'), which product is subjected, if necessary or if desired, to any one or more of the following reactions, in any order:

a) cutting of all or part of the protective group or groups by hydrolysis, hydrogenolysis or by the action of thiourea;
b) esterification or salification of the carboxy group or groups by a base;
c) salification of the amino group or groups by an acid;
d) resolution of the molecule in order to obtain an optically active product;
e) oxidation of the sulphur atom in position 2 of the isocepheme ring.

**6.** Process according to claim 5 for the preparation of the products of formula (III'$_{Ai}$):

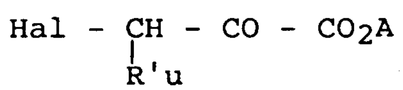

(III'$_{Ai}$)

in which Hal represents a halogen atom, R'u represents the values of $R'_1$ linked by a carbon atom, $R'_1$ and A having the meaning indicated in claim 1, characterized in that a product of formula R'uCHO is treated with a product of formula $(Hal_1)_2$ $CHCO_2A$ in the presence of a strong base in order to obtain a product of formula:

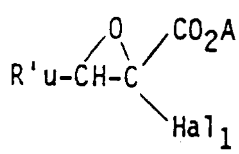

(III'$_{Ai}$)

in which formula $Hal_1$ represents a halogen atom, which product is treated with a halide in order to obtain the expected product of formula (III'$_{A1}$).

**7.** Variant of the process as described in claim 5 for the preparation of the products of formula (A'):

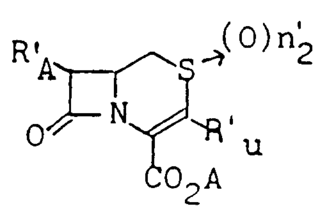

(A')

in which R'$_A$, $n_2$' and A have the meaning indicated in claim 5 and R'u has the meaning indicated in claim 6, characterized in that a product of formula (II'$_A$) as described in claim 5 is treated with a product of formula (III'$_{Ai}$) as described in claim 6.

**8.** Process according to claim 5 for the preparation of the products of formula (III"$_{A1}$):

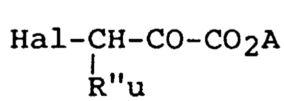

(III"$_{A1}$)

in which Hal represents a halogen atom, R''u represents the values of $R'_1$ linked by a heteroatom, $R'_1$ and A having the meaning indicated in claim 1, characterized in that a product of formula:

$N_2 = CH\text{-}COCO_2A$  ($III''_{A1}$)

is reacted with a product of formula R''U Hal in order to obtain the expected product of formula ($III''_{A1}$).

**9.** Process according to claim 4 for the preparation of the products of formula (IX):

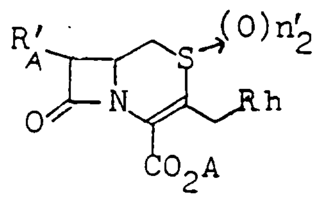

(IX)

in which $R'_A$, A and $n'_2$ have the meaning indicated in claims 1 to 5 and Rh represents an optionally substituted quaternary ammonium or an S-R'h radical in which R'h represents an optionally substituted carbocyclic or heterocyclic aryl radical as defined in claim 1 corresponding to the products of formula (I') when R'A represents the radical

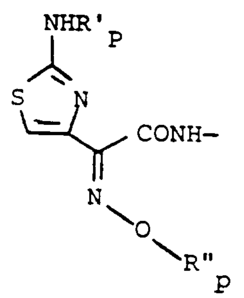

characterized in that, either a product of formula R'h Sh is reacted on a product of formula (VI):

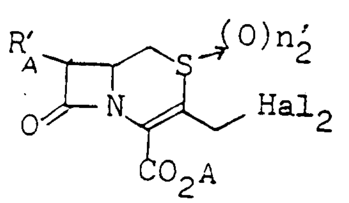

(VI)

in which formula $R_A$, $R_4$, A and $n_2$ have the previous meaning and $Hal_2$ represents a halogen atom, or an amine or an imine is reacted on a product of formula (VII):

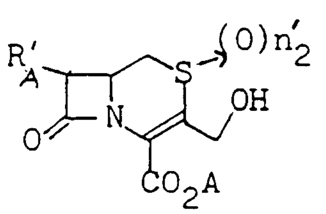

(VII)

in order to obtain a product of formula (IX) which is subjected, is necessary or if desired, to any one or more of the following reactions, in any order:

a) cutting of all or part of the protective group or groups by hydrolysis, hydrogenolysis or by the action of thiourea;
b) esterification or salification of the carboxy group or groups by a base;
c) salification of the amino group or groups by an acid;
d) resolution of the molecule in order to obtain an optically active product;
e) oxidation of the sulphur atom in position 2 of the isocepheme ring;
f) unblocking of the amine function when $R_A$ represents a protected amino radical;
g) treatment by a product of formula ($IV_a$), ($IV_b$) or ($IV_c$), in the conditions described in claim 6.

**10.** Process according to claim 9 for the preparation of the products of formula (VI) as defined in claim 9, characterized in that a product of formula (II'B):

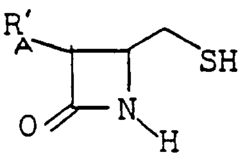

($II'_B$)

in which $R'_A$ has the meaning indicated in claim 5, is treated with a product of formula (III'B):

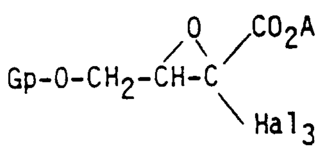

($III'_B$)

in which A has the meaning indicated in claim 1, $Hal_3$ represents a halogen atom and Gp a protective group of the hydroxyl radical in order to obtain a product of formula (VIII):

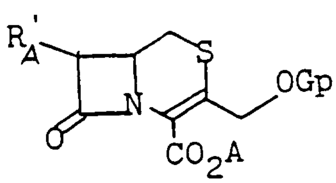

(VIII)

which product is subjected to a deprotection reaction in order to obtain the product of formula (VII'):

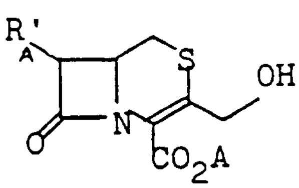

(VII')

which product is optionally subjected firstly to an oxidation reaction or is subjected to a halogenation reaction in order to obtain the product of formula (VI):

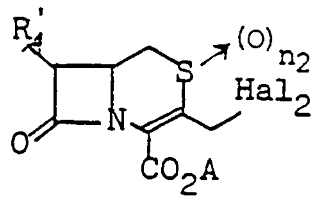

(VI)

which product is optionally subjected to an oxidation reaction.

**11.** Preparation process for the products of general formula (XI):

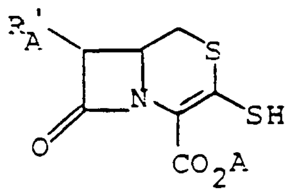

(XI)

in which $R'_A$ and $CO_2A$ have the meaning indicated in claim 5, characterized in that a product of formula (XII):

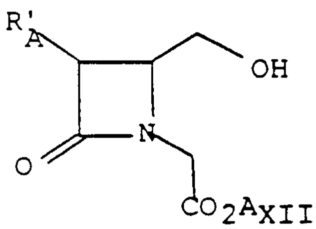

(XII)

in which $R'_A$ is defined as previously and $A_{XII}$ represents a hydrogen atom or an easily cleavable ester group, is converted into a derivative containing a reactive group of the hydroxy radical of formula (XIII):

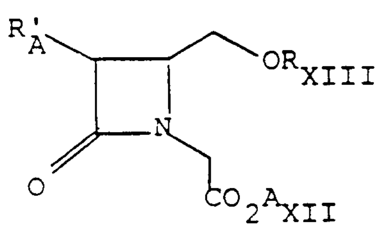

(XIII)

in which $R'_A$ and $A_{XII}$ are defined as previously and $R_{XIII}$ represents the remainder of a reactive group, which is treated with carbon sulphide in the presence of a base, in order to obtain the product of formula (XI).

**12.** As medicaments, the products of formula (I') as defined in claim 1, as well as their pharmaceutically acceptable acid salts.

**13.** As medicaments, the products of formula (I') as defined in claim 2, as well as their pharmaceutically acceptable acid salts.

**14.** As medicaments, the products as defined in claim 3, as well as their pharmaceutically acceptable acid salts.

**15.** As new industrial products, the products of formulae (III'$_A$):

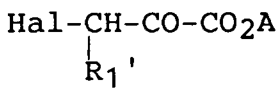

(III'$_A$)

and (III'$_{Ai}$):

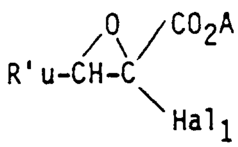

(III'$_{Ai}$)

in which formulae Hal, $R_1$' and A have the meaning indicated in claim 5 and R'u and $Hal_1$ have the meaning indicated in claim 6.

**16.** As new industrial products, the products of formula:

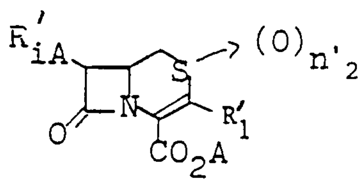

in which $R'_1$, A and $n'_2$ have the meaning indicated in claim 1 and $R_{iA}$ represents a free amino radical or protected by a mono- or divalent protective group.

**17.** As new industrial products, the products of formula (XI):

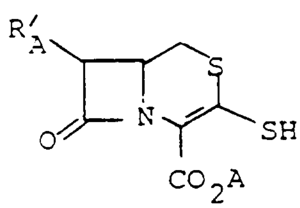

(XI)

in which $R'_A$ and $CO_2A$ have the value indicated in claim 5.

**Patentansprüche**

**1.** Verbindungen der allgemeinen Formel I' (syn-Isomere)

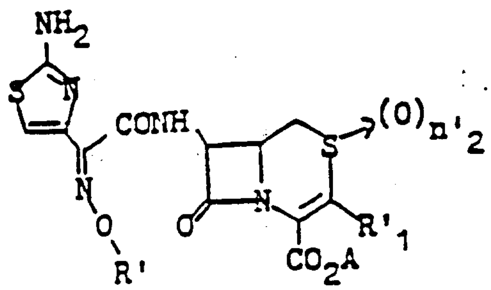

(I'),

worin bedeuten:

R'

- ein Wasserstoffatom oder eine Alkyl- oder Alkenylgruppe mit maximal 4 Kohlenstoffatomen,
- Carboxymethyl oder 1-Carboxyethyl, wobei diese Gruppen gegebenenfalls verestert sind oder in der Salzform vorliegen,
  oder
- eine Phenylgruppe,

$R'_1$

a) eine Gruppe $Z'-R'_2$, worin $R'_2$ eine Alkyl- oder Alkenylgruppe mit maximal 4 Kohlenstoffatomen bedeutet, die gegebenenfalls durch ein Heteroatom unterbrochen und gegebenenfalls mit einem Halogen, einer Aminogruppe, einer Cyanogruppe, einer freien, veresterten oder in die Salzform übergeführten Carboxygruppe oder einer Carbamoylgruppe substituiert ist, und Z' ein Schwefel- oder Sauerstoffatom bedeutet,
wobei $Z'-R'_2$ ausgewählt sein kann unter:

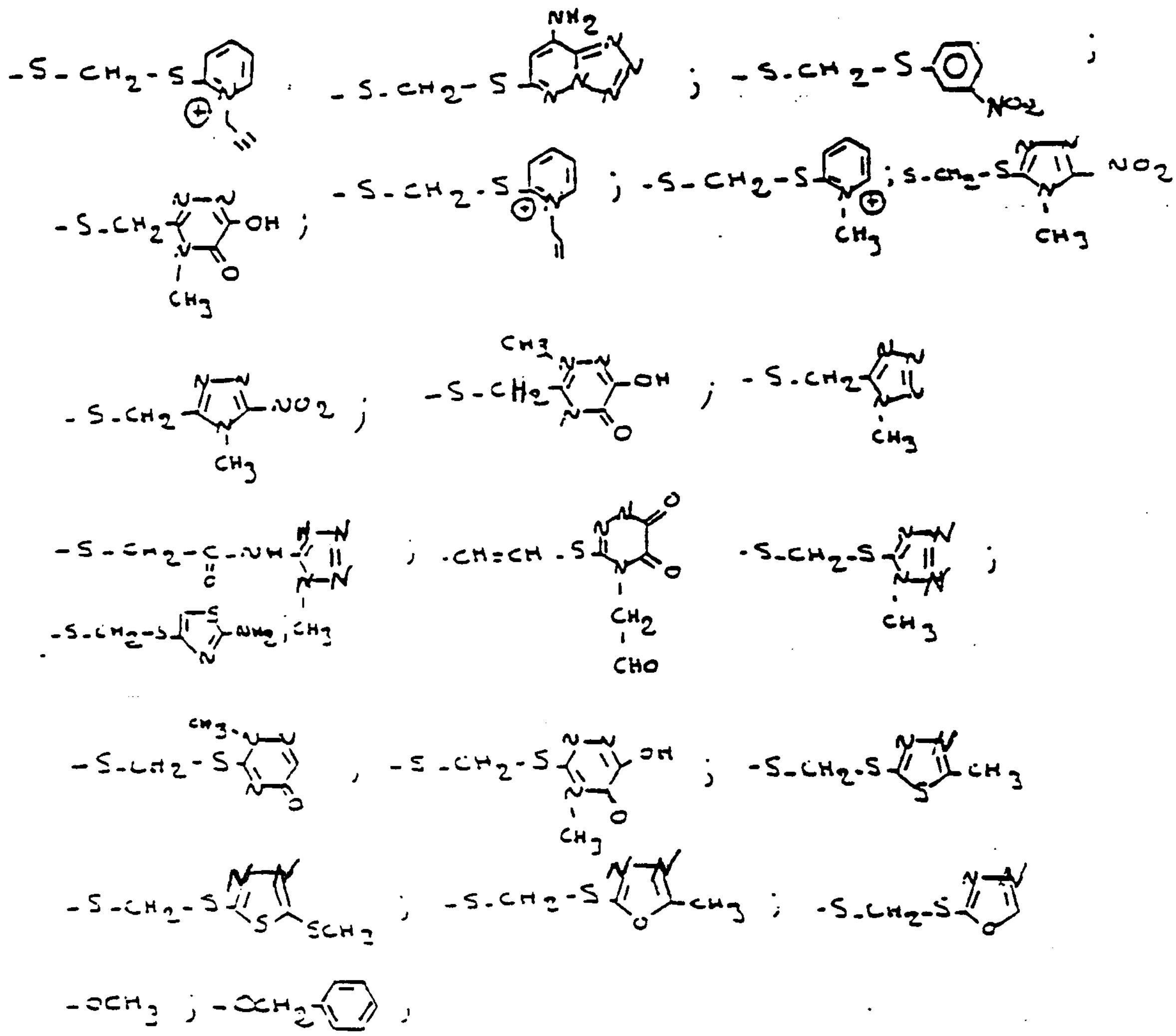

oder

b) eine Gruppe $Z'_a$-$R'_3$, worin $R'_3$ eine Phenylgruppe und $Z'_a$ eine Methylengruppe, $-CH_2-S-$, ein Schwefel-, Sauerstoff- oder Selenatom oder eine Einfachbindung bedeutet, wobei $Z'_a$-$R'_3$ ausgewählt sein kann unter

; 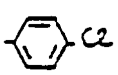 ; 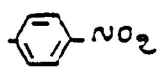 ; 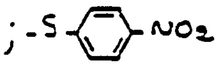 ; 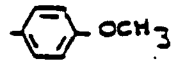 ;

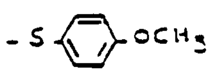 ; 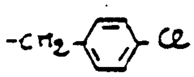 ; 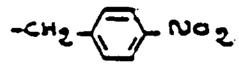 ;

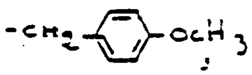 ; 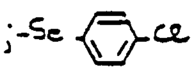 ; 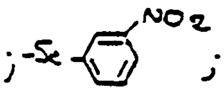 ; 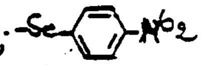 ;

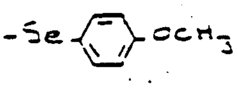 ; 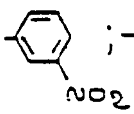 ; 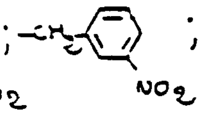 ; 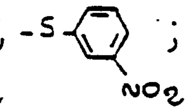 ; 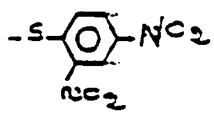 ;

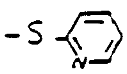 ; 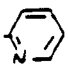 ; 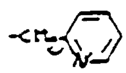 ; 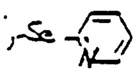 ; 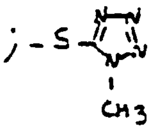 ;

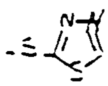 ; 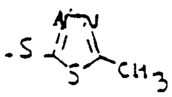 ; 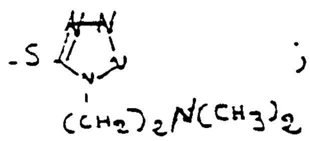 ; 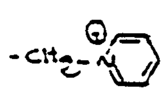 ; 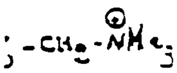 ;

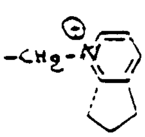 ; 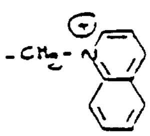 ; 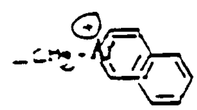 ;

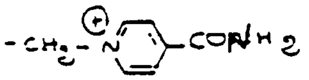 ; 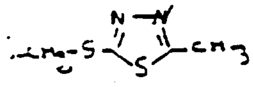 ; 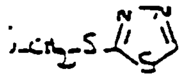 ; 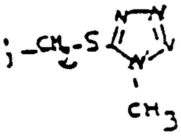

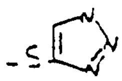 ; 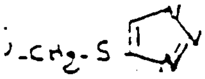 ; 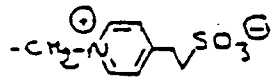 ; 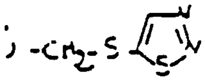 ;

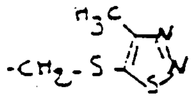 ; 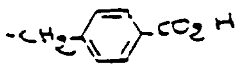 ; 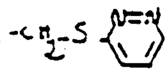 ; 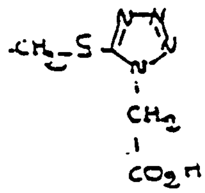 ;

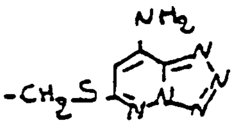 ; 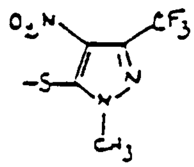 ; ; 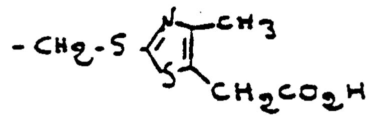 ;

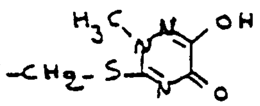 ; 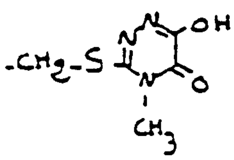 ; 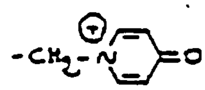 ; 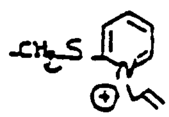 ;

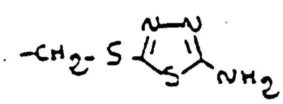 ; 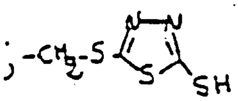 ; 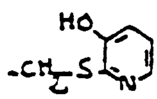 ; 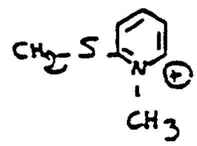 ;

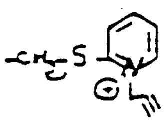 ; 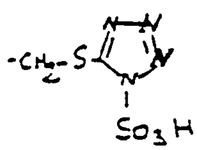 ; 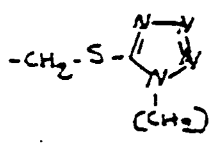 ; 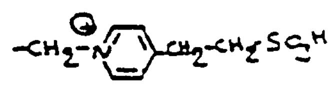

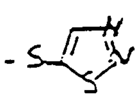 ; 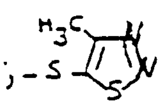 ; 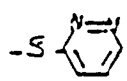 ; 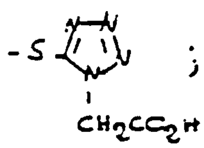 ; 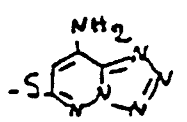 ;

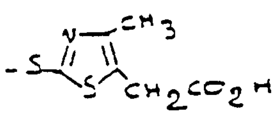 ; 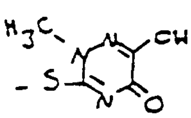 ; 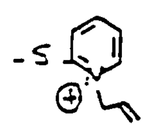 ; 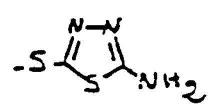 ;

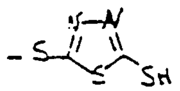 ; 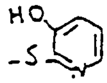 ; 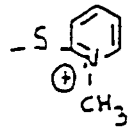 ; 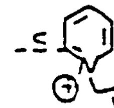 ; 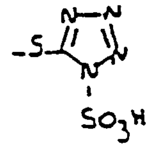 ;

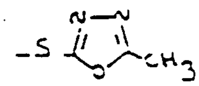 ; 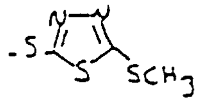 ; 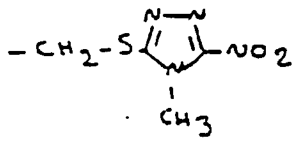 ; 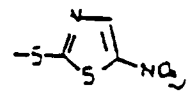

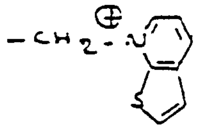 ; 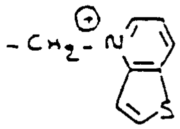 ; 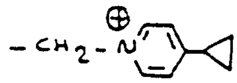 ; 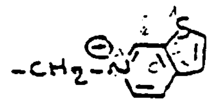

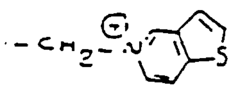 ; 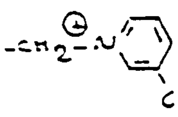 ; 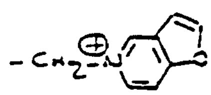 ; 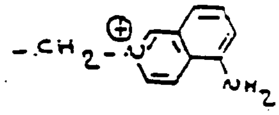

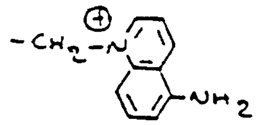 ;

n'$_2$ die Zahlen 0 oder 1
und

A ein Wasserstoffatom, Ammonium, ein Alkalimetall, ein Äquivalent eines Erdalkalimetalls einschließlich Magnesium oder einer organischen Aminbase oder eine Estergruppe, wobei $CO_2A$ auch eine $CO_2^-$-Gruppe bedeuten kann,

sowie die Salze der Verbindungen der Formel I' mit anorganischen oder organischen Säuren.

**2.** Verbindungen der allgemeinen Formel I' nach Anspruch 1, worin R' ein Wasserstoffatom, Methyl oder Allyl bedeutet und R'$_1$ unter den folgenden Gruppen ausgewählt ist: $-S-CH_2CO_2H$ ; $-S-CH_2CO_2Et$ ; $-S-CH_2-CO_2tBu$ ;

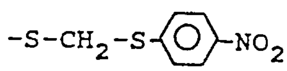 ;

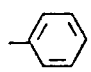 ; 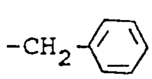 ; 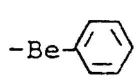 ; 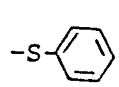 .

**3.** Die nachstehenden Verbindungen der allgemeinen Formel I' nach Anspruch 1 in racemischer oder optisch aktiver Form:

- 7-[2-(2-Aminothiazol-4-yl)-2-methoximinoacetamido]-3-(3-nitrophenylthio)-8-oxo-4-thia-1-azabicyclo-[4.2.0]oct-2-en-2-carbonsäure, syn-Isomer;
- 7-[2-(2-Aminothiazol-4-yl)-2-methoximinoacetamido]-3-(4-nitrophenyl)-8-oxo-4-thia-1-azabicyclo-[4.2.0]-oct-2-en-2-carbonsäure, syn-Isomer;
- 7-[2-(2-Aminothiazol-4-yl)-2-methoximinoacetamido]-3-(1-methyl-1H-tetrazol-5-yl-thio)-8-oxo-4-thia-1-azabicyclo[4.2.0]oct-2-en-2-carbonsäure, syn-Isomer;
- 7-[2-(2-Aminothiazol-4-yl)-2-methoximinoacetamido]-8-oxo-3-(1-methyl-1H-tetrazol-5-yl-thiomethyl)-4-thia-1-azabicyclo[4.2.0]oct-2-en-2-carbonsäure, syn-Isomer;
- 1-[[7-(2-Aminothiazol-4-yl)-2-methoximinoacetamido]-2-carboxy-8-oxo-4-thia-1-azabicyclo[4.2.0] oct-2-en-3-yl]-methylpyridinium, syn-Isomer;
- 1-[[7-(2-Aminothiazol-4-yl)-2-methoximinoacetamid]-2- carboxy-8-oxo-4-thia-1-azabicyclo[4.2.0]oct-2-en-3-yl]-methyl]-6,7-dihydro-5H-1-pyridinium, syn-Isomer;

- 6-[7-[2-(2-Aminothiazol-4-yl)-2-methoximinoacetamido]-2-carboxy-8-oxo-4-thia-1-azabicyclo[4.2.0]-oct-2-en-3-yl]-methylthieno[2,3-c]pyridinium, syn-Isomer;
- 7-[2-(2-Aminothiazol-4-yl)-2-methoximinoacetamido]-8-oxo-2-carboxy-4-thia-1-azabicyclo[4.2.0]oct-2-en-3-yl-methyl-trimethylammonium, syn-Isomer;
- [7- [2-(2-Aminothiazol-4-Yl)-2-methoximinoacetamido]-8-oxo-4-thia-2-carboxy-1-azabicyclo[4.2.0]-oct-2-en-3-yl]-methyl-4-cyclopropylpyridinium, syn-Isomer;
- 6-[7-[2-(2-Aminothiazol-4-yl)-2-methoximinoacetami-do]-8-oxo-4-thia-2-carboxy-1-azabicyclo-[4.2.0]oct-2-en-3-yl]-methyl-7-methylthieno[2,3-c]pyridinium, syn-Isomer;
- [7-[2-(2-Aminothiazol-4-yl)-2-methoximinoacetamido]-8-oxo-4-thia-2-carboxxy-1-azabicyclo[4.2.0.]-oct-2-en-3-yl]-methylthieno[2,3-b]pyridinium, syn-Isomer;
- [7-[2-(2-Aminothiazol-4-yl)-2-(2-propenyloxy)-imino-acetamido]-2-carboxy-8-oxo-4-thia-1-azabicyclo[4.2.0] oct-2-en-3-yl]-methylpyridinium, syn-Isomer;
- 1-[[7-[2-(2-Aminothiazol-4-yl)-2-methoximinoacetami-do]-2-carboxy-8-oxo-4-thia-1-azabicyclo-[4.2.0]oct-2 en-3-yl]-methyl]-(2-methylthio)-pyridinium, syn-Isomer.

**4.** Verfahren zur Herstellung der Verbindungen der allgemeinen Formel I' (syn-Isomere)

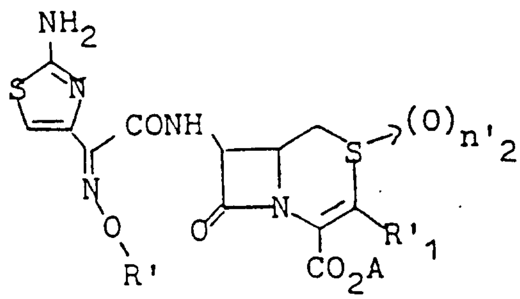

(I'),

worin R', R'$_1$, A und n' 2 die in Anspruch 1 angegebene Bedeutung besitzen,
gekennzeichnet durch
Behandlung einer Verbindung der Formel II',

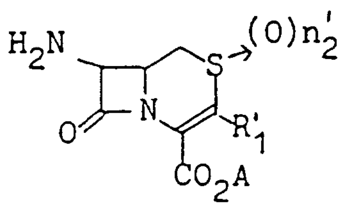

(II'),

worin R'$_1$, A und n'$_2$ die obige Bedeutung besitzen,
mit einer Säure der Formel IV'

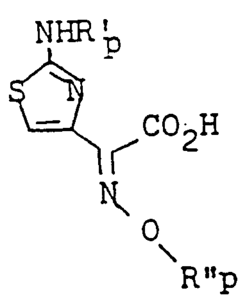

(IV'),

worin R'p ein Wasserstoffatom oder eine Amino-Schutzgruppe und R"p eine Hydroxy-Schutzgruppe oder R' bedeuten,

oder einem funktionellen Derivat dieser Säure unter Erhalt einer Verbindung der Formel V'

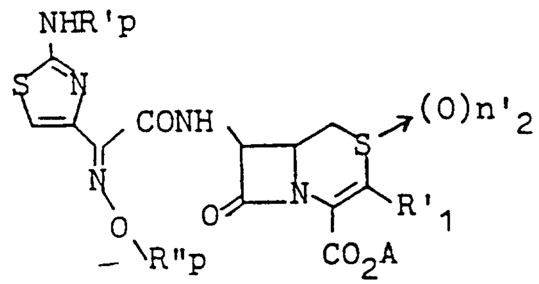

(V'),

die, wenn nötig oder wenn gewünscht, einer oder mehreren der folgenden Reaktionen in beliebiger Reihenfolge unterzogen wird:

a) Abspaltung aller oder eines Teils der Schutzgruppen durch Hydrolyse, Hydrogenolyse oder durch Einwirkung von Thioharnstoff;

b) Veresterung der Carboxygruppe(n) oder Überführung mit einer Base in Salze;

c) Überführung der Aminogruppe(n) mit einer Säure in die Salze;

d) Racematspaltung zur Herstellung einer optisch aktiven Verbindung;

e) Oxidation des Schwefelatoms in Position 2 des Isocephemrings.

**5.** Verfahren zur Herstellung der Verbindungen der Formel A'

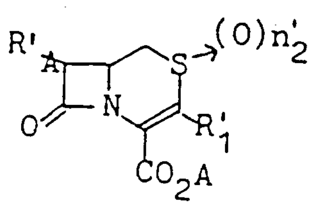

(A'),

Worin A, $R'_1$ und $n'_2$ die in Anspruch 1 angegebene Bedeutung besitzen und $R'_A$ eine freie oder mit einer ein- oder zweiwertigen Schutzgruppe geschützte Aminogruppe oder die folgende Gruppe bedeutet:

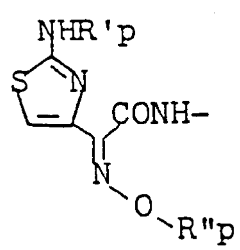

,

in deren Formel R'p und R''p die in Anspruch 4 angegebene Bedeutung besitzen,
gekennzeichnet durch
Behandlung einer Verbindung der Formel $II'_A$

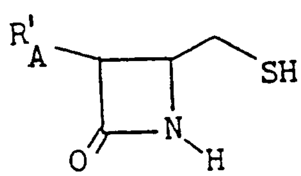

($II'_A$)

mit einer Verbindung der Formel III'$_A$,

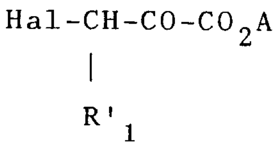

(III'$_A$),

worin Hal ein Halogenatom bedeutet und R'$_1$ und A die obige Bedeutung besitzen,
unter Erhalt einer Verbindung der Formel A', die, wenn nötig oder wenn gewünscht, einer oder mehreren der folgenden Reaktionen in beliebiger Reihenfolge unterzogen wird:

a) Abspaltung eines Teils oder aller Schutzgruppen durch Hydrolyse, Hydrogenolyse oder Einwirkung von Thioharnstoff;
b) Veresterung der Carboxygruppe(n) oder Überführung mit einer Base in Salze;
c) Überführung der Aminogruppe(n) mit einer Säure in Salze;
d) Racematspaltung zur Herstellung einer optisch aktiven Verbindung;
e) Oxidation des Schwefelatoms in Position 2 des Isocephemrings.

**6.** Verfahren nach Anspruch 5 zur Herstellung von Verbindungen der Formel III'$_{A1}$

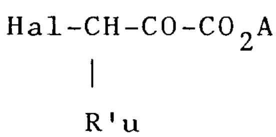

(III'$_{A1}$),

worin Hal ein Halogenatom und R'u über ein Kohlenstoffatom gebundenes R'$_1$ bedeuten, wobei R'$_1$ und A die in Anspruch 1 angegebene Bedeutung besitzen,
gekennzeichnet durch
Behandlung einer Verbindung der Formel R'uCHO mit einer Verbindung der Formel $(Hal_1)_2CHCO_2A$ in Gegenwart einer starken Base unter Erhalt einer Verbindung der Formel III'$_{Ai}$,

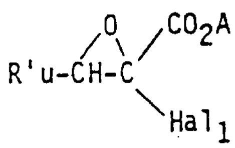

(III'$_{Ai}$)

worin Hal$_1$ ein Halogenatom bedeutet,
und Behandlung dieser Verbindung mit einem Halogenid unter Erhalt der angestrebten Verbindung der Formel III'$_{A1}$.

**7.** Variante des Verfahrens von Anspruch 5 zur Herstellung von Verbindungen der Formel A',

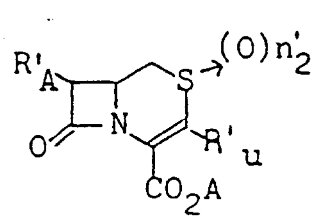

(A'),

worin R'$_A$, n'$_2$ und A die in Anspruch 5 angegebene Bedeutung und R'u die in Anspruch 6 angegebene Bedeutung besitzen,
gekennzeichnet durch
Behandlung einer Verbindung der Formel II'$_A$ wie in Anspruch 5 definiert mit einer Verbindung der

Formel $III'_{Ai}$ wie in Anspruch 6 beschrieben.

**8.** Verfahren nach Anspruch 5 zur Herstellung der Verbindungen der Formel $III''_{A1}$,

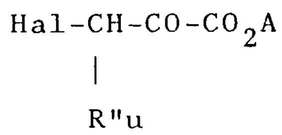

$(III''_{A1})$,

worin Hal ein Halogenatom und R"u über ein Heteroatom gebundenes $R'_1$ bedeuten und $R'_1$ und A die in Anspruch 1 angegebene Bedeutung besitzen,
gekennzeichnet durch
Umsetzung einer Verbindung der Formel $III''_{A1}$

$N_2 = CH\text{-}COCO_2A$ $(III''_{A1})$

mit einer Verbindung der Formel R"uHal unter Erhalt der angestrebten Verbindung der Formel $III''_{A1}$.

**9.** Verfahren nach Anspruch 4 zur Herstellung von Verbindungen der Formel IX,

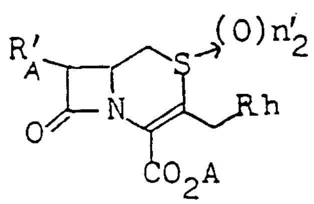

(IX),

worin $R'_A$, A und $n'_2$ die in den Ansprüchen 1 und 5 angegebene Bedeutung besitzen und Rh ein gegebenenfalls substituiertes quaternäres Ammonium oder eine Gruppe S-R'h bedeutet, worin R'h eine wie in Anspruch 1 definierte, gegebenenfalls substituierte carbocyclische oder heterocyclische Arylgruppe bedeutet,
die Verbindungen der Formel I' entspricht, wenn $R'_A$ die Gruppe

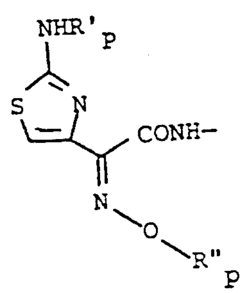

darstellt,
dadurch gekennzeichnet, daß man entweder eine Verbindung der Formel R'h Sh mit einer Verbindung der Formel VI

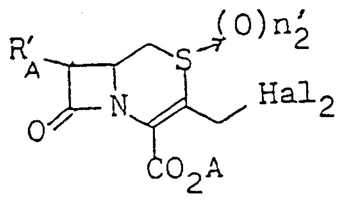

(VI)

umsetzt, worin $R_A$, $R_4$, A und $n_2$ die obige Bedeutung besitzen und $Hal_2$ ein Halogenatom darstellt, oder ein Amin oder ein Imin mit einer Verbindung der Formel VII

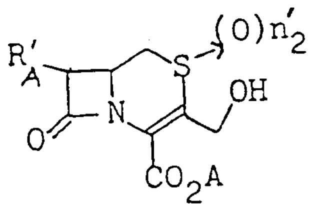

(VII)

umsetzt, wobei eine Verbindung der Formel IX erhalten wird, die, wenn nötig oder wenn gewünscht, einer oder mehreren der folgenden Reaktionen in beliebiger Reihenfolge unterzogen wird:

a) Abspaltung aller oder eines Teils der Schutzgruppen durch Hydrolyse, Hvdrogenolyse oder Einwirkung von Thioharnstoff;
b) Veresterung der Carboxygruppe oder Überführung mit einer Base in das Salz;
c) Überführung der Aminogruppe(n) mit einer Säure in Salze;
d) Racematspaltung zur Herstellung einer optisch aktiven Verbindung;
e) Oxidation des Schwefelatoms in Position 2 des Isocephemrings;
f) Abspaltung der Schutzgruppe von der Aminogruppe, wenn $R_A$ eine geschützte Aminogruppe bedeutet;
g) Behandlung mit einer Verbindung der Formeln $IV_a$, $IV_b$ oder $IV_c$ unter den in Anspruch 6 beschriebenen Bedingungen.

**10.** Verfahren nach Anspruch 9 zur Herstellung der Verbindungen der Formel VI wie in Anspruch 9 definiert gekennzeichnet durch
Behandlung einer Verbindung der Formel $II'_B$,

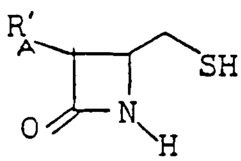

$(II'_B)$,

worin $R'_A$ die in Anspruch 5 angegebene Bedeutung besitzt, mit einer Verbindung der Formel $III'_B$

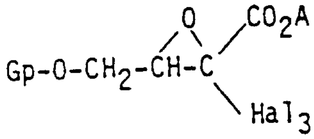

$(III'_B)$,

worin A die in Anspruch 1 angegebene Bedeutung besitzt und $Hal_3$ ein Halogenatom und Gp eine Hydroxy-Schutzgruppe bedeuten,
unter Erhalt einer Verbindung der Formel VIII,

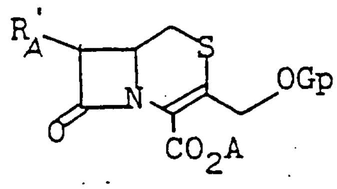

(VIII),

und
Abspaltung der Schutzgruppen von der Verbindung VIII unter Erhalt einer Verbindung der Formel VII',

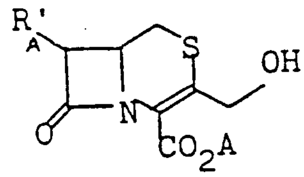

(VII'),

die gegebenenfalls zunächst mit einem Oxidationsmittel behandelt oder einer Halogenierungsreaktion unterzogen wird, wobei eine Verbindung der Formel VI erhalten wird,

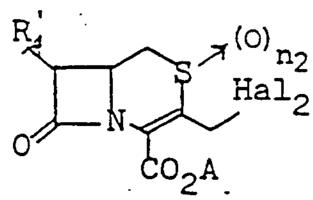

(VI),

die gegebenenfalls oxidiert wird.

**11.** Verfahren zur Herstellung der Verbindungen der allgemeinen Formel XI,

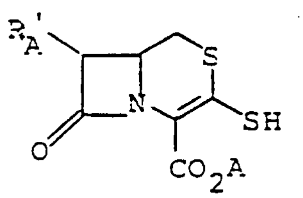

(XI),

worin $R'_A$ und $CO_2A$ die in Anspruch 5 angegebene Bedeutung besitzen,
gekennzeichnet durch
Überführung einer Verbindung der Formel XII,

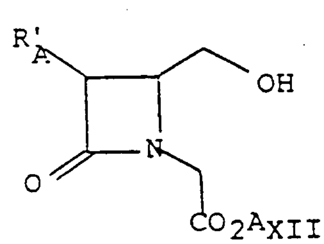

(XII),

worin $R'_A$ das gleiche wie oben definiert und $A_{XII}$ ein Wasserstoffatom oder eine leicht abspaltbare Veresterung bedeutet,
in ein Derivat der Formel XIII, das eine reaktive Hydroxygruppe aufweist,

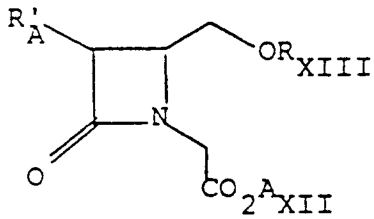

(XIII),

worin $R'_A$ und $A_{XII}$ das gleiche wie oben definiert und $R_{XIII}$ den Rest einer reaktiven Gruppe bedeuten, das man in Gegenwart einer Base mit Schwefelkohlenstoff behandelt, wobei die Verbindung der Formel XI erhalten wird.

**12.** Verbindungen der Formel I' nach Anspruch 1 und ihre pharmazeutisch akzeptablen Säuresalze als Medikamente.

**13.** Verbindungen der Formel I' nach Anspruch 2 und ihre Salze mit pharmazeutisch akzeptablen Säuren als Medikamente.

**14.** Verbindungen nach Anspruch 3 und ihre Salze mit pharmazeutisch akzeptablen Säuren als Medikamente.

**15.** Verbindungen der Formeln $III_A$

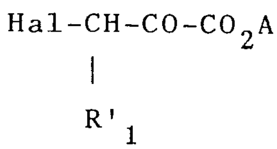

$(III_A)$

und $III'_{Ai}$

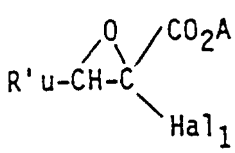

$(III'_{Ai})$,

worin Hal, $R'_1$ und A die in Anspruch 5 und R'u und $Hal_1$ die in Anspruch 6 angegebene Bedeutung besitzen, als neue technische Verbindungen.

**16.** Verbindungen der Formel

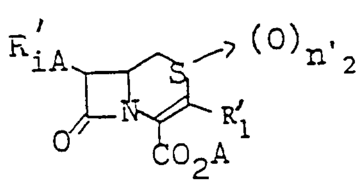

,

worin R'$_1$, A und n'$_2$ die in Anspruch 1 angegebene Bedeutung besitzen und $R_{iA}$ eine freie oder mit einer ein- oder zweiwertigen Schutzgruppe geschützte Aminogruppe bedeutet, als neue technische Verbindungen.

**17.** Verbindungen der Formel XI

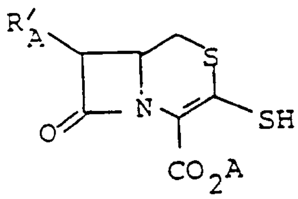

(XI),

worin R'$_A$ und $CO_2A$ die in Anspruch 5 angegebene Bedeutung besitzen, als neue technische Verbindungen.